# EUROPEAN PATENT APPLICATION

(11) **EP 1 642 588 A2**
(43) Date of publication of application: **05.04.2006**
(21) Application number: 05018781.4
(22) Date of filing: 30.08.2005
(51) Int. Cl.: A61K 38/20, C07K 14/54

(54) **Mimetics of interleukin-8 and methods of using them in the prevention, treatment, diagnosis, and ameliorization of symptoms of a disease**

(30) Priority: 31.08.2004 US 932208
(71) Applicant: Chemokine Therapeutics Corporation, Vancouver BC V6T 1Z3 (CA)
(72) Inventor: Merzouk, Ahmed, Richmond, Britsh Columbia V6V 2A4 (CA); Salari, Hassan, Tsawwassen, Britsh Columbia V4M 2K2 (CA); Wong, Donald, Vancouver, Britsh Columbia V5R 1C7 (CA)
(74) Representative: Kritzenberger & Zeuner

(57) **Abstract**

The present invention teaches compositions and uses of mimetics of IL-8 in the diagnosis, prevention, treatment, and ameliorization of symptoms of a variety of diseases.

## Description

### CROSS-REFERENCE

This application is a continuation-in-part of prior U.S. Patent Application No. 10/243,795, filed September 13, 2002, which is hereby incorporated herein in its entirety by reference.

### SEQUENCE LISTING

The instant application contains a "lengthy" sequence listing which has been submitted as a CD-R in lieu of a printed paper copy and is hereby incorporated by reference in its entirety. The CD-R versions, recorded on May 3, 2005, are labeled CRF, "Copy 1", and "Copy 2", respectively, and each contains only one identical 2.06 MB file (59296007.APP).

### BACKGROUND

### Field of the Invention

This invention relates to mimetics of the human chemokine interleukin-8.

### Description of the State-of-the-Art

Interleukin-8 (IL-8) is a chemokine that is responsible for the recruitment and activation of leukocytes and represents one of the several endogenous mediators of the acute inflammatory response. Until recently it was variously termed neutrophil-activating factor, monocyte-derived neutrophil chemotactic factor, neutrophil-activating peptide-1 and, of course, IL-8. The term "IL-8" has gained the widest acceptance and will be used herein.

The most abundant naturally occurring form of the IL-8 monomer is a 77 residue protein derived by processing of a 99-residue precursor. Other proteins with related sequences, including neutrophil-activating peptide-2 and GROα (with melanoma growth stimulatory activity) are IL-8 homologues, which have neutrophil-activating properties.

The IL-8 chemokine is a member of the chemokine super-family. Chemokines are divided into the following two functional classes: alpha (α) and beta (β). The members of each class share an organizing primary sequence motif. Members of the α class are potent chemoattractants, activators for leukocytes such as neutrophils, and have a C-X-C motif, which means that the first two cysteine residues are separated by an intervening residue. Members of the β class are potent chemoattractants, activators of monocytes, and have a C-C motif. Although these two families of chemokines have similar structures, they have a fairly low sequence homology (30-35%). The α class and β class are distinctive in their functions in that members of the α class cannot activate monocytes, and members of the β class have no effect on neutrophils. The IL-8 chemokines are members of the α class.

The *in vitro* effects of IL-8 on leukocytes such as neutrophils are similar to those of other chemotactic agonists such as C5a and fMet-Leu-Phe. The *in vitro* effects include induction of a transient rise in cytosolic free calcium; release of granules containing degradative enzymes such as elastase; burst of respiratory H₂O₂; change in neutrophil shape; and chemotaxis. The IL-8 chemokines appear to bind to at least one class of receptor sites on neutrophils with a frequency of approximately 64,000/cell and a K_{d} of 0.2 nM.

The three-dimensional structure of IL-8 is known by two-dimensional NMR and x-ray diffraction techniques. The IL-8 monomer has antiparallel P strands followed by a single overlying C-terminal α helix. Two disulfide bridges, between cysteine 7 and 34, and between cysteines 9 and 50 seem to stabilize the tertiary structure. Residues 1-6 and the loop residues 7-18 seem to have little defined secondary structure. The IL-8 is a noncovalent homodimer in solution and is stabilized primarily by interactions between the β strands of the two monomers. Examination of the three-dimensional structure indicates that following the cysteine at position 50, the residues form a type 1β turn (at residues 51 through 55) followed by an amhipatic α helix (at residues 55 through 72) that transverses the β sheet. Some of the interactions are between the two subunits of the dimeric molecule.

The IL-8 chemokines have shown both anti-tumor and anti-infective therapeutic activity such as, for example, by reducing the regression of macroscopic tumors in a model of peritoneal carcinomatosis in the rat. In this model, IL-8 was shown to recruit polymorphonuclear leukocyte (PMN) to the challenge site but did not enhance PMN infiltration of the tumor or the cytotoxic activity ofPMN. Regardless, it did have significant therapeutic activity, which may be secondary to PMN cytotoxicity and associated with other intermediate cells. It is suggested that lymphocytes could be involved since IL-8 has also demonstrated an ability to stimulate T-cell chemotaxis. *See* Lejeune, P., *et al.* Cancer Immunol. Immuno. 38:167-170(1994). Similarly, Interleukin-8 has shown therapeutic activity in non-neutropenic mice that received IL-8 shortly before challenge and at the site of infectious challenge with either *P. aeuginosa, Klebsiella-phenumoniae,* or *Plasmodium-berghei. See* Vogels, M. T., *et al.,* Antimicrob-Agents-Chemother. 37:276-280(1993).

The IL-8 chemokine has been produced through chemical synthesis and recombinant DNA methods. See Clark-Lewis, *et al.*, Biochemistry 30:3128-3135(1991); and Herbert, *et al.*, J. Biol. Chem. 286:18989-18994(1991), respectively.

Chemokines and their receptors have received increasing attention in the last few years as a result of their participation in many pathological conditions such as inflammation and conditions associated with autoimmune response. Accordingly, one of skill in the art would benefit from the identification of compositions that comprise IL-8 mimetics that can be administered in the treatment of disease and amelioration of one or more symptoms of a disease.

### SUMMARY

Embodiments of the present invention generally encompass compositions comprising IL-8 mimetics. In some embodiments, the invention provides methods for treating a disease in a subject, and/or ameliorating one or more symptoms thereof, comprising administering to the subject a composition comprising an effective amount of an IL-8 mimetic.

The compositions generally include a peptide sequence Glu-Leu-Arg and some embodiments also include a linker. The compositions comprising the IL-8 mimetics can be administered in a variety of ways including, but not limited to, alone, as part of a combination therapy with other agents, and/or in a codrug form.

In some embodiments, the invention provides a method comprising contacting a cell with an IL-8 mimetic, wherein the contacting comprises combining an IL-8 mimetic with a cell *in vitro.* In other embodiments, an article of manufacture is provided and includes a composition comprising an IL-8 mimetic and instructions for administering the composition to a subject and monitoring the subject. The articles of manufacture also provide for the administration of a second agent as part of a combination treatment and/or as a codrug.

### BRIEF DESCRIPTION OF THE FIGURES

FIG. I shows the CXCR2 receptor binding of the IL-8 mimetics as competing ligands.

FIG. 2 shows the response of circulating neutrophil counts to the administration of varying doses of the IL-8 mimetic a 161 (SEQ ID NO:1647) ("the test mimetic") following one hour of treatment.

FIG. 3 describes the kinetics of the rise in circulating neutrophil counts in response to the administration of the test mimetic.

FIG. 4 shows the response of circulating haematopoietic progenitor/stem cells to the administration of varying doses of the test mimetic.

FIG. 5 shows the induction of a rise in haematopoietic progenitor / stem cells in response to the administration of the test mimetic.

### DETAILED DESCRIPTION OF THE INVENTION

The present application is a continuation-in-part of U.S. Patent Application No. 10/243,795, which is hereby incorporated herein in its entirety by reference. The present invention is directed toward the compositions of and uses for new mimetics of the human CXC chemokine IL-8. The chemokine mimetics of the present invention are referred to as "IL-8 mimetics." For purposes of the present invention, the IL-8 mimetics can be analogs, homologs, prodrugs, codrugs, metabolites, congeners, variants, salts, and combinations thereof, of truncated forms of the human CXC chemokine IL-8.

The term "variant" refers to modifications to a peptide that allows the peptide to retain its binding properties, and such modifications include, but are not limited to, conservative substitutions in which one or more amino acids are substituted for other amino acids; deletion or addition of amino acids that have minimal influence on the binding properties or secondary structure; conjugation of a linker; post-translational modifications such as, for example, the addition of functional groups. Examples of such post-translational modifications can include, but are not limited to, the addition of modifying groups described below through processes such as, for example, glycosylation, acetylation, phosphorylation, modifications with fatty acids, formation of disulfide bonds between peptides, biotinylation, PEGylation, and combinations thereof. The term "conservatively modified variant" refers to a conservative amino acid substitution, which is an amino acid substituted by an amino acid of similar charge density, hydrophilicity/hydrophobicity, size, and/or configuration such as, for example, substituting valine for isoleucine. In comparison, a "non-conservatively modified variant" refers to a non-conservative amino acid substitution, which is an amino acid substituted by an amino acid of differing charge density, hydrophilicity/hydrophobicity, size, and/or configuration such as, for example, substituting valine for phenyalanine.

The IL-8 mimetics can be designed to include a wide variety of modifications to provide a diagnostic, therapeutic and/or prophylactic effect in the treatment of a disease or ameliorization of one or more symptoms of a disease in a subject. The term "subject" and "patient" are used interchangeably in the present invention and refer to an animal such as a mammal including, but not limited to, non-primates such as, for example, a cow, pig, horse, cat, dog, rat and mouse; and primates such as, for example, a monkey or a human.

The IL-8 mimetics can be used diagnostically, prophylactically, therapeutically, or to produce an ameliorative effect in a wide variety of diseases. The cells that are affected by the IL-8 mimetics include, but are not limited to, endothelial cells, leukocytes, red blood cells, megakaryocytes, stem cells, progenitor cells, and combinations thereof.

Endothelial cells are the main type of cell found in the inside lining of blood vessels, lymph vessels, and the heart. Leukocytes are white blood cells and are classified in two groups: granulocytes and agranulocytes. The granulocytes are neutrophils, eosinophils, and basophils, whereas the agranulocytes are lymphocytes and monocytes. The neutrophils represent 60-70% of all leukocytes, and lymphocytes represent 20-25% of all leukocytes. Red blood cells are the most common type of blood cell and are the principal means of delivering oxygen to body tissues through the blood. Red blood cells are also known as erythrocytes. Megakaryocytes are multinucleated cells that live in the bone marrow and generate blood platelets. Stem cells and progenitor cells are self-renewing cells. Stem cells have the ability to proliferate and differentiate into cell types of different tissues *in vitro* and *in vivo,* whereas progenitor cells are more limited. Many of these cell types are discussed in more detail herein in their respective sections.

The therapeutic and prophylactic effects of IL-8 mimetics can include, but are not limited to, one or more of the following: (a) an increase or decrease in the number of cells present at a specified location; (b) an increase or decrease in the ability of cells to migrate; (c) an increase or decrease in the response of cells to a stimulus; (d) an increase or decrease in the proliferation, growth, and/or differentiation of cells; (e) an inhibition or acceleration of apoptosis; (f) an ameliorization of one or more symptoms of disease; (g) an enhancement or inhibition of a cell function; and (h) an activation or inhibition of enzyme activity in cells.

An increase in the number of cells is "cell expansion," and the IL-8 mimetics can cause this increase to occur *in vivo* or *ex vivo.* Accordingly, the IL-8 mimetics can be used to create mammal cells for diagnostic, prophylactic, therapeutic applications as well as commercial applications that include, but are not limited to, medicinal research and development of products such as, for example, vaccines.

A function or activity of a chemokine receptor can be controlled by contacting the receptor with an IL-8 mimetic to increase or decrease the probability of forming a complex between the receptor and a natural binding partner. The term "natural binding partner" includes, but is not limited to, G proteins, polypeptides, lipids, small molecules, or nucleic acids that bind to chemokine receptors in cells or in the extracellular environment. The term natural binding partner also includes substrates that can be acted upon by a chemokine receptor.

In some embodiments, an IL-8 mimetic can increase the probability of forming a complex between a chemokine receptor and a natural binding partner. In other embodiments, an IL-8 mimetic can decrease the probability of forming a complex between a chemokine receptor and a natural binding partner. In other embodiments, the concentration of an IL-8 mimetic can control the probability that a complex between a chemokine receptor and a natural binding partner will form. The term "complex" refers to a binding between at least two molecules. In one example, a signal transduction complex contains at least two protein molecules bound to one another. In another example, a receptor-protein tyrosine kinase, an adaptor protein GRB2, son of sevenless protein (Sos), and G proteins Ras and Ras can assemble to form a signal transduction complex in response to a mitogenic ligand. In another example, a chemokine mimetic can be bound to a chemokine receptor. In another example, a G protein can be bound to a chemokine receptor.

In some embodiments, an IL-8 mimetic activates the catalytic activity of a chemokine receptor. In other embodiments, an IL-8 mimetic inhibits the catalytic activity of a chemokine receptor. In other embodiments, the activation or inhibition of a chemokine receptor can be dependent on the concentration of an IL-8 mimetic. The term "activate" refers to increasing a cellular or extracellular function of a chemokine receptor.

Examples of diseases that can be affected by the IL-8 mimetics include, but are not limited to, autoimmune disorders, inflammatory disorders, organ transplant rejections, disorders associated with the treatment of cancer, cardiovascular disorders, hematological disorders, neurological disorders, and infectious disorders. In some embodiments, IL-8 mimetic compositions can be administered in a therapeutically or prophylactically effective amount that to increase the hemocrit, assist in mobilizing and recovering stem cells or progenitor cells, stimulate the production of blood cells, assist in vaccine production, or assist in gene therapy. The term "hemocrit" refers to the number red cells in a unit of blood. An average hemocrit ranges from about 4.2 to about 5.9 million red cells per microliter of blood.

Due to a traditional view that cells cannot be replaced, current treatments have focused on preventing cell death, but evidence has shown that some cells have the ability to regenerate. The recent derivation of human stem cells and progenitor cells has catapulted cell replacement therapy as a real alternative to current treatments such as, for example, treatments for neurodegenerative and other diseases. The term "stem cell" refers to a cell that can be self-renewing over a prolonged time period and can generate multiple phenotypes in response to an exogenous signal. The term "progenitor cell" refers to a cell that is more restricted in its differentiation capability and undergoes only limited self-renewal. In one example, an eosinophil progenitor must become an eosinophil - it can't become a neutrophil, although both are granulocytes.

Due to the belief that brain and spinal cord cells cannot be replaced, treatments of neurological disorders have, as described above, focused on preventing neuronal death. Evidence has proven that some cells of the central nervous system such as, for example, neuronal stem cells, can regenerate. The term "neuronal stem cell" refers to cells that can self-renew, give rise to other cells through asymmetric cell division/differentiation, generate neural tissue, or are derived from the nervous system. Neuronal stem cells exist in a post-implantation developing mammalian nervous system as fetal stem cells and in the adult nervous system as adult stem cells. Neurones and neuronal progenitors can also be derived from the more primitive embryonic stem cell of the inner cell mass (ICM) of the pre- or peri-implantation embryo. In another example, progenitor cells are found in an adult mammalian spinal cord and can be manipulated to mature into all of the major cell types that are found in the brain and spinal cord such as, for example, neurons and glial cells. These progenitor cells can be manipulated within the brain and spinal cord by administering growth factors.

The term "embryonic stem cell" refers to a primitive form of cell that can be isolated from an embryo days after conception, is unformed and unprogrammed, and has an innate ability to develop into *any* other type of cell in the body such as, for example, new brain cells, insulin-producing pancreas cells, heart muscle, and other tissues that could take the place of damaged or diseased cells. Accordingly, the implanting of embryonic stem cells can create not only neurons but also cells, tissues and organs of all other systems. When implanted into degenerating areas of the brain, for example, these cells have the capacity to acquire relevant cellular phenotype of the degenerated region and take on functions of cells that were lost through disease.

Autoimmune disorders can be organ-specific or systemic and are provoked by different pathogenic mechanisms. Examples of organ-specific autoimmune disorders include, but are not limited to, diabetes, hyperthyroidism, autoimmune adrenal insufficiency, pure red cell anemia, multiple sclerosis, and rheumatic carditis. Examples of systemic autoimmune diseases include systemic lupus erythematosus, rheumatoid arthritis, chronic inflammation, Sjogren's syndrome polymyositis, dermatomyositis and scleroderma. In some embodiments, a treatment of an autoimmune disorder can include administering a composition comprising an IL-8 mimetic. In other embodiments, a treatment of an autoimmune disorder can include administering a composition comprising an IL-8 mimetic before, during or after administering one or more other agents that are diagnostic, bioactive, biobeneficial, or a combination thereof.

Inflammatory disorders are manifested by an inflammatory response, which is initiated by injury that can be a result of trauma, ischemia or the introduction of foreign particles; and infection, which can be bacterial or viral. Inflammation includes a complex series of events including chemical mediators such as cytokines and prostaglandins; and inflammatory cells such as, for example, leukocytes.

The inflammatory response is a delicate interplay between the humoral and cellular immune elements that enables elimination of harmful agents and initiation of tissue repair. Unfortunately, the inflammatory response can be a disorder in its own right by causing considerable, and potentially more, damage to tissue than the disorder that initiated the inflammatory response. Examples of inflammatory disorders include, but are not limited to, acute and chronic inflammatory diseases such as, for example, arthritis, atheromas, colitis, chronic inflammatory bowel disease, chronic inflammatory pelvic disease, asthma, psoriasis, and rhinitis. Current treatments for inflammatory disorders include the use of non-steroidal anti-inflammatory drugs, which can cause, *inter alia,* gastrointestinal side effects; corticosteroids, which can cause, *inter alia,* an increased risk of infection; and immunosuppressive agents, which can leave a subject defenseless to infections.

In some embodiments, a treatment of an inflammatory disorder can include administering a composition comprising an IL-8 mimetic. In other embodiments, a treatment of an inflammatory disorder can include administering a composition comprising an IL-8 mimetic, either an agonist or antagonist, before, during or after administering one or more other agents. In these embodiments, the IL-8 mimetic can contain the N-terminal portion of the IL-8 and the C-terminal portion of the IL-8 connected by a linker.

Transplant rejections occur in recipients receiving tissue from donors that differ genetically, and such rejections are mediated by T-cell dependent mechanisms. Immunosuppressive agents such as calcineurin phosphatase inhibitors and glucocorticosteroids are administered to transplant recipients to prevent allograft rejection. Immunosuppressive agents have a short lasting effect, so transplant recipients normally require life-long treatment with such agents. Life-long treatment with immunosuppressive agents creates serious adverse effects in a recipient such as, for example, the development of infections and tumors.

The IL-8 mimetics of the present invention can be administered to transplant recipients to modulate cellular response and achieve effects that are diagnostic, therapeutic, prophylactic, ameliorative or a combination thereof. In some embodiments, a treatment of a transplant rejection can include administering a composition comprising an IL-8 mimetic. In other embodiments, a treatment of a transplant rejection can include administering a composition comprising an IL-8 mimetic before, during or after administering one or more other agents. In these embodiments, the IL-8 mimetic can contain the N-terminal portion of the IL-8 and the C-terminal portion of the IL-8 connected by a linker.

Cardiovascular disease is a broad, all-encompassing term that refers to a collection of diseases and conditions and is any disorder of the heart and blood vessels. Examples of cardiovascular disorders and treatments include, but are not limited to, any disease involving heart or vascular tissue such as, for example, atherosclerosis, and ischemic heart or vascular tissue requiring reconstruction and/or management. Traditional therapies used to treat cardiovascular disease have used either angioplasty procedures to compress blockages in arteries or coronary artery bypass grafts to provide an alternate path for blood flow around clogged vascular passageways. A latest generation of treatments includes "therapeutic angiogenesis," which is an inducement of angiogenesis to produce new blood vessels that supplement or replace a diseased vascular passageway.

In some embodiments, a treatment of a cardiovascular disorder can include administering a composition comprising an IL-8 mimetic to modulate angiogenesis and assist in the reconstruction of heart or vascular tissue, which can include the proliferation and/or mobilization of endothelial cells such as, for example, vascular endothelial cells. In other embodiments, a treatment of a cardiovascular disorder can include administering compositions comprising an IL-8 mimetic before, during or after administering one more other agents. In these embodiments, the IL-8 mimetic can contain the N-terminal portion of the IL-8 and the C-terminal portion of the IL-8 connected by a linker.

The IL-8 mimetics can be used to treat conditions associated with cancer. Examples of conditions associated with cancer treatments such as, for example chemotherapy and radiotherapy and include, but are not limited to, a diverse group of hematopoietic stem cell disorders known as myelodysplastic syndrome (MDS). Such disorders are characterized by a cellular marrow with an impaired morphology and maturation (dysmyelopoiesis), peripheral blood cytopenias, and a variable risk of progression to acute leukemia that results from an ineffective blood cell production. *See* The Merck Manual 953, (17^{th} ed. 1999).

These disorders can result from an initial hematopoietic stem cell injury that can be from a variety of causes including, but not limited to, cytotoxic chemotherapy, radiation therapy, virus, chemical exposure and genetic predisposition. A clonal mutation predominates over bone marrow and suppresses healthy stem cells. In the early stages of MDS, for example, a main cause of cytopenia is an increase in programmed cell death, or apoptosis. As the disease progresses and converts into leukemia, gene mutation rarely occurs and a proliferation of leukemic cells overwhelms the healthy marrow. The course of the disease can vary, and some cases can behave as an indolent disease, whereas others can behave aggressively with a short clinical progression into an acute form of leukemia.
Subjects that survive a malignancy treatment with chemotherapy drugs such as, for example, alkylating agents, with or without radiotherapy, can have a high incidence of developing MDS or secondary acute leukemia.

Examples of treatments for MDS include, but are not limited to, bone marrow transplantation, transfusions, and administration of hematopoietic growth factors and cytokines. Since the IL-8 mimetics are chemokines, they are part of the family of cytokines. In some embodiments, a treatment of a condition associated with cancer can include the administrating a composition comprising an IL-8 mimetic before, during or after chemotherapy or radiotherapy. In other embodiments, a treatment of a condition associated with cancer can include administering a composition comprising an IL-8 mimetic before, during or after administering one more other treatments or agents. In one example, an IL-8 mimetic can be administered as an antagonist to inhibit or prevent the proliferation of cancer cells. In another example, an IL-8 mimetic can be administered as an agonist during recovery from chemotherapy and/or radiotherapy to expedite recovery of the blood count. In these embodiments, the IL-8 mimetic can contain the N-terminal portion of the IL-8 and the C-terminal portion of the IL-8 connected by a linker.

Examples of other hematological disorders include, but are not limited to, bone marrow depression, aplastic anemia, agranulocytosis, leukopenia, pancytopenia, thrombocytopenia, macrocytic or megaloblastic anemia. In some embodiments, a treatment of a hematological disorder can include managing white blood cells, platelets, red blood cells, stem cells and various progenitor subsets by administering a composition comprising an IL-8 mimetic. In one example, an IL-8 can be administered as an agonist to increase the proliferation of blood cells and/or mobilize the blood cells into the bloodstream. In other embodiments, a treatment of a hematological disorder can include administering a composition comprising an IL-8 mimetic before, during or after administration of one or more other treatments or agents. In these embodiments, the IL-8 mimetic can contain the N-terminal portion of the IL-8 and the C-terminal portion of the IL-8 connected by a linker.

Examples of neurological disorders include, but are not limited to, Parkinson's disease, Alzheimer's disease, multiple sclerosis, and any other conditions associated with neuronal stem cells. Parkinson's disease, for example, can be treated with limited success in some cases by drugs including, but not limited to, L-beta-3,4-dihydroxyphenylalanine hydrochloride (L-Dopa). Alzheimer's disease, for example, is irreversible despite treatments with drugs including, but not limited to, ARICEPT®. Such treatments have had limited success in ameliorating symptoms and slowing progression of the disease, but most patients with Alzheimer's disease require some sort of palliative care. Multiple sclerosis, for example, is sometimes responsive to the anti-inflammatory drugs including, but not limited to, β-interferon, but patients with multiple sclerosis remain incurable over the long term. As a result, subjects with multiple sclerosis develop permanent motor, sensory and cognitive deficits. In general, the treatment and reversal of neurodegenerative diseases of the brain and spinal cord remains a formidable challenge.

In some embodiments, a treatment of a neurological disorder can include administration of a composition comprising an IL-8 mimetic. In other embodiments, a treatment of a neurological disorder can include administering a composition comprising an IL-8 mimetic before, during or after administering one more other treatments or agents. In one example, an IL-8 mimetic can be administered as an agonist to mobilize stem cells in the treatment of a disease. In these embodiments, the IL-8 mimetic can contain the N-terminal portion of the IL-8 and the C-terminal portion of the IL-8 connected by a linker.

### The IL-8 Mimetics

The IL-8 mimetics correspond to modifications of a native IL-8 chemokine and include, but are not limited to, modifications of an N-terminus; modifications of a C-terminus; modifications of an internal region; modifications of an N-terminal region containing a sequence Glu-Leu-Arg; modifications of an internal region containing three antiparallel β-sheets in the structure; modifications of a C-terminal region containing an α-helical structure; modifications of a combination of N-terminal and C-terminal regions; combinations of these modifications linked together either directly or through a linker; combinations of N-terminal and internal regions and modifications thereof; combinations of internal and C-terminal regions and modifications thereof; combinations of N-terminal, internal and C-terminal regions and modifications thereof; and combinations thereof. In some embodiments, the N-terminal region of each sequence must include a sub-sequence of Glu-Leu-Arg, and each C-terminal region must include a sub-sequence of residues 56-71 (SEQ ID NO:26).

In some embodiments, the IL-8 mimetics may be created by either directly or indirectly connecting at least one modifying group. The term "modifying group" refers to any functional group composing a portion of an IL-8 mimetic that was either absent in the native IL-8 chemokine or that comprises an isolated sequence of less than four amino acids. Such sequences are "isolated" in that they are positioned differently in the IL-8 mimetic than they were positioned in the native IL-8 chemokine. A modifying group can also be a linker, and linkers are described below.

A modifying group can be connected, for example, to the N-terminus or C-terminus of a peptide; to a peptidic or peptidomimetic region flanking the core domain; to a side chain of at least one amino acid residue such as, for example, an ε-amino group of a lysyl residue, a carboxyl group of an aspartic acid or glutamic acid residue, a hydroxy group of a tyrosyl, serine or threonine residue, or other suitable reactive group on an amino acid side chain; or in-chain as a linker. Examples of chemical connections used to attach the modifying groups include, but are not limited to, ether, amide, ester, anhydride, orthoester, alkylamine, sulphide, disulphide, carbamate, all-aromatic carbonate, urea bonds, and the like.

In general, a modifying group can include any of the functional groups described below. In addition, the functional group may also be a "biotinyl structure", which includes biotinyl groups and analogues and derivatives thereof. Examples of biotinyl structures include, but are not limited to, iminiobiotinyl structures such as, for example, a 2-iminobiotinyl group.

In some embodiments, the modifications can control the pharmacokinetic or pharmacodynamic properties of an IL-8 mimetic without substantially reducing its bioactive function. In other embodiments, the modifications can alter *in vivo* stability, bioavailability, or half-life of a mimetic. In other embodiments, the modifications can provide a diagnostic capability such as, for example, by creating a means of detecting the presence or location of a mimetic *in vivo* or *in vitro.* Examples of detectable substances are described below.

In other embodiments, the IL-8 mimetics can act as an agonist or an antagonist to a native IL-8 chemokine. The agonistic activities of the IL-8 mimetics includes, but are not limited to, mimicking or inhibiting the biological activity of the native IL-8 chemokine. The term "biological activity" refers to the biological actions of the native IL-8 chemokine and its mimetics. In some embodiments, the biological actions may be determined by performing standard assays of, for example, receptor binding, white blood cell mobilization, other forms of chemotaxis, and other measurable biological actions related to the presence or absence of a native IL-8 chemokine or mimetic.

The amino acids are identified in the present application by the following conventional three-letter abbreviations. The single letter identifier is provided for ease of reference. The three-letter abbreviations are generally accepted in the peptide art, recommended by the IUPAC-IUB commission in biochemical nomenclature, and are required by WIPO Standard ST.25:

| | | | | | |
|---|---|---|---|---|---|
| Alanine | A | Ala | Leucine | L | Leu |
| Arginine | R | Arg | Lysine | K | Lys |
| Asparagine | N | Asn | Methionine | M | Met |
| Aspartic acid | D | Asp | Phenylalanine | F | Phe |
| Cysteine | C | Cys | Proline | P | Pro |
| Glutamic acid | E | Glu | Serine | S | Ser |
| Glutamine | Q | Gln | Threonine | T | Thr |
| Glycine | G | Gly | Tryptophan | W | Trp |
| Histidine | H | His | Tyrosine | Y | Tyr |
| Isoleucine | I | Ile | Valine | V | Val |
| Omithine | O | Om | Other | | Xaa |

Furthermore, the peptide sequences are taught according to the generally accepted convention of placing the N-terminus on the left and the C-terminus on the right of the sequence listing as required by WIPO Standard ST.25.

The functional groups of the present invention can be independently selected from substituted, unsubstituted, hetero-, straight-chained, branched, cyclic, saturated or unsaturated aliphatic radical; or a substituted, unsubstituted, or hetero- aromatic radicals. For example, a functional group can be selected from H; aliphatic hydrocarbon groups such as, for example, alkyl, alkenyl, and alkynyl groups; aromatic groups such as, for example, aryl, aralkyl, aralkenyl, and aralkynyl groups; and, various other groups as defined below.

In some embodiments of the present invention, the aliphatic radicals have from about 1 to about 50 carbon atoms, from about 2 to about 40 carbon atoms, from about 3 to about 30 carbon atoms, from about 4 to about 20 carbon atoms, from about 5 to about 15 carbon atoms, from about 6 to about 10 carbon atoms, and any range therein. In some embodiments, the aromatic radicals have from about 6 to about 180 carbon atoms, from about 12 to about 150 carbon atoms, from about 18 to about 120 carbon atoms, from about 24 to about 90 carbon atoms, from about 30 to about 60 carbon atoms, and any range therein.

The term "alkyl" can be used interchangeably with the term "alkylene" in some contexts and refers to a straight-chained or branched hydrocarbon chain. Examples of alkyl groups include lower alkyl groups such as, for example, methyl, ethyl, n-propyl, iso-propyl, n-butyl, iso-butyl, t-butyl or iso-hexyl; upper alkyl groups such as for example, n-heptyl, n-octyl, iso-octyl, nonyl, decyl, and the like; lower alkylene such as, for example, ethylene, propylene, butylenes, butadiene, pentene, n-hexene and iso-hexene; and upper alkylene such as, for example, n-heptene, n-octene, iso-octene, nonene, decene, and the like. Persons of ordinary skill in the art are familiar with numerous straight-chained and branched alkyl groups, which are within the scope of the present invention. In addition, such alkyl groups may also contain various substituents in which one or more hydrogen atoms can be replaced by a functional group, or the alkyl groups can contain an in-chain functional group.

The term "alkenyl" refers to a straight-chained or branched hydrocarbon chain where at least one of the carbon-carbon linkages is a carbon-carbon double bond. The term "alkynyl" refers to a straight-chained or branched hydrocarbon chain where at least one of the carbon-carbon linkages is a carbon-carbon triple bond.

The term "aryl" refers to a hydrocarbon ring bearing a system of conjugated double bonds often comprising at least six π (pi) electrons. Examples of aromatic groups include, but are not limited to, phenyl, pyrrolyl, furyl, thiophenyl, imidazolyl, oxazole, thiazolyl, triazolyl, pyrazolyl, pyridyl, pyrazinyl, pyridazinyl and pyrimidinyl, naphthyl, anysyl, toluyl, xylenyl, and the like. The term "aralkyl" refers to an alkyl group substituted with at least one aryl group. Examples of aralkyls include substituted benzyls such as, for example, phenylmethyl, 2-naphthylethyl, 2-(2-pyridyl) propyl, 5-dibenzosuberyl, and the like. The term "aralkenyl" refers to an alkenyl group substituted with at least one aryl group. Those aryl groups having heteroatoms in the ring structure may also be referred to as "aryl heterocycles" or "heteroaromatics." The aromatics can be substituted at one or more ring positions and can also be part of a polycyclic group. For example, aryl groups can include fused aromatic moieties such as naphthyl, anthracenyl, quinolyl, indolyl, and the like.

The phrase "straight-chained or branched" includes any substituted or unsubstituted acyclic carbon-containing compounds including, but not limited to, alkanes, alkenes and alkynes. A radical is "straight-chained" when it has less than 0.1 mole percent of sidechains having 1 or more carbon atoms. In some embodiments, a radical is straight-chained if it has less than 0.01 mole percent of such sidechains. In other embodiments, a radical is straight-chained if it has less than 0.001 mole percent of such sidechains. A radical is "branched" when it has more than 0.1 mole percent of sidechains having 1 or more carbon atoms. In some embodiments, a radical is branched when it has more than 0.01 mole percent of such sidechains. In other embodiments, a radical is branched when it has more than 0.001 mole percent of such sidechains.

The terms "radical," "group," "functional group," and "substituent" can be used interchangeably in some contexts and can be used together to further describe a chemical structure. For example, the term "functional group" can refer to a chemical "group" or "radical," which is a chemical structure variable that is in-chain, pendant and/or terminal to the chemical structure. In some embodiments, a straight chain or branched alkyl has from about 1 to about 20 carbon atoms, from about 2 to about 18 carbon atoms, from about 3 to about 17 carbon atoms, from about 5 to about 15 carbon atoms, from about 2 to about 10 carbon atoms, or any range therein. In other embodiments, a cycloalkyl may have a ring structure containing from about 2 to about 12 carbon atoms, from about 3 to about 11 carbon atoms, from about 4 to about 10 carbon atoms, or any range therein.

A functional group may comprise a cyclic or polycyclic group. The term "cyclic group" refers to a ring structure that can be substituted, unsubstituted, hetero-, saturated or unsaturated and have from 3 to 24 carbon atoms, from 3 to 18 carbon atoms, from 3 to 12 carbon atoms, or any range therein. Examples of cyclic groups include, but are not limited to, cycloalkyls such as cyclopropyl, cyclobutyl, cyclopentyl, cyclohexyl, and cyclooctyl structures; cycloalkenes; and aromatics. The term "polycyclic group" refers to two or more substituted, unsubstituted, hetero-, saturated or unsaturated cyclic rings in which two or more ring carbons are common among two adjoining rings such that the rings are "fused rings." The rings can also be "bridged rings" in that they are joined through atoms that are not common among the adjoining rings.

The term "substituted" is used to characterize a chemical structure that has been modified by the addition of at least one functional group to at least one position that can be in-chain, pendant, and/or terminal to the chemical structure. The terms "radical," "group," "functional group" and "substituent" can be used interchangeably in some contexts to describe a chemical that has been added to another chemical to modify its structure. In some embodiments, the functional groups can include, but are not limited to, aliphatics, aromatics, and combinations thereof; alkyls, alkenes, alkynes, cyclic structures, heterocyclic structures, and combinations thereof.

In other embodiments, the functional groups can include, but are not limited to, oxygen-containing groups such as, for example, alcohols, ethers, phenols, and derivatives thereof. Such oxygen-containing groups include, but are not limited to, acetonides, alcohols, alkoxides, bisphenols, carbinols, cresols, diols, enols, enolates, epoxides, ethers, glycols, hydroperoxides, peroxides, phenols, phenolates, phenoxides, pinacols, trioxides, and ynols.

In other embodiments, the functional groups can include, but are not limited to, oxygen-containing groups such as, for example, aldehydes, ketones, quinones and derivatives thereof. Such oxygen-containing groups include, but are not limited to, acetals, acyloins, aldehydes, carbonyl compounds, diosphenols, dypnones, hemiacetals, hemiketals, ketals, ketenes, keto compounds, ketones, quinhydrones, quinomethanes, quinines, and combinations thereof.

In other embodiments, the functional groups can include, but are not limited to, oxygen-containing groups such as, for example, carboxylic acids and derivatives thereof. Such oxygen-containing groups include, but are not limited to, carboxylic acids, oxoacids, sulfonic acids, acid anhydrides, acid thioanhydrides, acyl groups, acyl halides, acylals, anhydrides, carboxylic acids, cyclic acid anhydrides, cyclic anhydrides, esters, fulgides, lactides, lactols, lactones, macrolides, naphthenic acids, ortho acids, ortho esters, oxo carboxylic acids, peroxy acids, and combinations thereof,

In other embodiments, the functional groups can include, but are not limited to, nitrogen-containing groups containing one nitrogen such as, for example, aldimines, aldoximes, alkoxyamines, amic acids, amides, amines, amine oxides, amine ylides, carbamates, hemiaminals, carbonitriles, carboxamides, isocyanides, cyanates, isocyanates, diisocyanates, cyanides, cyanohydrins, diacylamines, enamines, fulminates, hemiaminals, hydroxamic acids, hydroximic acids, hydroxylamines, imides, imidic acids, imidines, imines, oximes, isoureas, ketenimines, ketimines, ketoximes, lactams, lactims, nitriles, nitro, nitroso, nitrosolic acids, oxime O-ethers, quaternary ammonium compounds, quinone imines, quinonoximes, azomethines, ureides, urethanes, and combinations thereof.

In other embodiments, the functional groups can include, but are not limited to, nitrogen-containing groups containing two or more nitrogens such as, for example, aldazines, amide hydrazones, amide oximes, amidines, amidrazones, aminals, amine imides, amine imines, isodiazenes, azans, azides, azo imides, azines, azo compounds, azomethine imides, azoxy compounds, carbodiimides, carboxamidines, diamidides, diazo compounds, diazoamino compounds, diazoates, diazooxides, formamidine disulfides, formazans, hydrazides, hydrazide hydrazones, hydrazide imides, hydrazidines, hydrazines, hydrazo compounds, hydrazones, ketazines, nitramines, nitrile imines, nitrimines, nitrolic acids, nitrosamides, nitrosamines, nitrosimines, ortho amides, semicarbazones, semioxamazones, triazanes, triazenes, and combinations thereof.

In other embodiments, the functional groups can include, but are not limited to, sulfur-containing groups such as thio, thiol, thioether, sulfonyl, sulfido, sulfinamides, sulfilimines, sulfimines, sulfimides, sulfinamidines, sulfines, sulfinic acids, sulfinic anhydrides, sulfinylamines, sulfonamides, sulfones, sulfonediimines, sulfonic acids, sulfonic anhydrides, sulfoxides, sulfoximides;

In other embodiments, the functional groups can include, but are not limited to, silyl groups, halogens, selenoethers, trifluoromethyls, thio-derivatives of urethanes where at least one oxygen atom is replaced by a sulfur atom; phosphoryls, phosphonates, phosphinates; and ethyleneically unsaturated groups such as, for example, allyl, acryloyl and methacrylol, and maleate and maleimido; and combinations thereof.

Examples of heteroatoms of the hetero- radicals include, but are not limited to, sulfur, phosphorous, oxygen, nitrogen and combinations thereof. Examples of heterocyclic groups include, but are not limited to, pyrrolidine, oxolane, thiolane, imidazole, oxazole, piperidine, piperazine, and morpholine. The heterocyclics may also be bridged or fused to other cyclic groups as described below.

In some embodiments, the modifying groups can include, but are not limited to, O-modified derivatives including, but not limited to, C-terminal hydroxymethyl benzyl ether, and other C-terminal hydroxymethyl derivatives; N-modified derivatives including, but not limited to, substituted amides such as alkylamides; hydrazides and compounds in which a C-terminal phenylalanine residue is replaced with a phenethylamide analogue such as, for example, by replacing Ser-Ile-Phe with Ser-Ile-phenethylamide.

In other embodiments, the functional group may include a fluorescein-containing group. Examples of fluorescein-containing groups include, but are not limited to, 5-(and 6-)-carboxyfluorescein succinimidyl ester and fluorescein isothiocyanate. In other embodiments, the modifying group may include a cholyl structure. An example of a cholyl derivative is 3-(O-aminoethyl-iso)-cholyl (Aic).

In other embodiments, the functional group may include N-acetylneuraminyl, trans-4-cotininecarboxyl, 2-imino-1-imidazolidineacetyl, (S)-(-)-indoline-2-carboxyl, 2-norbomaneacetyl, γ-oxo-5-acenaphthenebutyryl, (-)-2-oxo-4-thiazolidinecarboxyl group, tetrahydro-3-furoyl group, 4-morpholinecarbonyl group, 2-thiopheneacetyl group, 2-thiophenesulfonyl group, diethylene-triaminepentaacetyl group, (O)-methoxyacetyl group, N-acetylneuraminyl group, and combinations thereof. In other embodiments, the functional group may include light scattering groups, magnetic groups, nanogold, other proteins, a solid matrix, radiolabels, carbohydrates, and combinations thereof.

In other embodiments, the functional groups may include biobeneficial, bioactive, and/or diagnostic agents. A "bioactive agent" is a functional group that can be connected to the IL-8 mimetic to provide a therapeutic effect, a prophylactic effect, both a therapeutic and a prophylactic effect, or other biologically active effect. A "biobeneficial agent" is a functional group that can also be connected to an IL-8 mimetic to provide a biological benefit within a subject. In one example, a biobeneficial agent can be non-inflammatory, such as, for example, by acting as a biomimic to passively avoid attracting monocytes and neutrophils, which leads to the cascade of events creating inflammation.

A "diagnostic agent" is a type of bioactive agent that can be used, for example, in diagnosing the presence, nature, or extent of a disease or medical condition in a subject. In one embodiment, a diagnostic agent can be any agent that may be used in connection with methods for imaging an internal region of a patient and/or diagnosing the presence or absence of a disease in a patient. Diagnostic agents include, for example, contrast agents for use in connection with ultrasound imaging, magnetic resonance imaging (MRI), nuclear magnetic resonance (NMR), computed tomography (CT), electron spin resonance (ESR), nuclear medical imaging, optical imaging, elastography, radiofrequency (RF) and microwave laser. Diagnostic agents may also include any other agents useful in facilitating diagnosis of a disease or other condition in a patient, whether or not imaging methodology is employed.

In some embodiments, the biobeneficial agents can have a reactive group that can be used to connect the agent to an IL-8 mimetic. Examples of such reactive groups include, but are not limited to, hydroxyl, carboxyl, and amino groups. In other embodiments, the biobeneficial agents can remain attached to the IL-8 mimetic or be controllably released from the IL-8 mimetic.

In some embodiments, the molecular weight of an agent connected to an IL-8 mimetic should be at or below about 40,000 Daltons, or any range therein, to ensure elimination of the agent from a subject. In one embodiment, the molecular weight of the agent ranges from about 300 Daltons to about 40,000 Daltons, from about 8,000 Daltons to about 30,000 Daltons, from about 10,000 Daltons to about 20,000 Daltons, or any range therein. It is to be appreciated that one skilled in the art should recognize that some of the groups, subgroups, and individual biobeneficial agents may not be used in some embodiments of the present invention.

Examples of biobeneficial agents include, but are not limited to, many of the polymers listed above such as, for example, carboxymethylcellulose, poly(alkylene glycols), poly(N-vinyl pyrrolidone), poly(acrylamide methyl propane sulfonic acid), poly(styrene sulfonate), sulfonated dextran, polyphosphazenes, poly(orthoesters), poly(tyrosine carbonate), dermatan sulfate, hyaluronic acid, heparin and any derivatives, analogs, homologues, congeners, salts, copolymers and combinations thereof.

Examples of heparin derivatives include, but are not limited to, earth metal salts of heparin such as, for example, sodium heparin, potassium heparin, lithium heparin, calcium heparin, magnesium heparin, and low molecular weight heparin. Other examples of heparin derivatives include, but are not limited to, heparin sulfate, heparinoids, heparin-based compounds and heparin derivatized with hydrophobic materials.

Examples of poly(alkylene glycols) include, but are not limited to, PEG, mPEG, poly(ethylene oxide), poly(propylene glycol)(PPG), poly(tetramethylene glycol), and any derivatives, analogs, homologues, congeners, salts, copolymers and combinations thereof. In some embodiments, the poly(alkylene glycol) is PEG. In other embodiments, the poly(alkylene glycol) is mPEG. In other embodiments, the poly(alkylene glycol) is poly(ethylene glycol-co-hydroxybutyrate).

The copolymers that may be used as biobeneficial agents include, but are not limited to, any derivatives, analogs, homologues, congeners, salts, copolymers and combinations of the foregoing examples of agents. Examples of copolymers that may be used as biobeneficial agents in the present invention include, but are not limited to, dermatan sulfate, which is a copolymer of D-glucuronic acid or L-iduronic acid and N-acetyl-D-galactosamine; poly(ethylene oxide-co-propylene oxide); copolymers of PEG and hyaluronic acid; copolymers of PEG and heparin; copolymers of PEG and hirudin; graft copolymers of poly(L-lysine) and PEG; copolymers of PEG and a poly(hydroxyalkanoate) such as, for example, poly(ethylene glycol-co-hydroxybutyrate); and, any derivatives, analogs, congeners, salts, or combinations thereof. In some embodiments, the copolymer that may be used as a biobeneficial agent can be a copolymer of PEG and hyaluronic acid, a copolymer of PEG and hirudin, and any derivative, analog, congener, salt, copolymer or combination thereof. In other embodiments, the copolymer that may be used as a biobeneficial agent is a copolymer of PEG and a poly(hydroxyalkanoate) such as, for example, poly(hydroxybutyrate); and any derivative, analog, congener, salt, copolymer or combination thereof.

The bioactive agents can be any moiety capable of contributing to a therapeutic effect, a prophylactic effect, both a therapeutic and prophylactic effect, or other biologically active effect in a subject. A bioactive agent can also have diagnostic properties. The bioactive agents include, but are not limited to, small molecules, nucleotides, oligonucleotides, polynucleotides, amino acids, oligopeptides, polypeptides, and proteins. Bioactive agents include, but are not limited to, antiproliferatives, antineoplastics, antimitotics, anti-inflammatories, antiplatelets, anticoagulants, antifibrins, antithrombins, antibiotics, antiallergics, antioxidants, and any prodrugs, codrugs, metabolites, analogs, homologues, congeners, derivatives, salts and combinations thereof. It is to be appreciated that one skilled in the art should recognize that some of the groups, subgroups, and individual bioactive agents may not be used in some embodiments of the present invention.

Antiproliferatives include, for example, actinomycin D, actinomycin IV, actinomycin 11, actinomycin X1, actinomycin C1, and dactinomycin (Cosmegen®, Merck & Co., Inc.). Antineoplastics or antimitotics include, for example, paclitaxel (Taxol®, Bristol-Myers Squibb Co.), docetaxel (Taxotere®, Aventis S.A.), methotrexate, azathioprine, vincristine, vinblastine, fluorouracil, doxorubicin hydrochloride (Adriamycin®, Pfizer, Inc.) and mitomycin (Mutamycin®, Bristol-Myers Squibb Co.), and any prodrugs, codrugs, metabolites, analogs, homologues, congeners, derivatives, salts and combinations thereof. Antiplatelets, anticoagulants, antifibrin, and antithrombins include, for example, sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, and thrombin inhibitors (Angiomax®, Biogen, Inc.), and any prodrugs, codrugs, metabolites, analogs, homologues, congeners, derivatives, salts and combinations thereof. Cytostatic or antiproliferative agents include, for example, angiopeptin, angiotensin converting enzyme inhibitors such as captopril (Capoten® and Capozide®, Bristol-Myers Squibb Co.), cilazapril or lisinopril (Prinivil® and Prinzide®, Merck & Co., Inc.); calcium channel blockers such as nifedipine; colchicines; fibroblast growth factor (FGF) antagonists, fish oil (omega 3-fatty acid); histamine antagonists; lovastatin (Mevacor®, Merck & Co., Inc.); monoclonal antibodies including, but not limited to, antibodies specific for Platelet-Derived Growth Factor (PDGF) receptors; nitroprusside; phosphodiesterase inhibitors; prostaglandin inhibitors; suramin; serotonin blockers; steroids; thioprotease inhibitors; PDGF antagonists including, but not limited to, triazolopyrimidine; and nitric oxide, and any prodrugs, codrugs, metabolites, analogs, homologues, congeners, derivatives, salts and combinations thereof. Antiallergic agents include, but are not limited to, pemirolast potassium (Alamast®, Santen, Inc.), and any prodrugs, codrugs, metabolites, analogs, homologues, congeners, derivatives, salts and combinations thereof.

Examples of heparin derivatives include, but are not limited to, earth metal salts of heparin such as, for example, sodium heparin, potassium heparin, lithium heparin, calcium heparin, magnesium heparin, and low molecular weight heparin. Other examples of heparin derivatives include, but are not limited to, heparin sulfate, heparinoids, heparin-based compounds and heparin derivatized with hydrophobic materials.

Examples of hyaluronic acid derivates include, but are not limited to, sulfated hyaluronic acid such as, for example, O-sulphated or N-sulphated derivatives; esters of hyaluronic acid wherein the esters can be aliphatic, aromatic, arylaliphatic, cycloaliphatic, heterocyclic or a combination thereof; crosslinked esters of hyaluronic acid wherein the crosslinks can be formed with hydroxyl groups of a polysaccharide chain; crosslinked esters of hyaluronic acid wherein the crosslinks can be formed with polyalcohols that are aliphatic, aromatic, arylaliphatic, cycloaliphatic, heterocyclic, or a combination thereof; hemiesters of succinic acid or heavy metal salts thereof; quaternary ammonium salts of hyaluronic acid or derivatives such as, for example, the O-sulphated or N-sulphated derivatives.

Other bioactive agents useful in the present invention include, but are not limited to, free radical scavengers; nitric oxide donors; rapamycin; everolimus; tacrolimus; 40-O-(2-hydroxy)ethyl-rapamycin; 40-O-(3-hydroxy)propyl-rapamycin; 40-O-[2-(2-hydroxy)ethoxy]ethyl-rapamycin; tetrazole containing rapamycin analogs such as those described in U.S. Pat. No. 6,329,386; estradiol; clobetasol; idoxifen; tazarotene; alpha-interferon; host cells such as epithelial cells; genetically engineered epithelial cells; dexamethasone; cytokines; chemokines, chemokine mimetics, chemokine receptor ligands, and, any prodrugs, codrugs, metabolites, analogs, homologues, congeners, derivatives, salts and combinations thereof.

Free radical scavengers include, but are not limited to, 2,2',6,6'-tetramethyl-1-piperinyloxy, free radical (TEMPO); 4-amino-2,2',6,6'-tetramethyl-1-piperinyloxy, free radical (4-amino-TEMPO); 4-hydroxy-2,2',6,6'-tetramethyl-piperidene-1-oxy, free radical (TEMPOL), 2,2',3,4,5,5'-hexamethyl-3-imidazolinium-1-yloxy methyl sulfate, free radical; 16-doxyl-stearic acid, free radical; superoxide dismutase mimic (SODm) and any analogs, homologues, congeners, derivatives, salts and combinations thereof. Nitric oxide donors include, but are not limited to, S-nitrosothiols, nitrites, N-oxo-N-nitrosamines, substrates of nitric oxide synthase, diazenium diolates such as spermine diazenium diolate and any analogs, homologues, congeners, derivatives, salts and combinations thereof. Chemokines include, but are not limited to, IL-8, IP-10, MIP-1α, RANTES, 1-309, MCP-1, CCL28, and SDF-1. Chemokines and chemokine mimetics include, but are not limited to, those taught in U.S. Patent Application Publication Nos. 2002/0156034, 2002/0165123, and 2003/0148940; and U.S. Patent Application No. 10/243,795; each of which is incorporated by reference herein in its entirety. Chemokine receptor ligands include, but are not limited to, those taught in U.S. Patent Nos. 6,515,001 and 6,693,134; and U.S. Patent Application Publication Nos. 2003/0004136, 2003/0045550, 2003/0092674, and 2003/0125380; each of which is incorporated by reference herein in its entirety.

Diagnostic agents include, but are not limited to, materials that are radiopaque, radioactive, paramagnetic, fluorescent, lumiscent, and detectable by ultrasound. In some embodiments, the radiopaque agents are materials comprising iodine or iodine-derivatives such as, for example, iohexal and iopamidol. In other embodiments, the radioactive materials are radioisotopes, which can be detected by tracing radioactive emissions. Examples of radioactive materials include, but are not limited to, ¹⁴C, ¹²³I, ¹²⁴I, ¹²⁵I, ¹³¹I, ^{99m}Tc, ³⁵S or ³H. In other embodiments, the paramagnetic agents include, but are not limited to, gadolinium chelated compounds. Examples of fluorescent agents include, but are not limited to, indocyanine green, umbelliferone, fluorescein, fluorescein isothiocyanate, rhodamine, dichlorotriazinylamine fluorescein, dansyl chloride or phycoerythrin. Examples of agents detectable by ultrasound include, but are not limited to, perflexane, Albunex® and Optison®. Examples of agents used in PET include, but are not limited to, fluorodeoxyglucose, sodium fluoride, methionine, choline, deoxyglucose, butanol, raclopride, spiperone, bromospiperone, carfentanil, and flumazenil. Other examples of detectable substances include, but are not limited to, various enzymes and prosthetic groups. Examples of suitable enzymes include, but are not limited to, horseradish peroxidase, alkaline phosphatase, β-galactosidase, or acetylcholinesterase. Examples of suitable prosthetic group complexes include, but are not limited to, streptavidin/biotin and avidin/biotin.

Labeled IL-8 mimetics can be used to assess *in vivo* pharmacokinetics, as well as detect the progression of a disease or the propensity of a subject to develop a disease. For example, chemokine receptors for tissue distribution can be detected using a labeled IL-8 mimetic either *in vivo* or in an *in vitro* sample derived from a subject. In some embodiments, an IL-8 mimetic may be radioactively labeled with ¹⁴C, either by incorporation of ¹⁴C into the modifying group or one or more amino acid structures in the IL-8 mimetic.

A modifying group can be chosen to provide a chelation site for a diagnostic label. In one embodiment, the modifying group can be the Aic derivative of cholic acid, which provides a free amino group. In another example, a tyrosine residue within an IL-8 sequence may be substituted with radioactive iodotyrosyl. In other embodiments, an IL-8 mimetic may be labeled with radioactive technetium or iodine. In fact, any isotope of radioactive iodine may be incorporated to create a diagnostic agent. In some embodiments, ¹²³I has a half-life of 13.2 hours and can be used for whole body scintigraphy; ¹²⁴I has a half life of 4 days and can be used for PET; ¹²⁵I has a half life of 60 days and can be used for metabolic turnover studies; and, ¹³¹I has a half life of 8 days and can be used for whole body counting and delayed low resolution imaging studies.

Aminopeptidases and carboxypeptidases have been found to have important functions in biological activities such as, for example, diabetes, memory and learning, antigen formation, and angiogenesis. The term "aminopeptidase" refers to a multifunctional enzyme that cleaves proteins from the N-terminus. Aminopeptidases can be classified into a number families such as, for example, the zinc-containing (M1) aminopeptidase family which consists of nine aminopeptidases that include, but are not limited to, placental leucine aminopeptidase (P-LAP), adipocyte-derived leucine aminopeptidase (A-LAP) and leukocyte-derived arginine aminopeptidase (L-RAP). Modulation of aminopeptidase activity can have many therapeutic and prophylactic applications. In one example, control of the activity of P-LAP can control the inducement of uterine contractions and treat or prevent disorders such as premature delivery and spontaneous abortion, as well as other disorders associated with water resorption, memory and learning and glucose metabolism. In another example, control of the activity of A-LAP can treat disorders associated with antigen production, blood pressure and inflammation. In another example, control of the activity of L-RAP can treat disorders association with antigen formation.

Although both aminopeptidases and carboxypeptidases can terminate biological activity, the carboxypeptidases clearly predominate in such terminations. The term "carboxypeptidase" refers to a multifunctional enzyme that cleaves proteins from the C-terminus. Carboxypeptidases are derived from the zymogens, procarboxypeptidase A and B. Modulation of carboxypeptidase activity can have many therapeutic and prophylactic applications. In one example, control of the activity of the carboxypeptidases such as kininase II (angiotensin-converting enzyme), carboxypeptidase M, and carboxypeptidase N, can potentially control hypertensive disorders relating to cardiovascular and kidney disorders. These carboxypeptidases are efficient at cleaving the C-terminal arginine of kinins, which appear to be important regulators of cardiovascular function; and are likely participants in the actions of drugs that affect the heart, kidney, and circulation. The kinins also have some role in the regulation of local and systemic hemodynamics; vascular permeability; inflammatory response; activation of neuronal pathways; and movement of electrolytes, water, and metabolic substrates across epithelia and into other tissues. Accordingly, control of carboxypeptidase activity can control the activity of other chemicals such as, for example, kinins, and thus can have many therapeutic applications in the diagnosis and treatment of disease.

In some embodiments, a modification may be introduced at the C-terminus of a peptide, the N-terminus of a peptide, in the region between the C-terminus and N-terminus, or a combination thereof. In some embodiments, a modification to the C-terminus may reduce the ability of an IL-8 mimetic to act as a substrate for carboxypeptidases. Examples of such C-terminal modifiers include, but are not limited to, an amide group, an ethylamide group and various non-natural amino acids such as, for example, D-amino acids and β-alanine. In another embodiment, a modification of a C-terminus may be accompanied by a modification to the N-terminus to reduce the ability of an IL-8 mimetic to act as a substrate for aminopeptidases. Examples of such N-terminus modifiers include, but are not limited to acyl, alkyl, aryl, arylalkyl, hydroxyalkyl, alkanoyl groups, alkanoics, diacids, and other modifiers having a carboxyl functional group. In another embodiment, the modification to an N-terminus can be deamidation.

In another embodiment, an IL-8 mimetic may be prepared in a "prodrug" form, wherein the mimetic begins acting upon its metabolism *in vivo,* in which the mimetic can become, for example, an agonist or an antagonist. Accordingly, such a prodrug form of an IL-8 mimetic can also deliver another agent upon its metabolism *in vivo*, and is known as a "codrug" form of the IL-8 mimetic. Examples of such agents include the bioactive agents, biobeneficial agents, diagnostic agents, and additional IL-8 mimetics. In some embodiments, the agent comprises a glycosaminoglycan such as for example, heparin, hirudin, hyaluronic acid, and any prodrugs, codrugs, metabolites, analogs, homologues, congeners, derivatives, salts and combinations thereof. In other embodiments, the agent comprises a phospholipid such as, for example, phosphatidylcholine (lecithin). In some embodiments, the phospholipids can be conjugated to any functional group on an IL-8 mimetic, wherein the phospholipid and/or the IL-8 mimetic can be modified as necessary. In these embodiments, the phospholipids can be connected to an amino functional group, such as for example the N-terminus of an IL-8 mimetic. It is to be appreciated that one skilled in the art should recognize that some of the groups, subgroups, and individual biobeneficial agents described herein may not be used in some embodiments of the present invention.

Phosphatidylcholine is a phospholipid that is a major constituent of cell membranes. Phosphatidylcholine may have hepatoprotective activity, is important for normal cellular membrane composition and repair, and is the major delivery form of the essential nutrient choline, which is a precursor in the synthesis of the neurotransmitter acetylcholine. Phosphatidylcholine's role in the maintenance of cell-membrane integrity is vital to all of the basic biological processes such as, for example, information flow that occurs within cells in the transcription of DNA to RNA; the translation of RNA to proteins; the formation of cellular energy, and intracellular communication or signal transduction. Phosphatidylcholine has a fluidizing effect on cellular membranes, which is important in that a decrease in cell-membrane fluidization, a breakdown of cell-membrane integrity, and an impairment of cell-membrane repair mechanisms are associated with a number of disorders, including, but not limited to liver disease, neurological diseases, various cancers, cell death.

The IL-8 mimetics can be administered as a codrug with phosphatidylcholine in an effective amount to diagnose, prevent, treat, or ameliorate a symptom of a disease. In some embodiments, the disease can be liver disease. The liver diseases may include, but are not limited to, alcoholic and non-alcoholic liver disorders such as, for example, fibrosis; cirrhosis; and hepatitis A, B, C and E. In other embodiments, the disease can be neurological disease. The neurological diseases include, but are not limited to, manic conditions; cognitive disorders such as old-age memory loss, short-term memory loss, and Alzheimer's Disease; and tardive dyskinesia. In other embodiments, the disease can be any cancer that is associated with a deficiency in choline and phosphatidylcholine such as, for example, liver cancer. In other embodiments, the disease can be a choline deficiency that results in apoptosis, atherosclerosis or a loss of memory. In some embodiments, an effective amount of phosphatidylcholine is a daily administration that ranges from about 10 mg/kg to about 1000 mg/kg, from about 20 mg/kg to about 800 mg/kg, from about 30 mg/kg to about 600 mg/kg, from about 40 mg/kg to about 400 mg/kg, from about 40 mg/kg to about 200 mg/kg, from about 50 mg/kg to about 100 mg/kg, or any range therein.

The IL-8 mimetics may be prepared using techniques known in the art. A peptide or polypeptide component of an IL-8 mimetic may be composed, at least in part, of a peptide that has been synthesized, purified, and verified. Receptor agonist activity of the native IL-8 and its mimetics may also be assayed and compared, for example, using standard assay methods. In another example, the peptides and polypeptides may be dimerized through a disulfide bridge formed by gentle oxidation of the cysteines using 10% DMSO in water, purified by HPLC, and verified by mass spectrometry. In another example, one or more modifying groups may be attached to a peptide derived from a native IL-8 by standard methods such as, for example, connecting reactive groups on the amino acid to reactive groups on the modifying groups. Examples of such reactive groups include, but are not limited to, an amino group such as the alpha-amino group at the amino-terminus of a peptide; a carboxyl group at the carboxy terminus of a peptide; a hydroxyl group such as those present on a tyrosine, serine or threonine residue; or, other suitable reactive group on an amino acid side chain.

The amino acid sequence of the human CXC chemokine IL-8 is as follows:

Human CXC Chemokine IL-8

Ala-Val-Ile-Pro-Arg-Ser1-Ala-Lys-Glu-Leu-Arg-Cys-Gln-Cys-Ile 10

Lys-Thr-Tyr-Ser-Lys-Pro-Phe-His-Pro-Lys-Phe-Ile-Lys-Glu-Leu 25

Arg-Val-Ile-Glu-Ser-Gly-Pro-His-Cys-Ala-Asn-Thr-Glu-Ile-Ile 40

Val-Lys-Leu-Ser-Asp-Gly-Arg-Glu-Leu-Cys-Leu-Asp-Pro-Lys-Glu 55

Asn-Trp-Val-Gln-Arg-Val-Val-Glu-Lys-Phe-Leu-Lys-Arg-Ala-Glu 70

Asn-Ser72 72

(SEQ ID NO:1).

The human CXC chemokine IL-8 has a total of 77 amino acid residues. The portions of the human CXC chemokine IL-8 that are used to construct each group of IL-8 mimetics are provided in a header preceding each list of analogs. In the embodiments discussed below, the system of numbering the residues is based on a hypothetical "IL-8-1" having 72 residues, in which the first 5 residues of the human CXC chemokine IL-8 are disregarded. Accordingly, the numbering of residues begins at Ser residue 6 of the human CXC chemokine IL-8. For example, the first header, "1L-8-1(1-15)," refers to residues 1 through 15 of the IL-8-1 system of numbering, which corresponds to residues 6 through 20 of the human CXC chemokine IL-8. The human CXC chemokine IL-8 mimetics of the present invention are described herein by their amino acid sequences that are identified by sequence identification numbers SEQ ID NO:9 to SEQ ID NO:162, SEQ ID NO:1641 to SEQ ID NO:1675, variants a155-a342, and conservatively modified variants thereof.

In some embodiments, the mimetics of human CXC chemokine IL-8 ("the IL-8 mimetics") correspond to a portion of the N-terminal region of the human CXC chemokine IL-8 and have the following sequences of amino acids:

IL-8-1(1-15)

a155) R_{N}-Ser-Ala-Lys-Glu-Leu-Arg-Cys-Gln-Cys-Ile-Lys-Thr-Tyr-Ser-Lys-
R_{C} (SEQ ID NO:9);

a156) R_{N}-Ser-Ala-Lys-Glu-Leu-Arg-Xaa₁-Gln-Xaa₂-Ile-Lys-Thr-Tyr-Ser-
Lys-R_{C}
(SEQ ID NO:10);

a157) R_{N}-Xaa₃-Ala-Lys-Glu-Leu-Arg-Xaa₁-Gln-Xaa₂-Ile-Lys-Thr-Tyr-Ser-
Lys-R_{C}
(SEQ ID NO:11);

a158) R_{N}-Ser-Xaa₃-Lys-Glu-Leu-Arg-Xaa₁-Gln-Xaa₂-Ile-Lys-Thr-Tyr-Ser-
Lys-R_{C}
(SEQ ID NO:12);

a159) R_{N}-Ser-Ala-Xaa₃-Glu-Leu-Arg-Xaa₁-Gln-Xaa₂-Ile-Lys-Thr-Tyr-Ser-
Lys-R_{C}
(SEQ ID NO:13);

IL-8-1(1-13)

a160) R_{N}-Ser-Ala-Lys-Glu-Leu-Arg-Cys-Gln-Cys-Ile-Lys-Thr-Tyr-R_{C}
(SEQ ID NO:14);

a161) R_{N}-Ser-Ala-Lys-Glu-Leu-Arg-Xaa₁-Gln-Xaa₂-Ile-Lys-Thr-Tyr-R_{C}
(SEQ ID NO:15);
a162) R_{N}-Xaa₃-Ala-Lys-Glu-Leu-Arg-Xaa₁-Gln-Xaa₂-Ile-Lys-Thr-Tyr-R_{C}
(SEQ ID NO:16);
a163) R_{N}-Ser-Xaa₃-Lys-Glu-Leu-Arg-Xaa₁-Gln-Xaa₂-Ile-Lys-Thr-Tyr-R_{C}
(SEQ ID NO:17);
a164) R_{N}-Ser-Ala-Xaa₃-Glu-Leu-Arg-Xaa₁-Gln-Xaa₂-Ile-Lys-Thr-Tyr-R_{C}
(SEQ ID NO:18);

IL-8-1(1-11)

a165) RN-Ser-Ala-Lys-Glu-Leu-Arg-Cys-Gln-Cys-Ile-Lys-RC
(SEQ ID NO:19);

a166) RN-Ser-Ala-Lys-Glu-Leu-Arg-Xaa1-Gln-Xaa2-Ile-Lys-RC
(SEQ ID NO:20);

a167) RN-Xaa3-Ala-Lys-Glu-Leu-Arg-Xaa1-Gln-Xaa2-Ile-Lys-RC
(SEQ ID NO:21);

a168) RN-Ser-Xaa3-Lys-Glu-Leu-Arg-Xaa1-Gln-Xaa2-Ile-Lys-RC
(SEQ ID NO:22); and

a169) RN-Ser-Ala-Xaa3-Glu-Leu-Arg-Xaa1-Gln-Xaa2-Ile-Lys-RC
(SEQ ID NO:23).

In some embodiments, the IL-8 mimetics correspond to a portion of the internal-region of IL-8 and have the following sequences of amino acids:

[A9]-IL-8-1(9-33)

a170) RN-Ala-Ile-Lys-Thr-Tyr-Ser-Lys-Pro-Phe-His-Pro-Lys-Phe-Ile-Lys-
Glu-Leu-Arg-Val-Ile-Glu-Ser-Gly-Pro-His-RC
(SEQ ID NO:24); and

[A³⁴]-IL-8-1(34-49)

a171) R_N-Ala-Ala-Asn-Thr-Glu-Ile-Ile-Val-Lys-Leu-Ser-Asp-Gly-Arg-Glu-
Leu-R_C
(SEQ ID NO:25).

In some embodiments, the IL-8 mimetics correspond to a portion of the C-terminal of IL-8 and have the following sequence of amino acids:

IL-8-1(56-71)

a172) R_{N}-Asn-Trp-Val-Gln-Arg-Val-Val-Glu-Lys-Phe-Leu-Lys-Arg-Ala-
Glu-Asn-R_{C}
(residues 6-21 of SEQ ID NO:26).

In some embodiments, the IL-8 mimetics correspond to a portion of the N-terminal joined with a linker to the C-terminal region and have the following sequence of amino acids:

IL-8-1(1-15)-[linker]-IL-8-1(56-71)

a173) R_{N}-Ser-Ala-Lys-Glu-Leu-Arg-Cys-Gln-Cys-Ile-Lys-Thr-Tyr-Ser-Lys-
[linker]-Asn-Trp-Val-Gln-Arg-Val-Val-Glu-Lys-Phe-Leu-Lys-Arg-
Ala-Glu-Asn-R_{C}
(SEQ ID NO:27);

a174) R_{N}-Ser-Ala-Lys-Glu-Leu-Arg-Xaa₁-Gln-Xaa₂-Ile-Lys-Thr-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:28);

a175) R_{N}-Xaa₃-Ala-Lys-Glu-Leu-Arg-Xaa₁-Gln-Xaa₂-Ile-Lys-Thr-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:29);

a176) RN-Ser-Xaa3-Lys-Glu-Leu-Arg-Xaa1-Gln-Xaa2-Ile-Lys-Thr-Tyr-Ser-
Lys-{linker]-Asn-Trp-Val-Gln-Arg-Val-Val-Glu-Lys-Phe-Leu-Lys-
Arg-Ala-Glu-Asn-RC
(SEQ ID NO:30);

a177) RN-Ser-Ala-Xaa3-Glu-Leu-Arg-Xaa1-Gln-Xaa2-Ile-Lys-Thr-Tyr-Ser-
Lys-{linker]-Asn-Trp-Val-Gln-Arg-Val-Val-Glu-Lys-Phe-Leu-Lys-
Arg-Ala-Glu-Asn-RC
(SEQ ID NO:31);

a178) RN-Ser-Ala-Lys-Glu-Leu-Arg-Xaa1-Gln-Xaa2-Xaa2-Lys-Thr-Tyr-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:32);

a179) RN-Xaa3-Ala-Lys-Glu-Leu-Arg-Xaa1-Gln-Xaa2-Xaa4-Lys-Thr-Tyr-
Ser-Lys-[linker]-Asn-Trp-Val-Gln-Arg-Val-Val-Glu-Lys-Phe-Leu-
Lys-Arg-Ala-Glu-Asn-RC
(SEQ ID NO:33);

a180) RN-Ser-Xaa3-Lys-Glu-Leu-Arg-Xaa1-Gln-Xaa2-Xaa4-Lys-Thr-Tyr-
Ser-Lys-{linker]-Asn-Trp-Val-Gln-Arg-Val-Val-Glu-Lys-Phe-Leu-
Lys-Arg-Ala-Glu-Asn-RC
(SEQ ID NO:34);

a181) RN-Ser-Ala-Xaa3-Glu-Leu-Arg-Xaa1-Gln-Xaa2-Xaa4-Lys-Thr-Tyr-Ser-
Lys-{linker]-Asn-Trp-Val-Gln-Arg-Val-Val-Glu-Lys-Phe-Leu-Lys-
Arg-Ala-Glu-Asn-RC
(SEQ ID NO:35);

a182) RN-Ser-Ala-Lys-Glu-Leu-Arg-Xaa1-Gln-Xaa2-Ile-Xaa4-Thr-Tyr-Ser-
Lys-{linker]-Asn-Trp-Val-Gln-Arg-Val-Val-Glu-Lys-Phe-Leu-Lys-
Arg-Ala-Glu-Asn-RC
(SEQ ID NO:36);

a183) RN-Xaa3-Ala-Lys-Glu-Leu-Arg-Xaa1-Gln-Xaa2-Ile-Xaa4-Thr-Tyr-Ser-
Lys-{linker]-Asn-Trp-Val-Gln-Arg-Val-Val-Glu-Lys-Phe-Leu-Lys-

Arg-Ala-Glu-Asn-R_{C}
(SEQ ID NO:37);
a184) R_{N}-Ser-Xaa₃-Lys-Glu-Leu-Arg-Xaa₁-Gln-Xaa₂-Ile-Xaa₄-Thr-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:38);
a185) R_{N}-Ser-Ala-Xaa₃-Glu-Leu-Arg-Xaa₁-Gln-Xaa₂-Ile-Xaa₄-Thr-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:39);
a186) R_{N}-Ser-Ala-Lys-Glu-Leu-Arg-Xaa₁-Gln-Xaa₂-Ile-Lys-Xaa₄-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:40);
a187) R_{N}-Xaa₃-Ala-Lys-Glu-Leu-Arg-Xaa₁-Gln-Xaa₂-Ile-Lys-Xaa₄-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:41);
a188) R_{N}-Ser-Xaa₃-Lys-Glu-Leu-Arg-Xaa₁-Gln-Xaa₂-Ile-Lys-Xaa₄-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:42);
a189) R_{N}-Ser-Ala-Xaa₃-Glu-Leu-Arg-Xaa₁-Gln-Xaa₂-Ile-Lys-Xaa₄-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:43);
a190) R_{N}-Ser-Ala-Lys-Glu-Leu-Arg-Xaa₁-Gln-Xaa₂-Ile-Lys-Thr-Xaa₄-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:44);

a191) R_{N}-Xaa₃-Ala-Lys-Glu-Leu-Arg-Xaa₁-Gln-Xaa₂-Ile-Lys-Thr-Xaa₄-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:45);

a192) R_{N}-Ser-Xaa₃-Lys-Glu-Leu-Arg-Xaa₁-Gln-Xaa₂-Ile-Lys-Thr-Xaa₄-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:46);

a193) R_{N}-Ser-Ala-Xaa₃-Glu-Leu-Arg-Xaa₁-Gln-Xaa₂-Ile-Lys-Thr-Xaa₄-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:47);

a194) R_{N}-Ser-Ala-Lys-Glu-Leu-Arg-Xaa₁-Gln-Xaa₂-Ile-Lys-Thr-Tyr-Xaa₄-
Lys-[linker]-Asn-Trp-Val-Gln-Arg-Val-Val-Glu-Lys-Phe-Leu-Lys-
Arg-Ala-Glu-Asn-R_{C}
(SEQ ID NO:48);

a195) R_{N}-Xaa₃-Ala-Lys-Glu-Leu-Arg-Xaa₁-Gln-Xaa₂-Ile-Lys-Thr-Tyr-Xaa₄-
Lys-[linker]-Asn-Trp-Val-Gln-Arg-Val-Val-Glu-Lys-Phe-Leu-Lys-
Arg-Ala-Glu-Asn-R_{C}
(SEQ ID NO:49);

a196) R_{N}-Ser-Xaa₃-Lys-Glu-Leu-Arg-Xaa₁-Gln-Xaa₂-Ile-Lys-Thr-Tyr-Xaa₄-
Lys-[linker]-Asn-Trp-Val-Gln-Arg-Val-Val-Glu-Lys-Phe-Leu-Lys-
Arg-Ala-Glu-Asn-R_{C}
(SEQ ID NO:50);

a197) R_{N}-Ser-Ala-Xaa₃-Glu-Leu-Arg-Xaa₁-Gln-Xaa₂-Ile-Lys-Thr-Tyr-Xaa₄-
Lys-[linker]-Asn-Trp-Val-Gln-Arg-Val-Val-Glu-Lys-Phe-Leu-Lys-
Arg-Ala-Glu-Asn-R_{C}
(SEQ ID NO:51);

a198) R_{N}-Ser-Ala-Lys-Glu-Leu-Arg-Xaa₁-Gln-Xaa₂-Ile-Lys-Thr-Tyr-Ser-
Xaa₄-[linker]-Asn-Trp-Val-Gln-Arg-Val-Val-Glu-Lys-Phe-Leu-Lys-

Arg-Ala-Glu-Asn-R_{C}
(SEQ ID NO:52);
a199) R_{N}-Xaa₃-Ala-Lys-Glu-Leu-Arg-Xaa₁-Gln-Xaa₂-Ile-Lys-Thr-Tyr-Ser-
Xaa₄-[linker]-Asn-Trp-Val-Gln-Arg-Val-Val-Glu-Lys-Phe-Leu-Lys-
Arg-Ala-Glu-Asn-R_{C}
(SEQ ID NO:53);
a200) R_{N}-Ser-Xaa₃-Lys-Glu-Leu-Arg-Xaa₁-Gln-Xaa₂-Ile-Lys-Thr-Tyr-Ser-
Xaa₄-[linker]-Asn-Trp-Val-Gln-Arg-Val-Val-Glu-Lys-Phe-Leu-Lys-
Arg-Ala-Glu-Asn-R_{C}
(SEQ ID NO:54);
a201) R_{N}-Ser-Ala-Xaa₃-Glu-Leu-Arg-Xaa₁-Gln-Xaa₂-Ile-Lys-Thr-Tyr-Ser-
Xaa₄-[linker]-Asn-Trp-Val-Gln-Arg-Val-Val-Glu-Lys-Phe-Leu-Lys-
Arg-Ala-Glu-Asn-R_{C}
(SEQ ID NO:55);

IL-8-1(1-13)-[linker]-IL-8-1(56-71)

a202) RN-Ser-Ala-Lys-Glu-Leu-Arg-Cys-Gln-Cys-Ile-Lys-Thr-Tyr-[linker]-
Asn-Trp-Val-Gln-Arg-Val-Val-Glu-Lys-Phe-Leu-Lys-Arg-Ala-Glu-
Asn-RC
(SEQ ID NO:56);

a203) RN-Ser-Ala-Lys-Glu-Leu-Arg-Xaa1-Gln-Xaa2-Ile-Lys-Thr-Tyr-
[linker]-Asn-Trp-Val-Gln-Arg-Val-Val-Glu-Lys-Phe-Leu-Lys-Arg-
Ala-Glu-Asn-RC
(SEQ ID NO:57);

a204) RN-Xaa3-Ala-Lys-Glu-Leu-Arg-Xaa1-Gln-Xaa2-Ile-Lys-Thr-Tyr-
[linker]-Asn-Trp-Val-Gln-Arg-Val-Val-Glu-Lys-Phe-Leu-Lys-Arg-
Ala-Glu-Asn-RC
(SEQ ID NO:58);

a205) RN-Ser-Xaa3-Lys-Glu-Leu-Arg-Xaa1-Gln-Xaa2-Ile-Lys-Thr-Tyr-
[linker]-Asn-Trp-Val-Gln-Arg-Val-Val-Glu-Lys-Phe-Leu-Lys-Arg-

Ala-Glu-Asn-R_{C}
(SEQ ID NO:59);
a206) R_{N}-Ser-Ala-Xaa₃-Glu-Leu-Arg-Xaa₁-Gln-Xaa₂-Ile-Lys-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:60);
a207) R_{N}-Ser-Ala-Lys-Glu-Leu-Arg-Xaa₁-Gln-Xaa₂-Xaa₄-Lys-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:61);
a208) R_{N}-Xaa₃-Ala-Lys-Glu-Leu-Arg-Xaa₁-Gln-Xaa₂-Xaa₄-Lys-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:62);
a209) R_{N}-Ser-Xaa₃-Lys-Glu-Leu-Arg-Xaa₁-Gln-Xaa₂-Xaa₄-Lys-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:63);
a210) R_{N}-Ser-Ala-Xaa₃-Glu-Leu-Arg-Xaa₁-Gln-Xaa₂-Xaa₄-Lys-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:64);
a211) R_{N}-Ser-Ala-Lys-Glu-Leu-Arg-Xaa₁-Gln-Xaa₂-Ile-Xaa₄-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:65);
a212) R_{N}-Xaa₃-Ala-Lys-Glu-Leu-Arg-Xaa₁-Gln-Xaa₂-Ile-Xaa₄-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:66);

a213) RN-Ser-Xaa3-Lys-Glu-Leu-Arg-Xaa1-Gln-Xaa2-Ile-Xaa4-Thr-Tyr-
[linker]-Asn-Trp-Val-Gln-Arg-Val-Val-Glu-Lys-Phe-Leu-Lys-Arg-
Ala-Glu-Asn-RC
(SEQ ID NO:67);

a214) RN-Ser-Ala-Xaa3-Glu-Leu-Arg-Xaa1-Gln-Xaa2-Ile-Xaa4-Thr-Tyr-
[linker]-Asn-Trp-Val-Gln-Arg-Val-Val-Glu-Lys-Phe-Leu-Lys-Arg-
Ala-Glu-Asn-RC
(SEQ ID NO:68);

a215) RN-Ser-Ala-Lys-Glu-Leu-Arg-Xaa1-Gln-Xaa2-Ile-Lys-Xaa4-Tyr-
[linker]-Asn-Trp-Val-Gln-Arg-Val-Val-Glu-Lys-Phe-Leu-Lys-Arg-
Ala-Glu-Asn-RC
(SEQ ID NO:69);

a216) RN-Xaa3-Ala-Lys-Glu-Leu-Arg-Xaa1-Gln-Xaa2-Ile-Lys-Xaa4-Tyr-
[linker]-Asn-Trp-Val-Gln-Arg-Val-Val-Glu-Lys-Phe-Leu-Lys-Arg-
Ala-Glu-Asn-RC
(SEQ ID NO:70);

a217) RN-Ser-Xaa3-Lys-Glu-Leu-Arg-Xaa1-Gln-Xaa2-Ile-Lys-Xaa4-Tyr-
[linker]-Asn-Trp-Val-Gln-Arg-Val-Val-Glu-Lys-Phe-Leu-Lys-Arg-
Ala-Glu-Asn-RC
(SEQ ID NO:71);

a218) RN-Ser-Ala-Xaa3-Glu-Leu-Arg-Xaa1-Gln-Xaa2-Ile-Lys-Xaa4-Tyr-
[linker]-Asn-Trp-Val-Gln-Arg-Val-Val-Glu-Lys-Phe-Leu-Lys-Arg-
Ala-Glu-Asn-RC
(SEQ ID NO:72);

a219) RN-Ser-Ala-Lys-Glu-Leu-Arg-Xaa1-Gln-Xaa2--Ile-Lys-Thr-Xaa4-
[linker]-Asn-Trp-Val-Gln-Arg-Val-Val-Glu-Lys-Phe-Leu-Lys-Arg-
Ala-Glu-Asn-RC
(SEQ ID NO:73);

a220) RN-Xaa3-Ala-Lys-Glu-Leu-Arg-Xaa1-Gln-Xaa2-Ile-Lys-Thr-Xaa4-
[linker]-Asn-Trp-Val-Gln-Arg-Val-Val-Glu-Lys-Phe-Leu-Lys-Arg-

Ala-Glu-Asn-R_{C}
(SEQ ID NO:74);
a221) R_{N}-Ser-Xaa₃-Lys-Glu-Leu-Arg-Xaa₁-Gln-Xaa₂-Ile-Lys-Thr-Xaa₄-
[linker]-Asn-Trp-Val-Gln-Arg-Val-Val-Glu-Lys-Phe-Leu-Lys-Arg-
Ala-Glu-Asn-R_{C}
(SEQ ID NO:75);
a222) R_{N}-Ser-Ala-Xaa₃-Glu-Leu-Arg-Xaa₁-Gln-Xaa₂-Ile-Lys-Thr-Xaa₄-
[linker]-Asn-Trp-Val-Gln-Arg-Val-Val-Glu-Lys-Phe-Leu-Lys-Arg-
Ala-Glu-Asn-R_{C}
(SEQ ID NO:76);

IL-8-1(1-11)-[linker]-IL-8-1(56-71)

a223) R_{N}-Ser-Ala-Lys-Glu-Leu-Arg-Cys-Gln-Cys-Ile-Lys-[linker]-Asn-Trp-
Val-Gln-Arg-Val-Val-Glu-Lys-Phe-Leu-Lys-Arg-Ala-Glu-Asn-R_{C}
(SEQ ID NO:77);

a224) R_{N}-Ser-Ala-Lys-Glu-Leu-Arg-Xaa₁-Gln-Xaa₂-Ile-Lys-[linker]-Asn-
Trp-Val-Gln-Arg-Val-Val-Glu-Lys-Phe-Leu-Lys-Arg-Ala-Glu-Asn-
R_{C}
(SEQ ID NO:78);

a225) R_{N}-Xaa₃-Ala-Lys-Glu-Leu-Arg-Xaa₁-Gln-Xaa₂-Ile-Lys-[linker]-Asn-
Trp-Val-Gln-Arg-Val-Val-Glu-Lys-Phe-Leu-Lys-Arg-Ala-Glu-Asn-
R_{C}
(SEQ ID NO:79);

a226) R_{N}-Ser-Xaa₃-Lys-Glu-Leu-Arg-Xaa₁-Gln-Xaa₂-Ile-Lys-[linker]-Asn-
Trp-Val-Gln-Arg-Val-Val-Glu-Lys-Phe-Leu-Lys-Arg-Ala-Glu-Asn-
R_{C}
(SEQ ID NO:80);

a227) R_{N}-Ser-Ala-Xaa₃-Glu-Leu-Arg-Xaa₁-Gln-Xaa₂-Ile-Lys-[linker]-Asn-
Trp-Val-Gln-Arg-Val-Val-Glu-Lys-Phe-Leu-Lys-Arg-Ala-Glu-Asn-
R_{C}
(SEQ ID NO:81);

a228) RN-Ser-Ala-Lys-Glu-Leu-Arg-Xaa1-Gln-Xaa2-Xaa4-Lys-[linker]-Asn-
Trp-Val-Gln-Arg-Val-Val-Glu-Lys-Phe-Leu-Lys-Arg-Ala-Glu-Asn-
RC
(SEQ ID NO:82);

a229) RN-Xaa3-Ala-Lys-Glu-Leu-Arg-Xaa1-Gln-Xaa2-Xaa4-Lys-[linker]-
Asn-Trp-Val-Gln-Arg-Val-Val-Glu-Lys-Phe-Leu-Lys-Arg-Ala-Glu-
Asn-RC
(SEQ ID NO:83);

a230) RN-Ser-Xaa3-Lys-Glu-Leu-Arg-Xaa1-Gln-Xaa2-Xaa4-Lys-[linker]-
Asn-Trp-Val-Gln-Arg-Val-Val-Glu-Lys-Phe-Leu-Lys-Arg-Ala-Glu-
Asn-RC
(SEQ ID NO:84);

a231) RN-Ser-Ala-Xaa3-Glu-Leu-Arg-Xaa1-Gln-Xaa2-Xaa4-Lys-[linker]-
Asn-Trp-Val-Gln-Arg-Val-Val-Glu-Lys-Phe-Leu-Lys-Arg-Ala-Glu-
Asn-RC
(SEQ ID NO:85);

a232) RN-Ser-Ala-Lys-Glu-Leu-Arg-Xaa1-Gln-Xaa2-Ile-Xaa4-[linker]-Asn-
Trp-Val-Gln-Arg-Val-Val-Glu-Lys-Phe-Leu-Lys-Arg-Ala-Glu-Asn-
RC
(SEQ ID NO:86);

a233) RN-Xaa3-Ala-Lys-Glu-Leu-Arg-Xaa1-Gln-Xaa2-Ile-Xaa4-[linker]-Asn-
Trp-Val-Gln-Arg-Val-Val-Glu-Lys-Phe-Leu-Lys-Arg-Ala-Glu-Asn-
RC
(SEQ ID NO:87);

a234) RN-Ser-Xaa3-Lys-Glu-Leu-Arg-Xaa1-Gln-Xaa2-Ile-Xaa4-[linker]-Asn-
Trp-Val-Gln-Arg-Val-Val-Glu-Lys-Phe-Leu-Lys-Arg-Ala-Glu-Asn-
RC
(SEQ ID NO:88); and

a235) RN-Ser-Ala-Xaa3-Glu-Leu-Arg-Xaa1-Gln-Xaa2-Ile-Xaa4-[linker]-Asn-
Trp-Val-Gln-Arg-Val-Val-Glu-Lys-Phe-Leu-Lys-Arg-Ala-Glu-Asn-

R_C
(SEQ ID NO:89).

In some embodiments, the IL-8 mimetics correspond to a cyclic portion of the N-terminal-region of IL-8 and have the following sequences of amino acids:

IL-8-1(1-13)-cyclo-(Xaa5-Xaa6)

a236) RN-Xaa5-Ala-Lys-Glu-Leu-Arg-Xaa1-Gln-Xaa2-Ile-Lys-Thr-Xaa6-RC
(SEQ ID NO:90);

a237) RN-Xaa5-Xaa3-Lys-Glu-Leu-Arg-Xaa1-Gln-Xaa2-Ile-Lys-Thr-Xaa6-RC
(SEQ ID NO:91); and

a238) RN-Xaa5-Ala-Xaa3-Glu-Leu-Arg-Xaa1-Gln-Xaa2-Ile-Lys-Thr-Xaa6-RC
(SEQ ID NO:92).

In some embodiments, the IL-8 mimetics correspond to a cyclic portion of the internal-region of IL-8 and have the following sequences of amino acids (the underlined residues below are cyclized):

IL-8-1(9-34)-cyclo-(Xaa5-Xaa6)

a239) RN-Xaa5-Ile-Lys-Thr-Tyr-Ser-Lys-Pro-Phe-His-Pro-Lys-Phe-Ile-Lys-
Glu-Leu-Arg-Val-Ile-Glu-Ser-Gly-Pro-His-Xaa6-RC
(SEQ ID NO:93); and

IL-8-1(34-50)-cyclo-(Xaa5-Xaa6)

a240) RN-Xaa5-Ala-Asn-Thr-Glu-Ile-Ile-Val-Lys-Leu-Ser-Asp-Gly-Arg-Glu-
Leu-Xaa6-RC
(SEQ ID NO:94).

In some embodiments, the IL-8 mimetics correspond to a cyclic portion of the C-terminal region of IL-8 and have the following sequences of amino acids (the underlined residues below are cyclized):

IL-8-1(50-72)-cyclo-(Xaa5-Xaa6)

a241) RN-Xaa5-Leu-Asp-Pro-Lys-Glu-Asn-Trp-Val-Gln-Arg-Val-Val-Glu-
Lys-Phe-Leu-Lys-Arg-Ala-Glu-Asn-Xaa6-RC
(SEQ ID NO:95);

IL-8-1(50-72)-cyclo-(Glu63-Lys67)

a242) RN-Ala-Leu-Asp-Pro-Lys-Glu-Asn-Trp-Val-Gln-Arg-Val-Val-Glu-
Lys-Phe-Leu-Lys-Arg-Ala-Glu-Asn-Ser-RC
(SEQ ID NO:96);

IL-8-1(50-72)-cyclo-(Lys67-Glu70)

a243) RN-Ala-Leu-Asp-Pro-Lys-Glu-Asn-Trp-Val-Gln-Arg-Val-Val-Glu-
Lys-Phe-Leu-Lys-Arg-Ala-Glu-Asn-Ser-RC
(SEQ ID NO:97); and

IL-8-1(50-72)-cyclo-(Lys64-Glu70)

a244) RN-Ala-Leu-Asp-Pro-Lys-Glu-Asn-Trp-Val-Gln-Arg-Val-Val-Glu-
Lys-Phe-Leu-Lys-Arg-Ala-Glu-Asn-Ser-RC
(SEQ ID NO:99).

In some embodiments, the IL-8 mimetics correspond to a portion the N-terminal region joined with a linker to a cyclic portion of the C-terminal region of IL-8 and have the following sequences of amino acids (the underlined residues below are cyclized):

IL-8-1(1-15)-[linker]-IL-8-1(56-71)-cyclo-(Glu63-Lys67)

a245) RN-Ser-Ala-Lys-Glu-Leu-Arg-Cys-Gln-Cys-Ile-Lys-Thr-Tyr-Ser-Lys-
[linker]-Asn-Trp-Val-Gln-Arg-Val-Val-Glu-Lys-Phe-Leu-Lys-Arg-
Ala-Glu-Asn-RC
(SEQ ID NO:100);

a246) RN-Ser-Ala-Lys-Glu-Leu-Arg-Xaa1-Gln-Xaa2-Ile-Lys-Thr-Tyr-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:101);

a247) RN-Xaa3-Ala-Lys-Glu-Leu-Arg-Xaa1-Gln-Xaa2-Ile-Lys-Thr-Tyr-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:102);

a248) RN-Ser-Xaa3-Lys-Glu-Leu-Arg-Xaa1-Gln-Xaa2-Ile-Lys-Thr-Tyr-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:103);

a249) RN-Ser-Ala-Xaa3-Glu-Leu-Arg-Xaa1-Gln-Xaa2-Ile-Lys-Thr-Tyr-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:104);

a250) RN-Ser-Ala-Lys-Glu-Leu-Arg-Xaa1-Gln-Xaa2-Xaa4-Lys-Thr-Tyr-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:105);

a251) RN-Xaa3-Ala-Lys-Glu-Leu-Arg-Xaa1-Gln-Xaa2-Xaa4-Lys-Thr-Tyr-
Ser-Lys-[linker]-Asn-Trp-Val-Gln-Arg-Val-Val-Glu-Lys-Phe-Leu-
Lys-Arg-Ala-Glu-Asn-RC
(SEQ ID NO:106);

a252) RN-Ser-Xaa3-Lys-Glu-Leu-Arg-Xaa1-Gln-Xaa2-Xaa4-Lys-Thr-Tyr-
Ser-Lys-[linker]-Asn-Trp-Val-Gln-Arg-Val-Val-Glu-Lys-Phe-Leu-
Lys-Arg-Ala-Glu-Asn-RC
(SEQ ID NO:107);

a253) RN-Ser-Ala-Xaa3-Glu-Leu-Arg-Xaa1-Gln-Xaa2-Xaa4-Lys-Thr-Tyr-Ser-
Lys-[linker]-Asn-Trp-Val-Gln-Arg-Val-Val-Glu-Lys-Phe-Leu-Lys-

Arg-Ala-Glu-Asn-R_{C}
(SEQ ID NO:108);
a254) R_{N}-Ser-Ala-Lys-Glu-Leu-Arg-Xaa₁-Gln-Xaa₂-Ile-Xaa₄-Thr-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:109);
a255) R_{N}-Xaa₃-Ala-Lys-Glu-Leu-Arg-Xaa₁-Gln-Xaa₂-Ile-Xaa₄-Thr-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:110);
a256) R_{N}-Ser-Xaa₃-Lys-Glu-Leu-Arg-Xaa₁-Gln-Xaa₂-Ile-Xaa₄-Thr-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:111);
a257) R_{N}-Ser-Ala-Xaa₃-Glu-Leu-Arg-Xaa₁-Gln-Xaa₂-Ile-Xaa₄-Thr-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:112);
a258) R_{N}-Ser-Ala-Lys-Glu-Leu-Arg-Xaa₁-Gln-Xaa₂-Ile-Lys-Xaa₄-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:113);
a259) R_{N}-Xaa₃-Ala-Lys-Glu-Leu-Arg-Xaa₁-Gln-Xaa₂-Ile-Lys-Xaa₄-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:114);
a260) R_{N}-Ser-Xaa₃-Lys-Glu-Leu-Arg-Xaa₁-Gln-Xaa₂-Ile-Lys-Xaa₄-Tyr-Ser-
Lys-[linker]-Asn-Trp-Val-Gln-Arg-Val-Val-Glu-Lys-Phe-Leu-Lys-
Arg-Ala-Glu-Asn-R_{C}
(SEQ ID NO:115);

a261) RN-Ser-Ala-Xaa3-Glu-Leu-Arg-Xaa1-Gln-Xaa2-Ile-Lys-Xaa4-Tyr-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:116);

a262) RN-Ser-Ala-Lys-Glu-Leu-Arg-Xaa1-Gln-Xaa2-Ile-Lys-Thr-Xaa4-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:117);

a263) RN-Xaa3-Ala-Lys-Glu-Leu-Arg-Xaa1-Gln-Xaa2-Ile-Lys-Thr-Xaa4-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:118);

a264) RN-Ser-Xaa3-Lys-Glu-Leu-Arg-Xaa1-Gln-Xaa2-Ile-Lys-Thr-Xaa4-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:119);

a265) RN-Ser-Ala-Xaa3-Glu-Leu-Arg-Xaa1-Gln-Xaa2-Ile-Lys-Thr-Xaa4-Ser-
Lys-[linker]-Asn-Trp-Val-Gln-Arg-Val-Val-Glu-Lys-Phe-Leu-Lys-
Arg-Ala-Glu-Asn-RC
(SEQ ID NO:120);

a266) RN-Ser-Ala-Lys-Glu-Leu-Arg-Xaa1-Gln-Xaa2-Ile-Lys-Thr-Tyr-Xaa4-
Lys-[linker]-Asn-Trp-Val-Gln-Arg-Val-Val-Glu-Lys-Phe-Leu-Lys-
Arg-Ala-Glu-Asn-RC
(SEQ ID NO:121);

a267) RN-Xaa3-Ala-Lys-Glu-Leu-Arg-Xaa1-Gln-Xaa2-Ile-Lys-Thr-Tyr-Xaa4-
Lys-[linker]-Asn-Trp-Val-Gln-Arg-Val-Val-Glu-Lys-Phe-Leu-Lys-
Arg-Ala-Glu-Asn-RC
(SEQ ID NO:122);

a268) RN-Ser-Xaa3-Lys-Glu-Leu-Arg-Xaa1-Gln-Xaa2-Ile-Lys-Thr-Tyr-Xaa4-
Lys-[linker]-Asn-Trp-Val-Gln-Arg-Val-Val-Glu-Lys-Phe-Leu-Lys-

Arg-Ala-Glu-Asn-R_{C}
(SEQ ID NO:123);
a269) R_{N}-Ser-Ala-Xaa₃-Glu-Leu-Arg-Xaa₁-Gln-Xaa₂-Ile-Lys-Thr-Tyr-Xaa₄-
Lys-[linker]-Asn-Trp-Val-Gln-Arg-Val-Val-Glu-Lys-Phe-Leu-Lys-
Arg-Ala-Glu-Asn-R_{C}
(SEQ ID NO:124);
a270) R_{N}-Ser-Ala-Lys-Glu-Leu-Arg-Xaa₁-Gln-Xaa₂-Ile-Lys-Thr-Tyr-Ser-
Xaa₄-[linker]-Asn-Trp-Val-Gln-Arg-Val-Val-Glu-Lys-Phe-Leu-Lys-
Arg-Ala-Glu-Asn-R_{C}
(SEQ ID NO:125);
a271) R_{N}-Xaa₃-Ala-Lys-Glu-Leu-Arg-Xaa₁-Gln-Xaa₂-Ile-Lys-Thr-Tyr-Ser-
Xaa₄-[linker]-Asn-Trp-Val-Gln-Arg-Val-Val-Glu-Lys-Phe-Leu-Lys-
Arg-Ala-Glu-Asn-R_{C}
(SEQ ID NO:126);
a272) R_{N}-Ser-Xaa₃-Lys-Glu-Leu-Arg-Xaa₁-Gln-Xaa₂-Ile-Lys-Thr-Tyr-Ser-
Xaa₄-[linker]-Asn-Trp-Val-Gln-Arg-Val-Val-Glu-Lys-Phe-Leu-Lys-
Arg-Ala-Glu-Asn-R_{C}
(SEQ ID NO:127);
a273) R_{N}-Ser-Ala-Xaa₃-Glu-Leu-Arg-Xaa₁-Gln-Xaa₂-Ile-Lys-Thr-Tyr-Ser-
Xaa₄-[linker]-Asn-Trp-Val-Gln-Arg-Val-Val-Glu-Lys-Phe-Leu-Lys-
Arg-Ala-Glu-Asn-R_{C}
(SEQ ID NO:128);

IL-8-1(1-13)-[linker]-IL-8-1(56-71)-cyclo-(Glu⁶³-Lys⁶⁷)

a274) R_{N}-Ser-Ala-Lys-Glu-Leu-Arg-Cys-Gln-Cys-Ile-Lys-Thr-Tyr-[linker]-
Asn-Trp-Val-Gln-Arg-Val-Val-Glu-Lys-Phe-Leu-Lys-Arg-Ala-Glu-
Asn-R_{C}
(SEQ ID NO:129);

a275) R_{N}-Ser-Ala-Lys-Glu-Leu-Arg-Xaa₁-Gln-Xaa₂-Ile-Lys-Thr-Tyr-
[linker]-Asn-Trp-Val-Gln-Arg-Val-Val-Glu-Lys-Phe-Leu-Lys-Arg-

Ala-Glu-Asn-R_C
(SEQ ID NO:130);
a276) R_N-Xaa_3-Ala-Lys-Glu-Leu-Arg-Xaa_1-Gln-Xaa_2-Ile-Lys-Thr-Tyr-
[linker]-Asn-Trp-Val-Gln-Arg-Val-Val-Glu-Lys-Phe-Leu-Lys-Arg-
Ala-Glu-Asn-R_C
(SEQ ID NO:131);
a277) R_N-Ser-Xaa_3-Lys-Glu-Leu-Arg-Xaa_1-Gln-Xaa_2-Ile-Lys-Thr-Tyr-
[linker]-Asn-Trp-Val-Gln-Arg-Val-Val-Glu-Lys-Phe-Leu-Lys-Arg-
Ala-Glu-Asn-R_C
(SEQ ID NO:132);
a278) R_N-Ser-Ala-Xaa_3-Glu-Leu-Arg-Xaa_1-Gln-Xaa_2-Ile-Lys-Thr-Tyr-
[linker]-Asn-Trp-Val-Gln-Arg-Val-Val-Glu-Lys-Phe-Leu-Lys-Arg-
Ala-Glu-Asn-R_C
(SEQ ID NO:133);
a279) R_N-Ser-Ala-Lys-Glu-Leu-Arg-Xaa_1-Gln-Xaa_2-Xaa_4-Lys-Thr-Tyr-
[linker]-Asn-Trp-Val-Gln-Arg-Val-Val-Glu-Lys-Phe-Leu-Lys-Arg-
Ala-Glu-Asn-R_C
(SEQ ID NO:134);
a280) R_N-Xaa_3-Ala-Lys-Glu-Leu-Arg-Xaa_1-Gln-Xaa_2-Xaa_4-Lys-Thr-Tyr-
[linker]-Asn-Trp-Val-Gln-Arg-Val-Val-Glu-Lys-Phe-Leu-Lys-Arg-
Ala-Glu-Asn-R_C
(SEQ ID NO:135);
a281) R_N-Ser-Xaa_3-Lys-Glu-Leu-Arg-Xaa_1-Gln-Xaa_2-Xaa_4-Lys-Thr-Tyr-
[linker]-Asn-Trp-Val-Gln-Arg-Val-Val-Glu-Lys-Phe-Leu-Lys-Arg-
Ala-Glu-Asn-R_C
(SEQ ID NO:136);
a282) R_N-Ser-Ala-Xaa_3-Glu-Leu-Arg-Xaa_1-Gln-Xaa_2-Xaa_4-Lys-Thr-Tyr-
[linker]-Asn-Trp-Val-Gln-Arg-Val-Val-Glu-Lys-Phe-Leu-Lys-Arg-
Ala-Glu-Asn-R_C
(SEQ ID NO:137);

a283) R_{N}-Ser-Ala-Lys-Glu-Leu-Arg-Xaa₁-Gln-Xaa₂-Ile-Xaa₄-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:138);

a284) R_{N}-Xaa₃-Ala-Lys-Glu-Leu-Arg-Xaa₁-Gln-Xaa₂-Ile-Xaa₄-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:139);

a285) R_{N}-Ser-Xaa₃-Lys-Glu-Leu-Arg-Xaa₁-Gln-Xaa₂-Ile-Xaa₄-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:140);

a286) R_{N}-Ser-Ala-Xaa₃-Glu-Leu-Arg-Xaa₁-Gln-Xaa₂-Ile-Xaa₄-Thr-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:141);

a287) R_{N}-Ser-Ala-Lys-Glu-Leu-Arg-Xaa₁-Gln-Xaa₂-Ile-Lys-Xaa₄-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:142);

a288) R_{N}-Xaa₃-Ala-Lys-Glu-Leu-Arg-Xaa₁-Gln-Xaa₂-Ile-Lys-Xaa₄-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:143);

a289) R_{N}-Ser-Xaa₃-Lys-Glu-Leu-Arg-Xaa₁-Gln-Xaa₂-Ile-Lys-Xaa₄-Tyr-
[linker]-Asn-Trp-Val-Gln-Arg-Val-Val-Glu-Lys-Phe-Leu-Lys-Arg-
Ala-Glu-Asn-R_{C}
(SEQ ID NO:144);

a290) R_{N}-Ser-Ala-Xaa₃-Glu-Leu-Arg-Xaa₁-Gln-Xaa₂-Ile-Lys-Xaa₄-Tyr-
[linker]-Asn-Trp-Val-Gln-Arg-Val-Val-Glu-Lys-Phe-Leu-Lys-Arg-

Ala-Glu-Asn-R_{C}
(SEQ ID NO:145);
a291) R_{N}-Ser-Ala-Lys-Glu-Leu-Arg-Xaa₁-Gln-Xaa₂-Ile-Lys-Thr-Xaa₄-
[linker]-Asn-Trp-Val-Gln-Arg-Val-Val-Glu-Lys-Phe-Leu-Lys-Arg-
Ala-Glu-Asn-R_{C}
(SEQ ID NO:146);
a292) R_{N}-Xaa₃-Ala-Lys-Glu-Leu-Arg-Xaa₁-Gln-Xaa₂-Ile-Lys-Thr-Xaa₄-
[linker]-Asn-Trp-Val-Gln-Arg-Val-Val-Glu-Lys-Phe-Leu-Lys-Arg-
Ala-Glu-Asn-R_{C}
(SEQ ID NO:147);
a293) R_{N}-Ser-Xaa₃-Lys-Glu-Leu-Arg-Xaa₁-Gln-Xaa₂-Ile-Lys-Thr-Xaa₄-
[linker]-Asn-Trp-Val-Gln-Arg-Val-Val-Glu-Lys-Phe-Leu-Lys-Arg-
Ala-Glu-Asn-R_{C}
(SEQ ID NO:148);
a294) R_{N}-Ser-Ala-Xaa₃-Glu-Leu-Arg-Xaa₁-Gln-Xaa₂-Ile-Lys-Thr-Xaa₄-
[linker]-Asn-Trp-Val-Gln-Arg-Val-Val-Glu-Lys-Phe-Leu-Lys-Arg-
Ala-Glu-Asn-R_{C}
(SEQ ID NO:149);

IL-8-1(1-11)-[linker]-IL-8-(56-71)-cyclo-(Glu63-Lys67)

a295) RN-Ser-Ala-Lys-Glu-Leu-Arg-Cys-Gln-Cys-Ile-Lys-[linker]-Asn-Trp-
Val-Gln-Arg-Val-Val-Glu-Lys-Phe-Leu-Lys-Arg-Ala-Glu-Asn-RC
(SEQ ID NO:150);

a296) RN-Ser-Ala-Lys-Glu-Leu-Arg-Xaa1-Gln-Xaa2-Ile-Lys-[linker]-Asn-
Trp-Val-Gln-Arg-Val-Val-Glu-Lys-Phe-Leu-Lys-Arg-Ala-Glu-Asn-
RC
(SEQ ID NO:151);

a297) RN-Xaa3-Ala-Lys-Glu-Leu-Arg-Xaa1-Gln-Xaa2-Ile-Lys-[linker]-Asn-
Trp-Val-Gln-Arg-Val-Val-Glu-Lys-Phe-Leu-Lys-Arg-Ala-Glu-Asn-
RC
(SEQ ID NO:152);

a298) R_{N}-Ser-Xaa₃-Lys-Glu-Leu-Arg-Xaa₁-Gln-Xaa₂-Ile-Lys-[linker]-Asn-
Trp-Val-Gln-Arg-Val-Val-Glu-Lys-Phe-Leu-Lys-Arg-Ala-Glu-Asn-
R_{C}
(SEQ ID NO:153);

a299) R_{N}-Ser-Ala-Xaa₃-Glu-Leu-Arg-Xaa₁-Gln-Xaa₂-Ile-Lys-[linker]-Asn-
Trp-Val-Gln-Arg-Val-Val-Glu-Lys-Phe-Leu-Lys-Arg-Ala-Glu-Asn-
R_{C}
(SEQ ID NO:154);

a300) R_{N}-Ser-Ala-Lys-Glu-Leu-Arg-Xaa₁-Gln-Xaa₂-Xaa₄-Lys-[linker]-Asn-
Trp-Val-Gln-Arg-Val-Val-Glu-Lys-Phe-Leu-Lys-Arg-Ala-Glu-Asn-
R_{C}
(SEQ ID NO:155);

a301) R_{N}-Xaa₃-Ala-Lys-Glu-Leu-Arg-Xaa₁-Gln-Xaa₂-Xaa₄-Lys-[linker]-
Asn-Trp-Val-Gln-Arg-Val-Val-Glu-Lys-Phe-Leu-Lys-Arg-Ala-Glu-
Asn-R_{C}
(SEQ ID NO:156);

a302) R_{N}-Ser-Xaa₃-Lys-Glu-Leu-Arg-Xaa₁-Gln-Xaa₂-Xaa₄-Lys-[linker]-
Asn-Trp-Val-Gln-Arg-Val-Val-Glu-Lys-Phe-Leu-Lys-Arg-Ala-Glu-
Asn-R_{C}
(SEQ ID NO:157);

a303) R_{N}-Ser-Ala-Xaa₃-Glu-Leu-Arg-Xaa₁-Gln-Xaa₂-Xaa₄-Lys-[linker]-
Asn-Trp-Val-Gln-Arg-Val-Val-Glu-Lys-Phe-Leu-Lys-Arg-Ala-Glu-
Asn-R_{C}
(SEQ ID NO:158);

a304) R_{N}-Ser-Ala-Lys-Glu-Leu-Arg-Xaa₁-Gln-Xaa₂-Ile-Xaa₄-[linker]-Asn-
Trp-Val-Gln-Arg-Val-Val-Glu-Lys-Phe-Leu-Lys-Arg-Ala-Glu-Asn-
R_{C}
(SEQ ID NO:159);

a305) R_{N}-Xaa₃-Ala-Lys-Glu-Leu-Arg-Xaa₁-Gln-Xaa₂-Ile-Xaa₄-[linker]-Asn-
Trp-Val-Gln-Arg-Val-Val-Glu-Lys-Phe-Leu-Lys-Arg-Ala-Glu-Asn-

RC
(SEQ ID NO:160);
a306) RN-Ser-Xaa3-Lys-Glu-Leu-Arg-Xaa1-Gln-Xaa2-Ile-Xaa4-{linker]-Asn-
Trp-Val-Gln-Arg-Val-Val-Glu-Lys-Phe-Leu-Lys-Arg-Ala-Glu-Asn-
RC
(SEQ ID NO:161); and
a307) RN-Ser-Ala-Xaa3-Glu-Leu-Arg-Xaa1-Gln-Xaa2-Ile-Xaa4-[linker]-Asn-
Trp-Val-Gln-Arg-Val-Val-Glu-Lys-Phe-Leu-Lys-Arg-Ala-Glu-Asn-
RC
(SEQ ID NO:162).

In some embodiments, the IL-8 mimetics can be specifically defined and have the following sequences of amino acids:

IL-8-1(1-15)-NH2

a308) H-Ser-Ala-Lys-Glu-Leu-Arg-Cys-Gln-Cys-Ile-Lys-Thr-Tyr-Ser-Lys-
NH2 (SEQ ID NO:1641);

IL-8-1(56-71)-NH2

a309) H-Asn-Trp-Val-Gln-Arg-Val-Val-Glu-Lys-Phe-Leu-Lys-Arg-Ala-Glu-
Asn-NH2
(SEQ ID NO:1642);

IL-8-1(1-11)-[Gly]4-IL-8-1(56-71)-NH2

a310) H-Ser-Ala-Lys-Glu-Leu-Arg-Cys-Gln-Cys-Ile-Lys-[Gly]4-Asn-Trp-
Val-Gln-Arg-Val-Val-Glu-Lys-Phe-Leu-Lys-Arg-Ala-Glu-Asn-NH2
(SEQ ID NO:1643);

IL-8-1(1-13)-[Gly]4-IL-8-1(56-71)-NH2

a311) H-Ser-Ala-Lys-Glu-Leu-Arg-Cys-Gln-Cys-Ile-Lys-Thr-Tyr-[Gly]4-
Asn-Trp-Val-Gln-Arg-Val-Val-Glu-Lys-Phe-Leu-Lys-Arg-Ala-Glu-

Asn-NH2
(SEQ ID NO:1644);

IL-8-1(1-15)-[Gly]4-IL-8-1(56-71)-NH2

a312) H-Ser-Ala-Lys-Glu-Leu-Arg-Cys-Gln-Cys-Ile-Lys-Thr-Tyr-Ser-Lys-
[Gly]4-Asn-Trp-Val-Gln-Arg-Val-Val-Glu-Lys-Phe-Leu-Lys-Arg-Ala-
Glu-Asn-NH2
(SEQ ID NO:1645);

IL-8-1(1-15)-[linker]-IL-8-1(56-71)-NH2

a313) H-Ser-Ala-Lys-Glu-Leu-Arg-Cys-Gln-Cys-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1646);

[Ala7, Phe9]-IL-8-1(1-15)-[linker]-IL-8-1(56-71)-NH2

a314) H-Ser-Ala-Lys-Glu-Leu-Arg-Ala-Gln-Phe-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1647);

[Ala⁷, Phe⁹]-IL-8-1(1-15)-[linker]-IL-8-1(56-71)-NH₂

a315) H-Ser-Ala-Lys-Glu-Leu-Arg-Ala-Gln-Phe-Ile-Lys-Thr-Tyr-Ser-Lys-
[8-amino-3,6-dioxoctanoic acid]-Asn-Trp-Val-Gln-Arg-Val-Val-Glu-
Lys-Phe-Leu-Lys-Arg-Ala-Glu-Asn-NH₂
(SEQ ID NO:1648);

[Ser7, Ser9]-IL-8-1(1-15)-[linker]-IL-8-1(56-71)-NH2

a316) H-Ser-Ala-Lys-Glu-Leu-Arg-Ser-Gln-Ser-Ile-Lys-Thr-Tyr-Ser-Lys-
[11-aminoundecanoic acid]-Asn-Trp-Val-Gln-Arg-Val-Val-Glu-Lys-

Phe-Leu-Lys-Arg-Ala-Glu-Asn-NH2
(SEQ ID NO:1649);

Ac-[Ala7, Phe9]-IL-8-1(1-15)-[linker]-IL-8-1(56-71)-NH2

a317) Ac-Ser-Ala-Lys-Glu-Leu-Arg-Ala-Gln-Phe-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1650);

Ac-[Ser7, Ser9]-IL-8-1(1-15)-[linker]-IL-8-1(56-71 -NH2

a318) Ac-Ser-Ala-Lys-Glu-Leu-Arg-Ser-Gln-Ser-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1651);

[Ala7, Tyr9]-IL-8-1(1-15)-[linker]-IL-8-1(56-71)-NH2

a319) H-Ser-Ala-Lys-Glu-Leu-Arg-Ala-Gln-Tyr-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1652);

Ac-[Ala7, Tyr9]-IL-8-1(1-15)-[linker]-IL-8-1(56-71)-NH2

a320) Ac-Ser-Ala-Lys-Glu-Leu-Arg-Ala-Gln-Tyr-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1653);

[Tyr7, Phe9]-IL-8-1(1-15)-[linker]-IL-8-1(56-71)-NH2

a321) H-Ser-Ala-Lys-Glu-Leu-Arg-Tyr-Gln-Phe-Ile-Lys-Thr-Tyr-Ser-Lys-
[11-aminoundecanoic acid]-Asn-Trp-Val-Gln-Arg-Val-Val-Glu-Lys-

Phe-Leu-Lys-Arg-Ala-Glu-Asn-NH2
(SEQ ID NO:1654);

Ac-[Tyr7, Phe9]-IL-8-1(1-15)-[linker]-IL-8-1(56-71)-NH2

a322) Ac-Ser-Ala-Lys-Glu-Leu-Arg-Tyr-Gln-Phe-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1655);

[Tyr7, Ala9]-IL-8-1(1-15)-[linker]-IL-8-1(56-71)-NH2

a323) H-Ser-Ala-Lys-Glu-Leu-Arg-Tyr-Gln-Ala-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1656);

Ac-[Tyr⁷, Ala⁹]-IL-8-1(1-15)-[linker]-IL-8-1(56-71)-NH₂

a324) Ac-Ser-Ala-Lys-Glu-Leu-Arg-Tyr-Gln-Ala-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH₂
(SEQ ID NO:1657);

[Tyr7, Tyr9]-IL-8-1(1-15)-[linker]-IL-8-1(56-71)-NH2

a325) H-Ser-Ala-Lys-Glu-Leu-Arg-Tyr-Gln-Tyr-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1658);

Ac-[Tyr7, Tyr9]-IL-8-1(1-15)-[linker]-IL-8-1(56-71)-NH2

a326) Ac-Ser-Ala-Lys-Glu-Leu-Arg-Tyr-Gln-Tyr-Ile-Lys-Thr-Tyr-Ser-Lys-
[11-aminoundecanoic acid]-Asn-Trp-Val-Gln-Arg-Val-Val-Glu-Lys-

Phe-Leu-Lys-Arg-Ala-Glu-Asn-NH2
(SEQ ID NO:1659);

Ac-[Ala7, Phe9, Arg11]-IL-8-1(1-15)-[linker]-IL-8-1(56-71)-NH2

a327) Ac-Ser-Ala-Lys-Glu-Leu-Arg-Ala-Gln-Phe-Ile-Arg-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO: 1660);

Ac-[Tyr7, Phe9, Arg11]-IL-8-1(1-15)-[linker]-IL-8-1(56-71)-NH2

a328) Ac-Ser-Ala-Lys-Glu-Leu-Arg-Tyr-Gln-Phe-Ile-Arg-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1661);

Ac-[Tyr7, Phe9, Arg11, Arg15]-IL-8-1(1-15)-[linker]-IL-8-1(56-71)-NH2

a329) Ac-Ser-Ala-Lys-Glu-Leu-Arg-Tyr-Gln-Phe-Ile-Arg-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1662);

[Ala7, Phe9]-IL-8-1(1-15)-[linker]-IL-8-1(56-71)-cyclo-(Glu63-Lys67)-NH2

a330) H-Ser-Ala-Lys-Glu-Leu-Arg-Ala-Gln-Phe-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1663);

Ac-[Ala7, Phe9]-IL-8-1(1-15)-[linker]-IL-8-1(56-71)-cyclo-(Glu63-Lys67)-NH2

a331) Ac-Ser-Ala-Lys-Glu-Leu-Arg-Ala-Gln-Phe-Ile-Lys-Thr-Tyr-Ser-Lys-
[11-aminoundecanoic acid]-Asn-Trp-Val-Gln-Arg-Val-Val-Glu-Lys-

Phe-Leu-Lys-Arg-Ala-Glu-Asn-NH2
(SEQ ID NO:1664);

Ac-[Ala7, Tyr9]-IL-8-1(1-15)-[linker]-IL-8-1(56-71)-cyclo-(Glu63-Lys67)-NH2

a332) Ac-Ser-Ala-Lys-Glu-Leu-Arg-Ala-Gln-Tyr-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1665);

Ac-[Tyr7, Phe9]-IL-8-1(1-15)-[linker]-IL-8-1(56-71)-cyclo-(Glu63-Lys67)-NH2

a333) Ac-Ser-Ala-Lys-Glu-Leu-Arg-Tyr-Gln-Phe-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1666);

Ac-[Tyr7, Tyr9]-IL-8-1(1-15)-[linker]-IL-8-(56-71)-cyclo-(Glu63-Lys67)-NH2

a334) Ac-Ser-Ala-Lys-Glu-Leu-Arg-Tyr-Gln-Tyr-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1667);

Ac-[His7, Tyr9]-IL-8-1(1-15)-[linker]-IL-8-(56-71)-cyclo-(Glu63-Lys67)-NH2

a335) Ac-Ser-Ala-Lys-Glu-Leu-Arg-His-Gln-Tyr-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1668);

Ac-[Tyr⁷, His⁹]-IL-8-1(1-15)-[linker]-IL-8-1(56-71)-cyclo-(Glu⁶³-Lys⁶⁷)-NH₂

a336) Ac-Ser-Ala-Lys-Glu-Leu-Arg-Tyr-Gln-His-Ile-Lys-Thr-Tyr-Ser-Lys-
[11-aminoundecanoic acid]-Asn-Trp-Val-Gln-Arg-Val-Val-Glu-Lys-

Phe-Leu-Lys-Arg-Ala-Glu-Asn-NH2
(SEQ ID NO:1669);

Ac-[Tyr7, Trp9]-IL-8-1(1-15)-[linker]-IL-8-1(56-71)-cyclo-(Glu63-Lys67)-NH2

a337) Ac-Ser-Ala-Lys-Glu-Leu-Arg-Tyr-Gln-Trp-Ile-Lys-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1670);

Ac-[Ala7, Phe9, Arg11]-IL-8-1(1-15)-[linker]-IL-8-1(56-71)-cyclo-(Glu63-Lys67)-NH2

a338) Ac-Ser-Ala-Lys-Glu-Leu-Arg-Ala-Gln-Phe-Ile-Arg-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1671);

Ac-[Tyr7, Trp9, Arg11]-IL-8-1(1-15)-[linker]-IL-8-1(56-71)-cyclo-(Glu63-Lys67)-NH2

a339) Ac-Ser-Ala-Lys-Glu-Leu-Arg-Tyr-Gln-Trp-Ile-Arg-Thr-Tyr-Ser-Lys-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1672);

Ac-[Tyr7, Trp9, Arg11, Arg15]-IL-8-1(1-15)-[linker]-IL-8-1(56-71)-cyclo-(Glu63-Lys67)-NH2

a340) Ac-Ser-Ala-Lys-Glu-Leu-Arg-Tyr-Gln-Trp-Ile-Arg-Thr-Tyr-Ser-Arg-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1673);

Ac-[Trp⁷, Trp⁹, Arg¹¹, Arg¹⁵]-IL-8-1(1-15)-[linker]-IL-8-1(56-71)-cyclo-(Glu⁶³-Lys⁶⁷)-NH₂

a341) Ac-Ser-Ala-Lys-Glu-Leu-Arg-Trp-Gln-Trp-Ile-Arg-Thr-Tyr-Ser-Arg-
[11-aminoundecanoic acid]-Asn-Trp-Val-Gln-Arg-Val-Val-Glu-Lys-

Phe-Leu-Lys-Arg-Ala-Glu-Asn-NH2
(SEQ ID NO:1674); and

Ac-[His7, Trp9, Arg11, Arg15]-IL-8-1(1-15)-[linker]-IL-8-1(56-71)-cyclo-(Glu63-Lys67)-NH2

a342) Ac-Ser-Ala-Lys-Glu-Leu-Arg-His-Gln-Trp-Ile-Arg-Thr-Tyr-Ser-Arg-
[11-aminoundecanoic acid]-Asn-Trp-Val-Gln-Arg-Val-Val-Glu-Lys-
Phe-Leu-Lys-Arg-Ala-Glu-Asn-NH2
(SEQ ID NO:1675).

wherein,
R_{N} can be independently selected from substituted, unsubstituted, hetero-, straight-chained, branched, cyclic, saturated or unsaturated aliphatic radicals; or a substituted, unsubstituted, or hetero- aromatic radicals; PEG and derivatives thereof; and any other modifying group;
R_{C} can be independently selected from substituted, unsubstituted, hetero-, straight-chained, branched, cyclic, saturated or unsaturated aliphatic radical; or a substituted, unsubstituted, or hetero- aromatic radicals, PEG and derivatives thereof; and any other modifying group;
**Xaa**_{**1**} can be any L- or D-natural amino acid and any non-natural amino acid;
**Xaa**_{**2**} can be any L- or D-natural amino acid and any non-natural amino acid;
**Xaa**_{**3**} can be L-Pro, D-Pro, **P*, Btd** and any L- or D-natural and non-natural amino acid;
**Xaa**_{**4**} can be **P*, Btd** and any L- or D-natural amino acid and any non-natural amino acid, wherein
**P*** can be represented by a formula 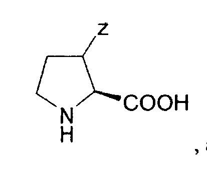 and
**Btd** can be represented by formula 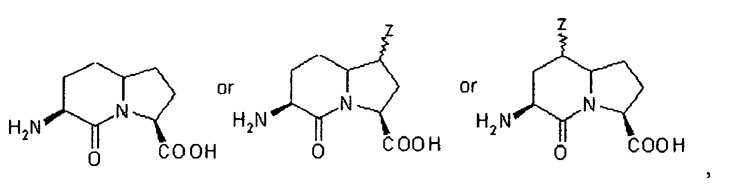 wherein, z can be a hydrogen, can be independently selected from substituted, unsubstituted, hetero-, straight-chained, branched, cyclic, saturated or unsaturated aliphatic radical; or a substituted, unsubstituted, or heteroaromatic radicals; PEG and derivatives thereof; any other functional group.

In some embodiments, z can be any functional group taught herein. In other embodiments, z can be hydrogen; hydroxyl, amino, sulfhydryl; aminoalkyl, alkylcarbonyl, arylcarbonyl, aryl; polyethylene glycol, heparin, hirudin, hyaluronic acid; and, any derivative, analog, homolog or combination thereof. In other embodiments, z can be any agent taught herein.
**Xaa**_{**5**} can be any L- or D-natural amino acid and any non-natural amino acid with functional side chain to allow cyclization with Xaa₆.
**Xaa**_{**6**} can be any L- or D-natural amino acid and any non-natural amino acid with functional side chain to allow cyclization with Xaa₅;
the linker can be optional and can comprise, for example, any natural or non-natural amino acid; and,
any additional amino acids identified in square brackets in a header indicates that the peptide described by the header is at least an analog of a native fragment, and the position of the amino acid substitutions are noted by the standard abbreviation for the amino acid substituent with a superscript number indicating the residue positions at which the modification has occurred.
the underlining indicates that the underlined residues are cyclized, and the cyclization can include formation of a disulphide bond, an alkenyl bond, an ether bond, or a lactam.

The amino acids used in the present invention may be organic compounds comprising an amino group and a carboxyl group, and the amino group may be primary or secondary. Examples of amino acids include, but are not limited to, glycine, alanine, valine, leucine, isoleucine, methionine, phenylalanine, tyrosine, aspartic acid, glutamic acid, lysine, arginine, serine, threonine, cysteine, asparagine, proline, tryptophan, histidine and combinations thereof. In some embodiments, the amino acids are represented by a formula: 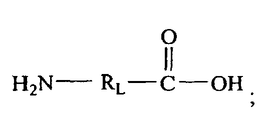 wherein R_{L} may be a substituted, unsubstituted, hetero-, straight-chained, branched, cyclic, saturated or unsaturated aliphatic radical; or a substituted, unsubstituted, or heteroaromatic radical. In some embodiments, R_{L} can be substituted, unsubstituted, or hetero-forms of methyl, iso-propyl, sec-butyl, iso-butyl, benzyl, or a combination thereof.

In embodiments where R_{L} is substituted, examples of substitutents include, but are not limited to, hydroxyl, carboxyl, amino, imino groups and combinations thereof. In embodiments where R_{L} is heteroaliphatic, examples of heteroatoms include, but are not limited to, sulfur, phosphorous, oxygen, nitrogen and combinations thereof. In other embodiments, R_{L} can comprise substituted or unsubstituted poly(alkylene glycols), which include, but are not limited to, PEG, PEG derivatives such as mPEG, poly(ethylene oxide), PPG, poly(tetramethylene glycol), poly(ethylene oxide-co-propylene oxide), or copolymers and combinations thereof.

In some embodiments, the amino acids may be limited to bifunctional amino acids. In some embodiments, the amino acids may be limited to trifunctional amino acids. In some embodiments, the amino acids may be limited to diamines. In some embodiments, the amino acids may be limited to triamines. In some embodiments, the amino acids may be limited to monocarboxylics. In some embodiments, the amino acids may be limited to dicarboxylics. In some embodiments, the amino acids may be limited to aliphatics. In some embodiments, the amino acids may be limited to aromatics. In some embodiments, the amino acids may be limited to amides. In some embodiments, the amino acids may not include lysine. It is to be appreciated that one skilled in the art should recognize that some of the groups, subgroups, and individual amino acids may not be used in some embodiments of the present invention.

In some embodiments, R_{L} can be a substituted or unsubstituted alkylene comprising Cₙ carbons in the alkylene backbone, wherein n is an integer ranging from 1 to about 20; from about 2 to about 16; from about 3 to about 12; from about 4 to about 10; from about 3 to about 8, and any range therein. In these embodiments, R_{L} can be, for example, 11-amino-undecanoic acid.

In other embodiments, the linker comprises any combination of natural or non-natural amino acids, (Xaa), wherein the number of amino acids ranges from 1 to about 50; from about 2 to about 40; from about 3 to about 30, or any range therein. In other embodiments, R_{L} comprises any combination of four natural or non-natural amino acids such as, for example, -(Gly)₄-, otherwise referred to as SEQ ID NO:1640 in the present application.

In some embodiments, there is no linker. In other embodiments, the mimetics are comprised of portions of the human CXC chemokine IL-8 that are connected directly to each other through amide bonds. In other embodiments, the mimetics are comprised of the human CXC chemokine IL-8 that are connected by disulfide bonds such as, the disulfide bonds that can form between Cys residues. In other embodiments, R_{N} can comprise the peptide sequence Glu-Leu-Arg and a linker. In other embodiments, R_{N} can comprise the peptide sequence Glu-Leu-Arg and a linker, wherein the Glu-Leu-Arg are the last three residues in R_{N} and the linker attaches R_{N} to a C-terminal region containing at least SEQ ID NO:26.

It is to be appreciated that a wide variety of amino acid substitutions may also be made in the polypeptide sequences. Examples of such substitutions include, but are not limited to, substituting lysine for glutamic acid, lysine for aspartic acid, ornithine for glutamic acid, and ornithine for aspartic acid.

The IL-8 mimetics may be prepared, analyzed and modified using techniques known to those skilled in the art. In some embodiments, a peptide or polypeptide component of a CXCR1 or CXCR2 agonist may be comprised of a peptide synthesized using standard techniques such as, for example, a commercially available automated peptide synthesizer. In other embodiments, the peptides and polypeptides may be purified by HPLC and analyzed by mass spectrometry. In other embodiments, the peptides and polypeptides may be assayed for CXCR1 or CXCR2 agonist activity in accordance with standard methods. In other embodiments, the peptides and polypeptides may be dimerized through a disulfide bridge formed by gentle oxidation of cysteines with 10% dimethyl sulfoxide (DMSO) in water, and the dimer formation may be verified using mass spectrometry after HPLC purification.

In some embodiments, one or more modifying groups may be attached to the IL-8 mimetics at any suitable reactive group on the IL-8 mimetic such as, for example, an amino group, a carboxyl group, or a hydroxyl group using methods known to those skilled in the art. As described above, examples of chemical connections used to attach the modifying groups include, but are not limited to, ethers, amides, esters, anhydride, orthoester, alkylamine, sulphide, disulphide, carbamate, all-aromatic carbonate, urea bonds, and the like.

### Pharmaceutical Compositions

The invention further provides pharmaceutical compositions containing the IL-8 mimetics. The pharmaceutical compositions include an IL-8 mimetic in an amount that is diagnostic, therapeutic and/or prophylactic in the diagnosis, prevention, treatment and amelioration of symptoms of disease.

The amount of an IL-8 mimetic used in the compositions can vary according to factors such as type of disease, age, sex, and weight of the subject. Dosage regimens may be adjusted to optimize a therapeutic response. In some embodiments, a single bolus may be administered; several divided doses may be administered over time; the dose may be proportionally reduced or increased; or any combination thereof, as indicated by the exigencies of the therapeutic situation and factors known one of skill in the art. It is to be noted that dosage values may vary with the severity of the condition to be alleviated. Dosage regimens may be adjusted over time according to the individual need and the professional judgment of the person administering or supervising the administration of the compositions, and the dosage ranges set forth herein are exemplary only and do not limit the dosage ranges that may be selected by medical practitioners.

The terms "administration" or "administering" refer to a method of incorporating a compound into the cells or tissues of a subject, either *in vivo* or *ex vivo* to diagnose, prevent, treat, or ameliorate a symptom of a disease. In one example, a compound can be administered to a subject *in vivo* parenterally. In another example, a compound can be administered to a subject by combining the compound with cell tissue from the subject *ex vivo* for purposes that include, but are not limited to, cell expansion and mobilization assays. When the compound is incorporated in the subject in combination with one or active agents, the terms "administration" or "administering" can include sequential or concurrent incorporation of the compound with the other agents such as, for example, any agent described above. A pharmaceutical composition of the invention is formulated to be compatible with its intended route of administration. Examples of routes of administration include, but are not limited to, parenteral such as, for example, intravenous, intradermal, intramuscular, and subcutaneous injection; oral; inhalation; intranasal; transdermal; transmucosal; and rectal administration.

An "effective amount" of a compound of the invention can be used to describe a therapeutically effective amount or a prophylactically effective amount. A "therapeutically effective amount" refers to an amount that is effective at the dosages and periods of time necessary to achieve a desired therapeutic result and may also refer to an amount of active compound, prodrug or pharmaceutical agent that elicits any biological or medicinal response in a tissue, system, or subject that is sought by a researcher, veterinarian, medical doctor or other clinician that may be part of a treatment plan leading to a desired effect. In some embodiments, the therapeutically effective amount may need to be administered in an amount sufficient to result in amelioration of one or more symptoms of a disorder, prevention of the advancement of a disorder, or regression of a disorder. In one example, treatment of an inflammatory disorder or an autoimmune disorder characterized by inflammation, a therapeutically effective amount preferably refers to the amount of a therapeutic agent that reduces the inflammation of a joint, organ or tissue by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. The term "treating" refers to the administering one or more diagnostic, therapeutic or prophylactic agents including, but not limited to IL-8 mimetics.

A "prophylactically effective amount" refers to an amount that is effective at the dosages and periods of time necessary to achieve a desired prophylactic result such as, for example, preventing or inhibiting a cytotoxic effect of a cytotoxic agent. Typically, a prophylactic dose is used in a subject prior to the onset of a disease, or at an early stage of the onset of a disease, to prevent or inhibit onset of the disease or symptoms of the disease. A prophylactically effective amount may be less than, greater than, or equal to a therapeutically effective amount.

In some embodiments, the administration can be oral. In other embodiments, the administration can be subcutaneous injection. In other embodiments, the administration can be intravenous injection using a sterile isotonic aqueous buffer. In another embodiment, the administration can include a solubilizing agent and a local anesthetic such as lignocaine to ease discomfort at the site of injection. In other embodiments, the administrations may be parenteral to obtain, for example, ease and uniformity of administration.

The compounds can be administered in dosage units. The term "dosage unit" refers to discrete, predetermined quantities of a compound that can be administered as unitary dosages to a subject. A predetermined quantity of active compound can be selected to produce a desired therapeutic effect and can be administered with a pharmaceutically acceptable carrier. The predetermined quantity in each unit dosage can depend on factors that include, but are not limited to, (a) the unique characteristics of the active compound and the particular therapeutic effect to be achieved, and (b) the limitations inherent in the art of creating and administering such dosage units.

A "pharmaceutically acceptable carrier" is a diluent, adjuvant, excipient, or vehicle with which the IL-8 mimetic is administered. A carrier is pharmaceutically acceptable after approval by a state or federal regulatory agency or listing in the U.S. Pharmacopeial Convention or other generally recognized sources for use in subjects.

The pharmaceutical carriers include any and all physiologically compatible solvents, dispersion media, coatings, antibacterial and antifungal agents, isotonic and absorption delaying agents, and the like. Examples of pharmaceutical carriers include, but are not limited to, sterile liquids, such as water, oils and lipids such as, for example, phospholipids and glycolipids. These sterile liquids include, but are not limited to, those derived from petroleum, animal, vegetable or synthetic origin such as, for example, peanut oil, soybean oil, mineral oil, sesame oil, and the like. Water can be a preferred carrier for intravenous administration. Saline solutions, aqueous dextrose and glycerol solutions can also be liquid carriers, particularly for injectable solutions.

Suitable pharmaceutical excipients include, but are not limited to, starch, sugars, inert polymers, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The composition can also contain minor amounts of wetting agents, emulsifying agents, pH buffering agents, or a combination thereof. The compositions can take the form of solutions, suspensions, emulsion, tablets, pills, capsules, powders, sustained-release formulations and the like. Oral formulations can include standard carriers such as, for example, pharmaceutical grades mannitol, lactose, starch, magnesium stearate, sodium saccharine, cellulose, magnesium carbonate, and the like. *See* Martin, E.W. Remington's Pharmaceutical Sciences. Supplementary active compounds can also be incorporated into the compositions.

In some embodiments, the carrier is suitable for parenteral administration. In other embodiments, the carrier can be suitable for intravenous, intraperitoneal, intramuscular, sublingual or oral administration. In other embodiments, the pharmaceutically acceptable carrier may comprise pharmaceutically acceptable salts.

Pharmaceutical formulations for parenteral administration may include liposomes. Liposomes and emulsions are delivery vehicles or carriers that are especially useful for hydrophobic drugs. Depending on biological stability of the therapeutic reagent, additional strategies for protein stabilization may be employed. Furthermore, one may administer the drug in a targeted drug delivery system such as, for example, in a liposome coated with target-specific antibody. The liposomes will bind to the target protein and be taken up selectively by the cell expressing the target protein.

Therapeutic compositions typically must be sterile and stable under the conditions of manufacture and storage. The composition can be formulated as a solution, microemulsion, liposome, or other ordered structure suitable for a high drug concentration. In some embodiments, the carrier can be a solvent or dispersion medium including, but not limited to, water; ethanol; a polyol such as for example, glycerol, propylene glycol, liquid polyethylene glycol, and the like; and, combinations thereof. The proper fluidity can be maintained in a variety of ways such as, for example, using a coating such as lecithin, maintaining a required particle size in dispersions, and using surfactants.

In some embodiments, isotonic agents can be used such as, for example, sugars; polyalcohols that include, but are not limited to, mannitol, sorbitol, glycerol, and combinations thereof; and sodium chloride. Sustained absorption characteristics can be introduced into the compositions by including agents that delay absorption such as, for example, monostearate salts, gelatin, and slow release polymers. Carriers can be used to protect active compounds against rapid release, and such carriers include, but are not limited to, controlled release formulations in implants and microencapsulated delivery systems. Biodegradable and biocompatible polymers can be used such as, for example, ethylene vinyl acetate, polyanhydrides, polyglycolic acid, collagen, polyorthoesters, polylactic acid, polycaprolactone, polyglycolic copolymer (PLG), and the like. Such formulations can generally be prepared using methods known to one of skill in the art.

The compounds may be administered as suspensions such as, for example, oily suspensions for injection. Lipophilic solvents or vehicles include, but are not limited to, fatty oils such as, for example, sesame oil; synthetic fatty acid esters, such as ethyl oleate or triglycerides; and liposomes. Suspensions that can be used for injection may also contain substances that increase the viscosity of the suspension such as, for example, sodium carboxymethyl cellulose, sorbitol, or dextran. Optionally, a suspension may contain stabilizers or agents that increase the solubility of the compounds and allow for preparation of highly concentrated solutions.

In one embodiment, a sterile and injectable solution can be prepared by incorporating an effective amount of an active compound in a solvent with any one or any combination of desired additional ingredients described above, filtering, and then sterilizing the solution. In another embodiment, dispersions can be prepared by incorporating an active compound into a sterile vehicle containing a dispersion medium and any one or any combination of desired additional ingredients described above. Sterile powders can be prepared for use in sterile and injectable solutions by vacuum drying, freeze-drying, or a combination thereof, to yield a powder that can be comprised of the active ingredient and any desired additional ingredients. Moreover, the additional ingredients can be from a separately prepared sterile and filtered solution. In another embodiment, an IL-8 mimetic may be prepared in combination with one or more additional compounds that enhance the solubility of the IL-8 mimetic.

In some embodiments, the compounds can be administered by inhalation through an aerosol spray or a nebulizer that may include a suitable propellant such as, for example, dichlorodifluoromethane, trichlorofluoromethane, dichlorotetrafluoroethane, carbon dioxide, or a combination thereof. In one example, a dosage unit for a pressurized aerosol may be delivered through a metering valve. In another embodiment, capsules and cartridges of gelatin, for example, may be used in an inhaler and can be formulated to contain a powderized mix of the compound with a suitable powder base such as, for example, starch or lactose.

In some embodiments, a therapeutically or prophylactically effective amount of an IL-8 mimetic may range in concentration from about 0.001 nM to about 0.1 M; from about 0.001 nM to about 0.05 M; from about 0.01 nM to about 15 µM; from about 0.01 nM to about 10 µM, or any range therein. In some embodiments, the IL-8 mimetics may be administered in an amount ranging from about 0.001 mg/kg to about 50 mg/kg; from about 0.005 mg/kg to about 40 mg/kg; from about 0.01 mg/kg to about 30 mg/kg; from about 0.01 mg/kg to about 25 mg/kg; from about 0.1 mg/kg to about 20 mg/kg; from about 0.2 mg/kg to about 15 mg/kg; from about 0.4 mg/kg to about 12 mg/kg; from about 0.15 mg/kg to about 10 mg/kg, or any range therein, wherein a human subject is assumed to average about 70 kg.

The IL-8 mimetics of the present invention can be administered as a diagnostic, therapeutic or prophylactic agent in a combination therapy with the administering of one or more other agents. The agents of the present invention can be administered concomitantly, sequentially, or cyclically to a subject. Cycling therapy involves the administering a first agent for a predetermined period of time, administering a second agent for a second predetermined period of time, and repeating this cycling for any desired purpose such as, for example, to enhance the efficacy of the treatment. The agents of the present invention can also be administered concurrently. The term "concurrently" is not limited to the administration of agents at exactly the same time, but rather means that the agents can be administered in a sequence and time interval such that the agents can work together to provide additional benefit. Each agent can be administered separately or together in any appropriate form using any appropriate means of administering the agent or agents.

Each of the agents described herein can be administered to a subject in combination therapy. In some embodiments, the agents can be administered at points in time that vary by about 15 minutes, 30 minutes, 1 hour, 2 hours, 4 hours, 8 hours, 12 hours, 18 hours, 24 hours, 48 hours or 1 week in time. In some embodiments, at least one of the agents is an immunomodulatory agent. In other embodiments, the agents can include antiproliferatives, antineoplastics, antimitotics, anti-inflammatories, antiplatelets, anticoagulants, antifibrins, antithrombins, antibiotics, antiallergics, antioxidants, and any prodrugs, codrugs, metabolites, analogs, homologues, congeners, derivatives, salts and combinations thereof.

The present invention encompasses sustained release formulations for the administration of one or more agents. In some embodiments, the sustained release formulations can reduce the dosage and/or frequency of the administrations of such agents to a subject.

### Articles of Manufacture

The present invention provides for articles of manufacture that encompass finished, packaged and labelled pharmaceutical products. The articles of manufacture include the appropriate unit dosage form in an appropriate vessel or container such as, for example, a glass vial or other container that is hermetically sealed. In the case of dosage forms suitable for parenteral administration, the active ingredient, e.g. one or more agents including an IL-8 mimetic, is sterile and suitable for administration as a particulate-free solution. In other words, the invention encompasses both parenteral solutions and lyophilized powders, each being sterile, and the latter being suitable for reconstitution prior to injection. Alternatively, the unit dosage form may be a solid suitable for oral, transdermal, topical or mucosal delivery.

In some embodiments, the unit dosage form is suitable for intravenous, intramuscular, topical or subcutaneous delivery. Thus, the invention encompasses solutions, which are preferably sterile and suitable for each route of delivery. The concentration of agents and amounts delivered are included as described herein.

As with any pharmaceutical product, the packaging material and container are designed to protect the stability of the product during storage and shipment. In addition, the articles of manufacture can include instructions for use or other information material that can advise the user such as, for example, a physician, technician or patient, regarding how to properly administer the composition as a diagnostic, prophylactic, therapeutic, or ameliorative treatment of the disease of concern. In some embodiments, instructions can indicate or suggest a dosing regimen that includes, but is not limited to, actual doses and monitoring procedures.

In other embodiments, the instructions can include informational material indicating that the administering of the compositions can result in adverse reactions including but not limited to allergic reactions such as, for example, anaphylaxis. The informational material can indicate that allergic reactions may exhibit only as mild pruritic rashes or may be severe and include erythroderma, vasculitis, anaphylaxis, Steven-Johnson syndrome, and the like. The informational material should indicate that anaphylaxis can be fatal and may occur when any foreign protein is introduced into the body. The informational material should indicate that these allergic reactions can manifest themselves as urticaria or a rash and develop into lethal systemic reactions and can occur soon after exposure such as, for example, within 10 minutes. The informational material can further indicate that an allergic reaction may cause a subject to experience paresthesia, hypotension, laryngeal edema, mental status changes, facial or pharyngeal angioedema, airway obstruction, bronchospasm, urticaria and pruritus, serum sickness, arthritis, allergic nephritis, glomerulonephritis, temporal arthritis, eosinophilia, or a combination thereof.

In some embodiments, the articles of manufacture can comprise one or more packaging materials such as, for example, a box, bottle, tube, vial, container, sprayer, insufflator, intravenous (I.V.) bag, envelope, and the like; and at least one unit dosage form of an agent comprising an IL-8 mimetic within the packaging material. In other embodiments, the articles of manufacture may also include instructions for using the composition as a diagnostic, prophylactic, therapeutic, or ameliorative treatment for the disease of concern.

In other embodiments, the articles of manufacture can comprise one or more packaging materials such as, for example, a box, bottle, tube, vial, container, sprayer, insufflator, intravenous (I.V.) bag, envelope, and the like; and a first composition comprising at least one unit dosage form of an agent comprising an IL-8 mimetic within the packaging material, along with a second composition comprising a second agent such as, for example, a glycosaminoglycan, phospholipid, poly(alkylene glycol), any other bioactive agent taught herein, or any prodrugs, codrugs, metabolites, analogs, homologues, congeners, derivatives, salts and combinations thereof. In other embodiments, the articles of manufacture may also include instructions for using the composition as a diagnostic, prophylactic, therapeutic, or ameliorative treatment for the disease of concern, which may include, for example, autoimmune disorders, inflammatory disorders, organ transplant rejections, disorders associated with the treatment of cancer, cardiovascular disorders, hematological disorders, neurological disorders, and infectious disorders.

### EXAMPLES

The following examples are presented to illustrate embodiments of the present invention and are not intended to limit the scope of the invention in any way:

### Example 1

Peptides of the invention may be synthesized chemically from the C-terminus to the N-terminus ("reverse sequence") using the Fmoc/tBu strategy on a continous flow peptide synthesizer. The following procedure is used, for example, to couple residues 56-71 of the native IL-8 sequence:

### Reagents and Procedures

Main Solvent: a grade certified, ACS spectroanalyzed, N, N-dimethylformamide (DMF) (Fisher, D131-4).

Deblocking Agent: 20% piperidine (Aldrich, 10,409-4) in DMF containing 0.5 % (v/v) triton X100 (Sigma, T-9284).

Activating Agents: 2-(H-benzotriazol-lyl)-1,1,3,3-tetramethyluronium tetrafluoroborate (TBTU) (Quantum Richelieu, R0139); hydroxybenzotriazole (HOBt) (Quantum Richelieu, R0166-100), each at a concentration of 0.52 M in DMF; and 4-methylmorpholine (NMM) (Aldrich, M5 655-7) at a concentration of 0.9 M in DMF. In the case of amino acids sensitive to racemization such as, for example, cysteine, a 2,4,6-collidine (Aldrich, 14,238-7) is used at a concentration of 0.78 M in a 1/1 (v/v) mixture of DMF/dichloromethane (DCM).

Support Resin: TentaGel R RAM (90 µm) beads are used with a 9-fluorenylmethoxycarbonyl (Fmoc) Rink-type linker (Peptides Int'1, RTS -9995-PI) in a column. The synthesis begins using 0.5 g of the resin with a degree of substitution of 0.21 mmol/g for 0.21 (0.5) or 0.101 mmol of peptide.

An Fmoc-L-amino derivative is prepared with protected side-chains. The side-chains are protected using t-butoxycarbonyl (Boc), t-butyl (tBu), and triphenylmethyl (Trt) groups in a 4 fold excess (Peptides Int'1; Bachem; Novabiochem; Chem-Impex, Inc). The Glu⁶³ and Lys⁶⁷ residues are Allyl-protected (Millipore/Perseptive Biosys.).

### Initial Amino Loading and Peptide Synthesis Procedure

The synthesis starts from the C-terminus, and the first amino acid attached to the resin in the synthesis is Asn⁷¹. The remaining residues are double coupled automatically at ambient temperature using a 4-fold excess of the residues and the coupling reagents, TBTU and HOBt in DMF, for each coupling. Double coupling is used to ensure a high yield of coupling and can be a second coupling step that follows single coupling.

The synthesis is interrupted after the first Val residue in the reverse sequence, Val⁶², for lactamization of residues Glu⁶³ and Lys⁶⁷ away from the column. The peptide bound to the support is cyclized by first removing the lateral allyl groups from Glu²⁴ and Lys²⁸ as described below. The peptide synthesis was then resumed.

### Removal of the Allyl Groups

The support-bound peptide is removed from the column and a 3-fold solution (347mg) oftetrakis(triphenylphosphine) palladium(0) (Pd(PPh₃)₄) (Sigma-Aldrich, 21,666-6) and 0.1 mmol of the peptide attached to the resin is dissolved in 5% acetic acid. The peptide is activated using 2.5% NMM in CHCl₃ at a concentration of 0.14 M under an argon purge. The solution is added to the support-bound peptide in a reaction vial containing a small magnetic bar for gentle stirring. The mixture is flushed with argon, sealed and stirred at room temperature for 6 hours. The support-bound peptide is transferred to a filter funnel and subject to a series of washes: (i) the first wash is with a 30 ml of a 0.5% (w/w) solution of sodium diethyldithiocarbonate in DMF; (ii) the second wash is with DCM alone; (iii) the third wash is with a 1/1 (v/v) mixture of DCM/DMF; and (iv) the fourth wash is with DMF alone. A positive Kaiser test indicated the deprotection of the amino side chained of the Lys⁶⁷.

### Lactam Formation:

Activating Agent: 7-azabenztriazol-1-yloxytris (pyrrolindino) phosphonium-hexafluorophosphate (PyAOP) (PerSeptive Biosys. GmbH, GEN076531) is used at a concentration that is 1.4-fold over the 0.105 mmol peptide sample size (*e.g.*, 0.105 mmol x 1.4 fold x 521.7 MW = 76.6 mg PyAOP); and NMM is used at a concentration that is 1.5-fold over the PyAOP (*e.g*., 0.105 mmol x 1.4 fold x 1 .5 fold = 0.23 mmol NMM).

The lactamization is a cyclization reaction that is carried out with the support-bound peptide in an amino acid vial at room temperature overnight (*e.g.*, ~16 hours) with gentle agitation. The support-bound peptide is poured back into the column, washed with DMF, and then allowed to continue through completion of the cyclization process, wherein a cyclic amide bridge is thereby introduced into the peptide. A negative Kaiser test is used to indicate the completion of the cyclization process.

### Removal of the Final Product from the Support

The support-bound peptide is removed from the synthesizer, placed in a medium filter funnel, washed with DCM to replace the non-volatile DMF, and thoroughly dried under high vacuum for at least two hours, or preferably, overnight.

Cleavage Mixture (reagent K): 100 ml of a trifluoroacetic acid
(TFA)/Phenol/Water/Thio-Anisol/EDT (82/5/5/5/2.5)(v/v) mixture is prepared. The support-bound peptide (0.5 g) is poured into 7.5 ml of reagent K with gentle agitation on a rocker, allowed to react for 4 hours at room temperature, filtered, and washed with neat TFA. The 7.5 ml of reagent K contains the following:

| | |
|---|---|
| TFA | 6.15ml (Halocarbon) |
| Phenol | 0.375ml (Aldrich) |
| Water | 0.375ml (MillQ) |
| Thio-Anisol | 0.375ml (Aldrich) |
| EDT | 0.187ml(Aldrich) |
| Total | 7.5ml |

### Precipitation of the Peptide

The cleaved (free) peptide solution is filtered through a filter funnel into a 50 ml round bottom flask. The support is rinsed twice with 4 ml TFA to release the free peptide. The solution of TFA and peptide is concentrated on a rotavap and added drop wise into cold diethyl ether previously treated with activated neutral aluminum oxide to make it free of peroxide. An excess of ether is used at approximately 10-fold the weight of the support. The support beads from which the peptide was cleaved was stored until the yield was determined and the peptide was characterized. The precipitate is collected at room temperature in a screw-capped 50 ml polypropylene vial after centrifugation for 4 minutes at 2000 rpm in a bench-top centrifuge. The pellets of free peptide were washed 3x with cold ether, centrifuged and dried under a flow of argon. The precipitate was dissolved in 20% acetonitrile with 0.1% TFA and lyophilized.

### Crude Product Characterization

The product is purified and characterized using an analytical HPLC procedure. A Vydac 218TP54 column (C18 reversed-phase, 4.6 mm x 150 mm inner column dimensions, and 5µm particle size). A multisolvent mobile phase is used, and the eluants are a 0.1% TFA/H₂O (solvent A) and a 0.1 % TFA/acetonitrile (solvent B).

Elution Conditions: A multisolvent delivery system is used and combines solvent A and solvent B to alter the polarity of the mobile phase during elution. The mobile phase is delivered at a flow rate of 1.0 ml/min and at a concentration of 20-50% B for 40 minutes; at a concentration of 60-90% B for 5 minutes; at a concentration of 90-20% B for 5 minutes; and at a concentration of 20% B for 10 minutes. The detector is set at 214 nm to read 0.5 absorbance units over a full scale.

### Sample Preparation:

An aliquot of the product is weighed and dissolved in a mixture of 20% acetonitrile/0.1% TFA (v/v) at a concentration of 2 mg/ml. The solution is microfuged and 20µl is injected into the HPLC column. Samples corresponding to the main and major peaks are collected, SpeedVac dried, and characterized by molecular weights using mass spectroscopy.

### Example 2

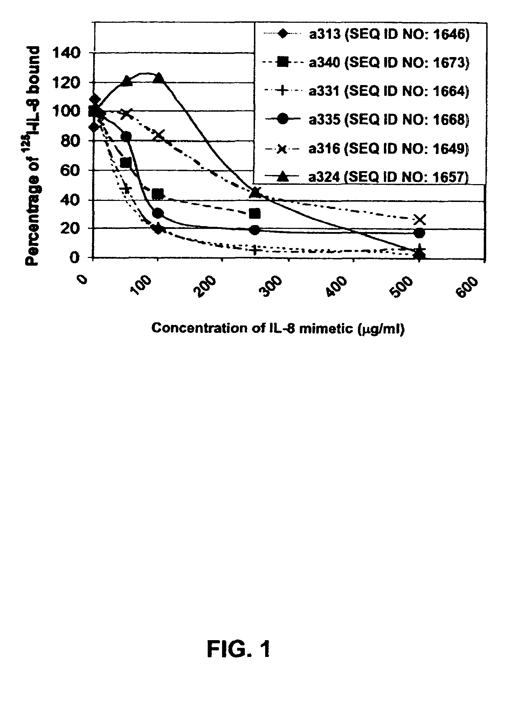

A competitive-dose-response binding assay was used to compare the ability of the native IL-8 to bind to the CXCR1/CXCR2 receptors with the ability of IL-8 agonists to bind to the CXCR1/CXCR2 receptors. An ¹²⁵I radiolabeled derivative of native IL-8 ("¹²⁵-IL-8") was used to measure the binding activity of native IL-8. The competitive dose response is shown in FIG. 1.

FIG. 1 shows the CXCR2 receptor binding of the IL-8 mimetics as competing ligands. Differentiated HL-60 cells were assessed for ¹²⁵ I-IL-8 binding following 2 hours of incubation with IL-8 or its agonist, and ¹²⁵I-IL-8. The ¹²⁵I-IL-8 was added at a concentration of 2nM in the presence of native IL-8 and the IL-8 mimetics at their respective concentrations as shown. The results are expressed as percentages of the maximal specific binding that was determined without competing ligand and are representative of one independent experiment.

The procedure used HL-60 cells (American Type Culture Collection) that were grown in an RPMI culture medium containing phenol red, 10% fetal bovine serum, and antibiotics consisting of 100U/ml penicillin G sodium and 100µg/ml streptomycin sulfate. The cells were added at a density ranging from about 2x10⁵ to about 8x10⁵ cells/ml. The cells were then induced to differentiate and express CXCR2 by treating the cells for 3-7 days with 1.25% DMSO. Millipore MultiScreen plates and a Durapore® membrane (Millipore Corp.) were used for high throughput binding assays.

The binding buffer used for the assay consisted of 0.5% (w/v) bovine serum albumin (BSA) (*e.g*., 0.5 g BSA/100 ml buffer), 50mM 4-(2-hydroxyethyl)-1-piperazineethanesulfonic acid (HEPES), 150mM NaCl, 5mM MgCl₂, 1mM CaCl₂ and 0.02% sodium azide. The HL-60 cells were harvested, washed with plain RPMI, and resuspended in binding buffer at density of about 5x 10⁶ cells/ml. The cells were preincubated with the IL-8 mimetics for 30 minutes. The binding buffer, the ¹²⁵I-IL-8, and the cells incubating with native IL-8 or IL-8 mimetic were then added to wells used to hold the cells in the assay. The cells were then incubated in the wells for another 2 hours with shaking. The IL-8 mimetics were used in concentrations indicated in FIG. 1 with a competitive dose of 2 nM of radiolabeled ¹²⁵I-IL-8.

After three washes with cold phosphate buffered saline (PBS), plates were dried and radioactivity counted using a CliniGamma gamma counter (LKB Wallac). Controls include (i) wells with only binding buffer and radiolabeled IL-8 chemokine for background, and (ii) wells with binding buffer, an unlabelled native IL-8 chemokine standard, radiolabeled chemokine and cells for standardization. A dose response curve is developed using a range of native IL-8 concentrations, and a concentration of 0 µg/ml is included.

Each data point is expressed as a percentage of the maximal specific binding that was determined using the radiolabeled native IL-8 without the competing IL-8 mimetics ("IL-8 agonist") and represents measurements obtained from two or three wells in a representative experiment. A concentration-dependent inhibition of ¹²⁵I-IL-8 binding is shown in FIG. 1 and summarized in Table 1 and indicates the affinity of native IL-8 and IL-8 mimetics for the receptor.

**Table 1.**

| SEQ ID NO: | IC₅₀ (µg/Ml) | ¹²⁵I-IL-8 bound at maximal inhibition (%) |
|---|---|---|
| (Native IL-8) | 2.8 | 2.2 |
| 1646 | 47.5 | 2.9 |
| 1673 | 50.0 | 30.4 |
| 1664 | 56.7 | 7.0 |
| 1670 | 56.7 | 19.3 |
| 1674 | 60.0 | 34.5 |
| 1665 | 61.7 | 7.9 |
| 1667 | 61.7 | 15.8 |
| 1666 | 63.3 | 15.4 |
| 1671 | 65.0 | 16.6 |
| 1663 | 66.7 | 3.3 |
| 1672 | 70.0 | 22.2 |
| 1675 | 70.0 | 18.0 |
| 1654 | 78.3 | 8.9 |
| 1668 | 78.8 | 14.2 |
| 1661 | 91.7 | 13.1 |
| 1655 | 93.3 | 12.5 |
| 1642 | 105.0 | 51.5 |
| 1647 | 130.6 | 4.1 |
| 1658 | 136.7 | 22.4 |
| 1656 | 175.0 | 6.7 |
| 1659 | 193.8 | 21.0 |
| 1649 | 200.0 | 16.3 |
| 1652 | 200.0 | 4.2 |
| 1653 | 200.0 | 8.2 |
| 1657 | 225.0 | 16.0 |
| 1645 | 283.3 | 37.3 |

Table 1 provides (i) IC₅₀ values for a variety of IL-8 mimetics to show the concentration of a particular IL-8 mimetic that is necessary to provide 50% of the maximal inhibition of ¹²⁵I-IL-8 binding that can be obtained with a particular IL-8 mimetic; and (ii) the maximal inhibition of the percent of ¹²⁵I-IL-8 bound to CXCR2 receptors on differentiated HL-60 cells for both native IL-8 and IL-8 mimetics. The inhibition of ¹²⁵I-IL-8 binding by IL-8 mimetics is indicative of the relative ability of the analogs to bind to CXCR1/CXCR2 receptors.

### Example 3

The results of calcium mobilization assays are summarized in Table 2 to show the agonistic activation of the IL-8 receptor by the native IL-8 and IL-8 mimetics. The HL-60 cells are cultured as indicated in Example 2, harvested and suspended in Tyrode's salt solution at a density of about 2x10⁶ cells/ml. The Tyrode's salt solution contains about 137 mM NaCl, 2.7mM KCI, 1 mM MgCl₂, 1 mM CaCl₂, 0.2 mM NaH₂PO₄, 12 mM NaHCO₃, and 5.5 mM glucose.

The cells are labeled with 4 µM of Fluo-4/AM (Molecular Probes, Inc.) for 45 minutes at 37°C to measure calcium mobilization from cells. The label is a dye that fluoresces when bound to calcium. The cells are labeled with the dye to obtain a measure of the amount of calcium released by the cells when the cells are treated with the IL-8 mimetic or native IL-8. An increase in fluorescence indicates an increase in calcium mobilization. The cells are washed three times with the Tyrode's salt solution after labeling and resuspended at 5 x 10⁶ cells/ml.

The native IL-8 and IL-8 mimetics are injected to produce a final concentration of about 10 µg/ml to about 200 µg/ml in aliquots containing about 5 x 10⁵ cells. Changes in the level of cellular fluorescence are read in a Thermo Labsystems Fluorskan Acsent fluorescence plate reader (VWR Scientific Prod's). The controls include cells treated with either the native chemokine or the plain medium of Tyrode's Salt Solution. Data is expressed using 1.0 as the standard level of fluorescence in the plain medium. The reported values represent the mean of at least duplicate measurements from wells in one or more experiments.

Table 2 provides a summary of the average fold increase of calcium mobilization in differentiated HL-60 cells over the control wells for native IL-8 and IL-8 mimetics.

**Table 2.**

| SEQ ID NO: | Average fold increase in calcium mobilization |
|---|---|
| (Native IL-8) | 2.5 |
| 1664 | 147.5 |
| 1666 | 143.8 |
| 1671 | 134.8 |
| 1663 | 112.9 |
| 1668 | 105.4 |
| 1665 | 103.6 |
| 1661 | 100.6 |
| 1656 | 86.8 |
| 1670 | 65.4 |
| 1647 | 57.3 |
| 1667 | 57.3 |
| 1655 | 41.8 |
| 1646 | 33.9 |
| 1649 | 28.2 |
| 1672 | 13.4 |
| 1675 | 12.2 |
| 1673 | 5.7 |
| 1674 | 1.4 |

As shown by the data in Table 2, the incubation of the HL-60 cells with the IL-8 mimetics enhanced the receptor-mediated calcium mobilization. Similarly, 10 µg/ml of native IL-8 was used as a positive control that induced a two to three fold increase in calcium mobilization.

### Example 4

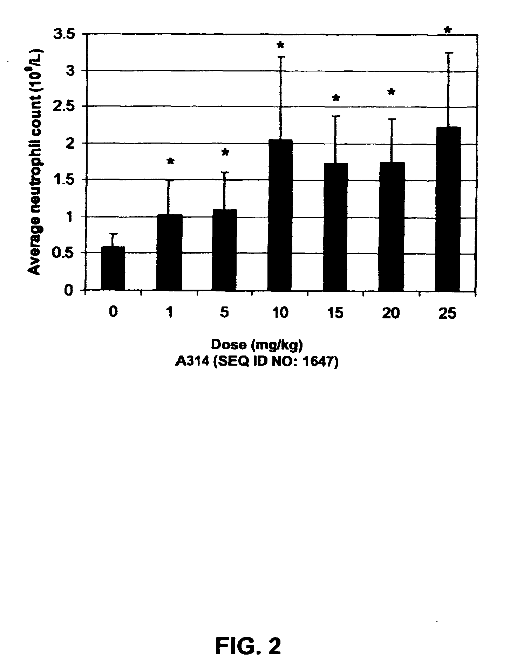

This example illustrates the efficacy of the IL-8 mimetic a161 (SEQ ID NO:1647) ("the test mimetic") in increasing the number of circulating neutrophils and hematopoietic progenitor/stem cells in a mouse model. The results are shown in FIGs. 2 through 5. The experiments consisted of the following groups of female Balb/c mice (Charles River Lab's): (1) an untreated control group of 10 mice; and (2) test groups of 10 mice each.

The control and test groups of 20-23 g mice were randomly grouped in appropriately labeled cages and identified by cage markings and ear punch. The test groups were treated one time subcutaneously with the test analog at doses of I, 5, 10, 15, 20, or 25mg/kg in volumes of approximately 200 µl. The mice were anesthetized immediately before blood collection. Blood samples were obtained from the mice at 30 minutes, 1 hour, 4 hours, 6 hours, 24 hours and/or 48 hours after administration of the test mimetic. Blood was collected with an EDTA S-Monovette syringe (Sarstedt) and 25G needle through a cardiac puncture. Blood was mixed gently by 5 inversions then expelled into a microcentrifuge tube. Differential and CBC analyses were performed on a Hemavet 850 FS (Drew Scientific). The end-point evaluations included complete blood counts with differentials and haematopoietic progenitor/stem cells as colony forming units (CFU).

The number ofhaematopoietic progenitor/stem cells was determined as follows. The volume of blood in each microfuge tube was determined and nine times the volume of ammonium chloride was added. Cells were incubated on ice for 10 minutes to lyse the red blood cells. The cells were washed twice and resuspended in 300 µL of Iscove's Modified Dulbecco's Medium (IMDM) containing 2% fetal bovine serum. The number of nucleated cells per mL of blood was counted, and all cells were plated in duplicate in standard methylcellulose to determine the number of CFUs including the colony-forming unit granulocyte-macrophage (CFU-GM), the burst-forming unit erythroid (BFU-E), and the colony-forming unit granulocyte erythrocyte macrophage megakaryocyte (CFU-GEMM). Plates were incubated for 7-14 days at 37°C in a fully humidified 5% CO₂-air atmosphere, and colonies containing more than 50 cells were scored using an inverted microscope. The total CFU per mL of blood from the individual mice was determined.

The differentials were used to evaluate the mobilization of neutrophils and were compared to the untreated control group. A time and concentration dependent increase in neutrophils and haematopoietic progenitor/stem cells in the circulation is shown in FIGs. 2 through 5, indicating the rapid and potent activity of the test analog *in vivo.*

FIG. 2 shows the response of circulating neutrophil counts to the administration of varying doses of the test mimetic following one hour of treatment. The test mimetic was administered by subcutaneous injection into female Balb/c mice in amounts of 1, 5, 10, 15, 20, or 25mg/kg. At 1 hour post-injection, the mice were euthanized and blood was collected by cardiac puncture. Complete blood counts and differentials were determined using a Hemavet. The values represent the mean (+/-) 1 standard deviation of 10 animals per treatment group. Statistically significant elevations as determined using a p value of<0.05 are indicated in FIG. 2 by a "*".

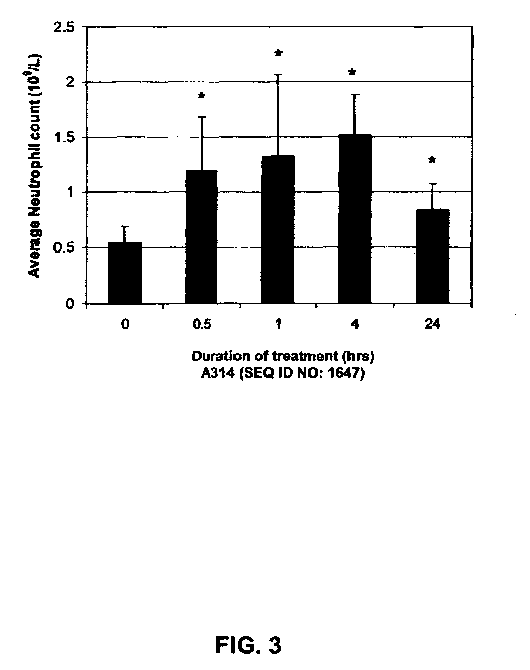

FIG. 3 describes the kinetics of the rise in circulating neutrophil counts in response to the administration of the test mimetic. The test mimetic was administered by subcutaneous injection into female Balb/c mice at 25mg/kg at time intervals of 30 minutes, 1 hour, 4 hours, and 24 hours. The mice were euthanized and blood was collected by cardiac puncture at each time interval. Complete blood counts and differentials were determined using a Hemavet®. The values represent the mean +/- one standard deviation for 10 animals per treatment group. Statistically significant elevations as determined using a p value of <0.05 are indicated in FIG. 3 by a "*".

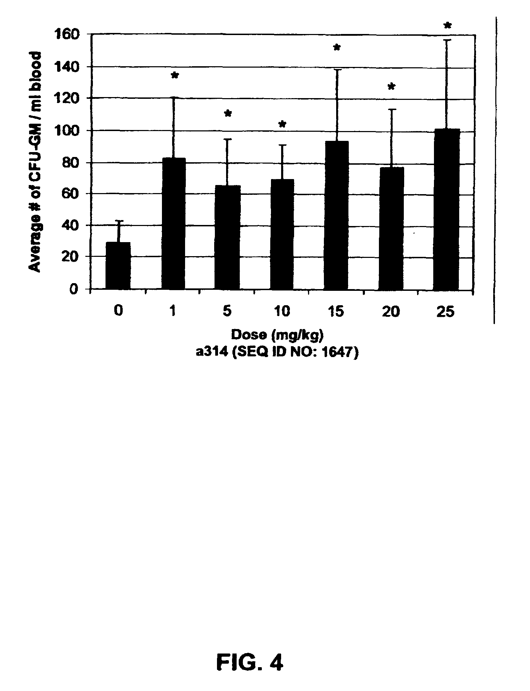

FIG. 4 shows the response of circulating haematopoietic progenitor/stem cells to the administration of varying doses of the test mimetic. The test mimetic was administered by subcutaneous injection into female Balb/c mice in amounts of 1, 5, 10, 15, 20, and 25mg/kg. At 1 hour post-injection, the mice were euthanized and blood was collected by cardiac puncture. The number of hematopoietic progenitor/stem cells (colony forming unit granulocyte-macrophage (CFU-GM), burst-forming unit erythroid (BFU-E) and colony forming unit granulocyte erythrocyte macrophage megakaryocyte (CFU-GEMM)) was determined by growing the cells in methylcellulose and counting the number of respective colonies. The values represent the mean +/- one standard deviation for 10 animals per treatment group. Statistically significant elevations as determined using a p value of <0.05 are indicated in FIG. 4 by a "*".

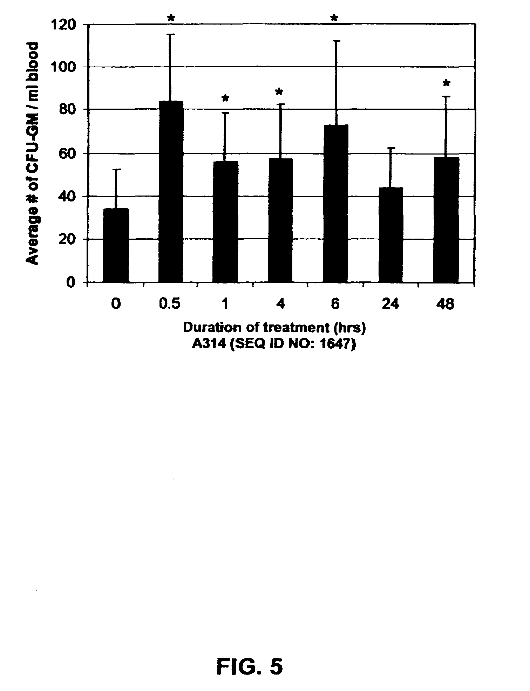

FIG. 5 describes the kinetics of the rise in haematopoietic progenitor/stem cells in response to the administration of the test mimetic. The test mimetic was administered by subcutaneous injection into female Balb/c mice at 25 mg/kg. At 30 minutes, 1 hour, 4 hours, 6 hours, 24 hours or 48 hours post-injection, mice were euthanized and blood collected by cardiac puncture. The number of haematopoietic progenitor/stem cells as measured by colony forming unit granulocyte-macrophage (CFU-GM), burst-forming unit erythroid (BFU-E), and colony forming unit granulocyte erythrocyte macrophage megakaryocyte (CFU-GEMM) were determined by growing the cells in methylcellulose and counting the number of respective colonies. The values represent the mean (+/-) one standard deviation for 10 animals per treatment group. Statistically significant elevations as determined using a p value of <0.05 are indicated in FIG. 5 by a "*".

### Example 5

Agents can be attached as modifying groups that are pendant or in-chain with an IL-8 mimetic. A trifunctional amino acid, for example, can be incorporated into the IL-8 mimetic as a linker and the third functionality can be connected to an agent. Protecting groups can be used to selectively attach an agent to the trifunctional amino acid. Benzyl esters are one type of protecting group that can be used for a lysine carboxyl, for example, and *t*-butoxycarbonyl can be used for amino groups such as, for example, the amino group in glutamic acid.

Amino, hydroxyl and carboxyl groups can be used, for example, as a connecting site for agents. Carboxyl groups can be used as a connecting site for agents having, for example, amino, hydroxyl, or thiol groups. Coupling agents include, but are not limited to, 1-ethyl-3(3-dimethylaminopropyl) carbodiimide (EDC) and 1,3-dicyclohexylcarbodiimide (DCC).

An example of an amine functional compound is 4-amino-TEMPO, an antioxidant that can be administered in combination with other therapies such as, for example, radiation therapy. Such an amine functional compound may be connected to a polymer containing free carboxyls such as, for example, the lysine-derived carboxyls, by first activating the carboxyls and coupling the amine in a solvent under agitation. The carboxyls may be activated with, for example, N-hydroxysuccinimide (NHS) and DCC in a solvent such as, for example, THF or chloroform, which produces N-hydroxysuccinimidyl ester. Examples of the solvent that may be used to couple the amine to the carboxyls include, but are not limited to, THF and DMF.

In some embodiments, the reaction occurs at a temperature ranging from about 5°C to about 50°C, from about 15°C to about 35°C, from about 20°C to about 30°C, or any range therein. In some embodiments, the reaction time ranges from about 0.5 hours to about 24 hours, from about 1 hour to about 18 hours, from about 4 hours to about 16 hours, from about 6 hours to about 12 hours, or any range therein.

A benzyl ester protecting group can be removed from a lysine carboxyl by hydrogenolysis with hydrogen gas over a catalyst such as, for example, palladium or platinum on carbon. Examples of suitable solvents include, but are not limited to, ethanol, methanol, isopropanol, and THF. The reaction may be conducted under about 1 atm of hydrogen for about 6 hours to about 24 hours, for about 8 hours to about 16 hours, for about 10 hours to about 14 hours, or any range therein.

### Example 6

A glycosaminoglycan can be connected to an amine functional group as an aldehyde-terminated heparin. An example of an aldehyde-terminated heparin is represented by the following formula: 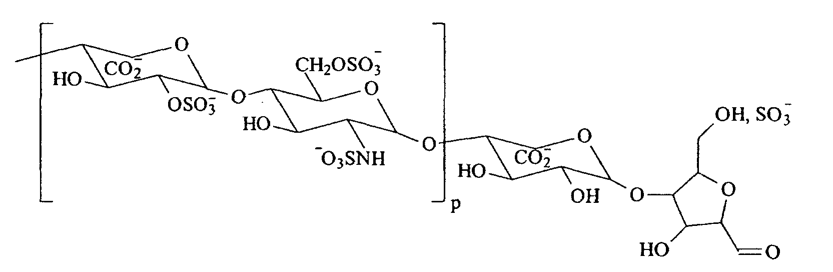 wherein m is an integer not equal to 0.

The aldehyde-terminated heparin can be combined with the amine functional group in a DMF/water solvent and subsequently reduced with NaCNBH₃ to produce the following structure: 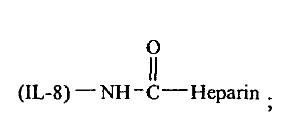 wherein m is an integer not equal to 0.

### Example 7

An IL-8 mimetic can be modified with a polyalkylene glycol using a variety of techniques. There are a variety of available PEG sizes and derivatives that are commercially designed for specific applications such as, for example, attachment to a variety of different chemical functionalities including, but not limited to, amines, thiols, hydroxyls, sulfhydryls, and carboxyls.

In one example, an amine group of an IL-8 can be combined with a carboxyl-terminated PEG (Nektar Corp.) in the presence of, for example, EDC or DCC to form the following structure: 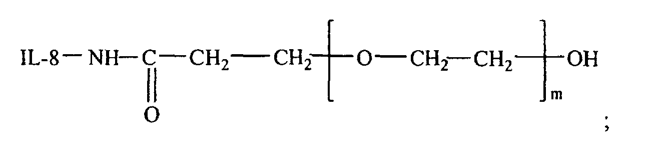 wherein m is an integer not equal to 0.

In another example, either a succinimidyl derivative of mPEG (Nektar Corp.) or an isocyanate-terminated mPEG (Nektar Corp.) can be combined with an IL-8 mimetic under conditions known to those of skill in the art. In another example, the carboxyl group of an IL-8 mimetic can be activated with, for example, EDC or DCC and combined with an amino-terminated mPEG (Nektar Corp.) In another example, an amine group of an IL-8 mimetic can be combined with a methacrylate-terminated mPEG (Nektar Corp.) in the presence of an initiator capable of undergoing thermal or photolytic free radical decomposition. Examples of suitable initiators include benzyl-N,N-diethyldithiocarbamate or *p*-xylene-N,N-diethyldithiocarbamate.

Those skilled in the art will recognize, or be able to ascertain using no more than routine experimentation, that there are many equivalents to the specific embodiments described herein. Such equivalents are intended to be encompassed by the following claims. All publications, patents, and patent applications mentioned in this application are herein incorporated by reference into the specification to the same extent as if each was specifically indicate to be herein incorporated by reference in its entirety.

## Claims

1. A composition comprising an IL-8 mimetic selected from a group consisting of SEQ ID NO:9 to SEQ ID NO:162, SEQ ID NO:1641 to SEQ ID NO:1675, variants a155 to a342, and conservatively modified variants, prodrugs, codrugs, and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, (polyethylene glycol) PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**1**} is independently selected from a group consisting of any L- or D-natural amino acid and any non-natural amino acid;
**Xaa**_{**2**} is independently selected from a group consisting of any L- or D-natural amino acid and any non-natural amino acid;
**Xaa**_{**3**} is independently selected from a group consisting of L-Pro, D-Pro, **P*, Btd** and any L- or D-natural and non-natural amino acid;
**Xaa**_{**4**} is independently selected from a group consisting of **P*, Btd** and any L-or D-natural amino acid and any non-natural amino acid, wherein
P* can be represented by a formula 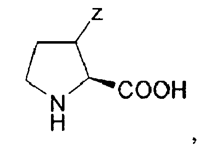 and
Btd can be represented by formula 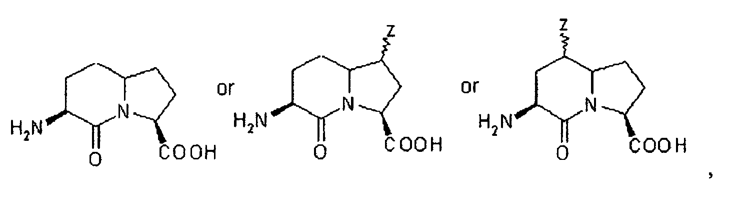 wherein z is independently selected from a group consisting of hydrogen, substituted, unsubstituted, hetero-, straight-chained, branched, cyclic, saturated or unsaturated aliphatic radicals; or substituted, unsubstituted, or heteroaromatic radicals, PEG and derivatives thereof, and combinations thereof;
**Xaa**_{**5**} is independently selected from the group consisting of any L- or D-natural amino acid and any non-natural amino acid with functional side chain to allow cyclization with Xaa₆.
**Xaa**_{**6**} is independently selected from the group consisting of any L- or D-natural amino acid and any non-natural amino acid with functional side chain to allow cyclization with Xaa₅; and
the **linker** is optional comprises any natural or non-natural amino acid,
wherein **R**_{**L**} is independently selected from a group consisting of substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof.

2. The composition of claim 1, wherein the IL-8 mimetic is selected from a group consisting of a 155 to a 169, and conservatively modified variants, prodrugs, codrugs and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**1**} is independently selected from a group consisting of any L- or D-natural amino acid and any non-natural amino acid;
**Xaa**_{**2**} is independently selected from a group consisting of any L- or D-natural amino acid and any non-natural amino acid; and
**Xaa**_{**3**} is independently selected from a group consisting of L-Pro, D-Pro, **P***, **Btd** and any L- or D-natural and non-natural amino acid.

3. The composition of claim 1, wherein the IL-8 mimetic is selected from a group consisting of a170 to a 171, and conservatively modified variants, prodrugs, codrugs and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof; and
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof.

4. The composition of claim 1, wherein the IL-8 mimetic is selected from a group consisting of a172, and conservatively modified variants, prodrugs, codrugs, and combinations thereof,
wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof; and
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof.

5. The composition of claim 4, wherein R_{N} comprises a peptide sequence Glu-Leu-Arg and a linker.

6. The composition of claim 1, wherein the IL-8 mimetic is selected from a group consisting of a173 to a235, and conservatively modified variants, prodrugs, codrugs, and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**1**} is independently selected from a group consisting of any L- or D-natural amino acid and any non-natural amino acid;
**Xaa**_{**2**} is independently selected from a group consisting of any L- or D-natural amino acid and any non-natural amino acid;
**Xaa**_{**3**} is independently selected from a group consisting of L-Pro, D-Pro, **P*,** Btd and any L- or D-natural and non-natural amino acid;
**Xaa**_{**4**} is independently selected from a group consisting of P*, **Btd** and any **L-**or D-natural amino acid and any non-natural amino acid, wherein
**P*** can be represented by a formula 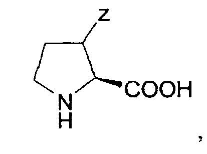 and
**Btd** can be represented by formula 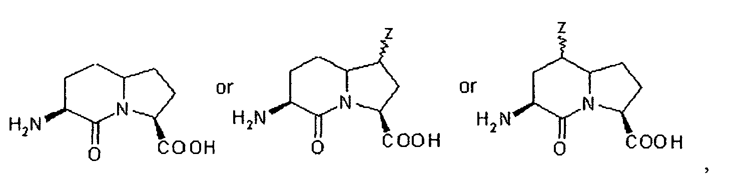 wherein z is independently selected from a group consisting of hydrogen, substituted, unsubstituted, hetero-, straight-chained, branched, cyclic, saturated or unsaturated aliphatic radicals; or substituted, unsubstituted, or heteroaromatic radicals, PEG and derivatives thereof, and combinations thereof; and
the **linker** is optional comprises any natural or non-natural amino acid,
wherein **R**_{**L**} is independently selected from a group consisting of substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof.

7. The composition of claim 1, wherein the IL-8 mimetic is selected from a group consisting of a236 to a 238, and conservatively modified variants, prodrugs, codrugs, and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**5**} is independently selected from the group consisting of any L- or D-natural amino acid and any non-natural amino acid with functional side chain to allow cyclization with Xaa₆; and
**Xaa**_{**6**} is independently selected from the group consisting of any L- or D-natural amino acid and any non-natural amino acid with functional side chain to allow cyclization with Xaa₅.

8. The composition of claim 1, wherein the IL-8 mimetic is selected from a group consisting of a239 and a 240, and conservatively modified variants, prodrugs, codrugs, and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**5**} is independently selected from the group consisting of any L- or D-natural amino acid and any non-natural amino acid with functional side chain to allow cyclization with Xaa₆; and
**Xaa**_{**6**} is independently selected from the group consisting of any L- or D-natural amino acid and any non-natural amino acid with functional side chain to allow cyclization with Xaa₅.

9. The composition of claim 1, wherein the IL-8 mimetic is selected from a group consisting of a241 to a244, and conservatively modified variants, prodrugs, codrugs, and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**5**} is independently selected from the group consisting of any L- or D-natural amino acid and any non-natural amino acid with functional side chain to allow cyclization with Xaa₆; and
**Xaa**_{**6**} is independently selected from the group consisting of any L- or D-natural amino acid and any non-natural amino acid with functional side chain to allow cyclization with Xaa₅.

10. The composition of claim 1, wherein the IL-8 mimetic is selected from a group consisting of a245 to a307, and conservatively modified variants, prodrugs, codrugs, and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**1**} is independently selected from a group consisting of any L- or D-natural amino acid and any non-natural amino acid;
**Xaa**_{**2**} is independently selected from a group consisting of any L- or D-natural amino acid and any non-natural amino acid;
**Xaa**_{**3**} is independently selected from a group consisting of L-Pro, D-Pro, **P***, Btd and any L- or D-natural and non-natural amino acid;
**Xaa**_{**4**} is independently selected from a group consisting of **P***, **Btd** and any L-or D-natural amino acid and any non-natural amino acid, wherein
**P*** can be represented by a formula 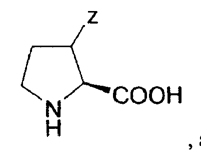 and
**Btd** can be represented by formula 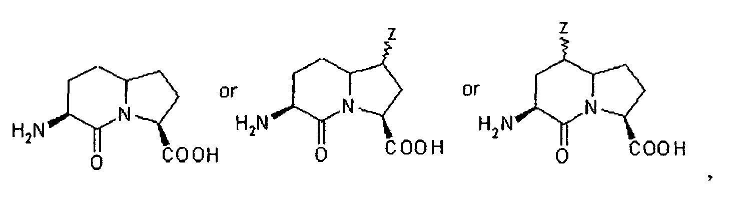 wherein z is independently selected from a group consisting of hydrogen, substituted, unsubstituted, hetero-, straight-chained, branched, cyclic, saturated or unsaturated aliphatic radicals; or substituted, unsubstituted, or heteroaromatic radicals, PEG and derivatives thereof, and combinations thereof; and
the linker is optional comprises any natural or non-natural amino acid,
wherein **R**_{**L**} is independently selected from a group consisting of substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof.

11. The composition of claim 1, 6 or 10, wherein R_{N}, R_{C}, or R_{L} comprises a functional group that is detectable using diagnostic or assay procedures.

12. The composition of claim 1, 6 or 10, wherein R_{N}, R_{C}, or R_{L} comprises a functional group that affects the pharmacokinetics of a native IL-8 sequence or an IL-8 mimetic.

13. The composition of claim 1, 6 or 10, wherein R_{N}, R_{C}, or R_{L} comprise a functional group that comprises a bioactive agent or biobeneficial agent.

14. The composition of claim 1, 6 or 10, wherein R_{C} or R_{L} comprises a functional group that can reduce an ability of a C-terminus to act as a substrate for carboxypeptidases.

15. The composition of claim 1, 6 or 10, wherein R_{N} or R_{L} comprises functional groups that can reduce an ability of an N-terminus to act as a substrate for aminopeptidases.

16. The composition of claim 1, 6 or 10, wherein R_{N}, R_{C}, or R_{L} comprises a functional group that is independently selected from a group consisting of hydrogen, acyl, alkyl, aryl, aralkyl, oxygen-containing groups, nitrogen-containing groups, sulfur-containing groups, thio-derivatives of urethanes where at least one oxygen atom is replaced by a sulfur atom, silyl, phosphoryl, phosphonate, phosphinate, ethylenically unsaturated groups, biotinylated groups, natural and non-natural amino acids, bioactive agents, biobeneficial agents, diagnostic agents, chemokines, chemokine mimetics, chemokine receptor ligands, and combinations thereof.

17. The composition of claim 1, 6 or 10, wherein R_{N}, R_{C}, or R_{L} comprises a functional group that is independently selected from a group consisting of acyl, hydroxyalkyl, hydroxyalkylaryl, hydroxy acid, polyalkylene glycol, amine, amide, azide, hydrazide, alkyl amine, alkyl amide, aralkyl amine, aralkyl amide, carbamate, cholyl, natural and non-natural amino acid, biotin-containing, enzyme-containing, radiopaque, radioactive, paramagnetic, fluorescent, and luminescent groups, and derivatives and combinations thereof.

18. The composition of claim 1, 6 or 10, wherein R_{N}, R_{C}, or R_{L} comprises a functional group that is independently selected from a group consisting of formyl, acetyl, diethylenetriaminepentaacetyl, O-methoxyacetyl, (-)-methoxyacetyl, 2-norbornaneacetyl, γ-oxo-5-acenaphthenebutyryl, 2-imino-1-imidazolidineacetyl, tetrahydro-3-furyl, (S)-(-)-indoline-2-carboxyl, PEGyl, hydroxyethyl, hydroxymethylbenzyl ether, ethyl amide, phenethyl amide, N-acetylneuraminyl, 3-(O-aminoethyl-iso-)-cholyl, trans-4-cotininecarboxyl, (-)-2-oxo-4-thiazolidinecarboxyl, (4-morpholine)carbonyl, 2-thiopheneacetyl, 2-thiophenesulfonyl, D-amino acid, β-alanine, 2-iminobiotinyl, horse radish peroxidase, alkaline phosphatase, β-galactosidase, acetylcholinesterase, streptavidin/biotin, avidin/biotin, umbelliferone, fluorescein, fluorescein isothiocyanate, rhodamine, dichlorotriazinylamine fluorescein, dansyl chloride, phycoerythrin, luminal, ¹⁴C, ¹²³I, ¹²⁴I, ¹²⁵I, ¹³¹I, ^{99m}Tc, ³²P, ³H, and radioactive iodotyrosyl groups, and derivatives and combinations thereof.

19. The composition of claim 2-4 or 7-9, wherein R_{N} or R_{C} comprises a functional group that is detectable using diagnostic or assay procedures.

20. The composition of claim 2-4 or 7-9, wherein R_{N} or R_{C} comprises a functional group that affects the pharmacokinetics of a native IL-8 sequence or an IL-8 mimetic.

21. The composition of claim 2-4 or 7-9, wherein R_{N} or R_{C} comprises a functional group that affects a pharmacokinetics of a native IL-8 sequence or an IL-8 mimetic and is detectable using diagnostic or assay procedures.

22. The composition of claim 2-4 or 7-9, wherein R_{N} or R_{C} comprise a functional group that comprises a bioactive or biobeneficial agent.

23. The composition of claim 2-4 or 7-9, wherein R_{N} or R_{C} comprises a functional group comprising a codrug.

24. The composition of claim 2-4 or 7-9, wherein R_{C} comprises a functional group that can reduce an ability of a C-terminus to act as a substrate for carboxypeptidases.

25. The composition of claim 2-4 or 7-9, wherein R_{N} comprises functional groups that can reduce an ability of an N-terminus to act as a substrate for aminopeptidases.

26. The composition of claim 2-4 or 7-9, wherein R_{N} or R_{C} comprises a functional group that is independently selected from a group consisting of hydrogen, alkyl, aryl, aralkyl, oxygen-containing groups, nitrogen-containing groups, sulfur-containing groups, thio-derivatives of urethanes where at least one oxygen atom is replaced by a sulfur atom, silyl, phosphoryl, phosphonate, phosphinate, ethylenically unsaturated groups, biotinylated groups, natural and non-natural amino acids, bioactive agents, biobeneficial agents, diagnostic agents, chemokines, chemokine mimetics, chemokine receptor ligands, and combinations thereof.

27. The composition of claim 2-4 or 7-9, wherein R_{N} or R_{C} comprises a functional group that is independently selected from a group consisting of acyl, hydroxyalkyl, hydroxyalkylaryl, hydroxy acid, polyalkylene glycol, amine, amide, azide, hydrazide, alkyl amine, alkyl amide, aralkyl amine, aralkyl amide, carbamate, cholyl, natural and non-natural amino acid, biotin-containing, enzyme-containing, radiopaque, radioactive, paramagnetic, fluorescent, and luminescent groups, and derivatives and combinations thereof.

28. The composition of claim 2-4 or 7-9, wherein R_{N} or R_{C} comprises a functional group that is independently selected from a group consisting of formyl, acetyl, diethylenetriaminepentaacetyl, O-methoxyacetyl, (-)-methoxyacetyl, 2-norbornaneacetyl, γ-oxo-5-acenaphthenebutyryl, 2-imino-1-imidazolidineacetyl, tetrahydro-3-furyl, (S)-(-)-indoline-2-carboxyl, PEGyl, hydroxyethyl, hydroxymethylbenzyl ether, ethyl amide, phenethyl amide, N-acetylneuraminyl, 3-(O-aminoethyl-iso-)-cholyl, trans-4-cotininecarboxyl, (-)-2-oxo-4-thiazolidinecarboxyl, (4-morpholine)carbonyl, 2-thiopheneacetyl, 2-thiophenesulfonyl, D-amino acid, β-alanine, 2-iminobiotinyl, horse radish peroxidase, alkaline phosphatase, β-galactosidase, acetylcholinesterase, streptavidin/biotin, avidin/biotin, umbelliferone, fluorescein, fluorescein isothiocyanate, rhodamine, dichlorotriazinylamine fluorescein, dansyl chloride, phycoerythrin, luminal, ¹⁴C, ¹²³I, ¹²⁴I, ¹²⁵I, ¹³¹I, ^{99m}Tc, ³²P, ³H, and radioactive iodotyrosyl groups, and derivatives and combinations thereof.

29. The composition of claim 1, wherein the IL-8 mimetic is selected from a group consisting of a308 and conservatively modified variants, prodrugs, codrugs, and combinations thereof.

30. The composition of claim 1, wherein the IL-8 mimetic is selected from a group consisting of a309 and conservatively modified variants, prodrugs, codrugs, and combinations thereof.

31. The composition of claim 1, wherein the IL-8 mimetic is selected from a group consisting of a310 to a312 and conservatively modified variants, prodrugs, codrugs, and combinations thereof.

32. The composition of claim 1, wherein the IL-8 mimetic is selected from a group consisting of a313, a314, a316 and conservatively modified variants, prodrugs, codrugs, and combinations thereof.

33. The composition of claim 1, wherein the IL-8 mimetic is selected from a group consisting of a315 and conservatively modified variants, prodrugs, codrugs, and combinations thereof.

34. The composition of claim 1, wherein the IL-8 mimetic is selected from a group consisting of a317, a318, a320, a322, a324, a326-a329 and conservatively modified variants, prodrugs, codrugs, and combinations thereof.

35. The composition of claim 1, wherein the IL-8 mimetic is selected from a group consisting of a319, a321, a323, a325 and conservatively modified variants, prodrugs, codrugs, and combinations thereof.

36. The composition of claim 1, wherein the IL-8 mimetic is selected from a group consisting of a330 to a342 and conservatively modified variants, prodrugs, codrugs, and combinations thereof.

37. The composition of claim 1, wherein the codrug comprises an agent selected from a group consisting of glycosaminoglycans, phospholipids, poly(alkylene glycols), and any prodrugs, codrugs, metabolites, analogs, homologues, congeners, derivatives, salts and combinations thereof.

38. The composition of claim 37, wherein the glycosaminoglycan comprises heparin, and any and any prodrugs, codrugs, metabolites, analogs, homologues, congeners, derivatives, salts and combinations thereof.

39. The composition of claim 37 wherein the phospholipid comprises phophatidylcholine, or any prodrugs, codrugs, metabolites, analogs, homologues, congeners, derivatives, salts and combinations thereof.

40. The composition of claim 37, wherein the poly(alkylene glycol) is PEG, and any analogs, homologues, congeners, derivatives, salts and combinations thereof.

41. A method for treating a disease in a subject, and/or ameliorating one or more symptoms thereof, comprising administering to a subject a composition comprising an effective amount of an IL-8 mimetic selected from a group consisting of SEQ ID NO:9 to SEQ ID NO:162, SEQ ID NO:1641 to SEQ ID NO:1675, variants a155-a342, and conservatively modified variants, prodrugs, codrugs, and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, (polyethylene glycol) PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**1**} is independently selected from a group consisting of any L- or D-natural amino acid and any non-natural amino acid;
**Xaa**_{**2**} is independently selected from a group consisting of any L- or D-natural amino acid and any non-natural amino acid;
**Xaa**_{**3**} is independently selected from a group consisting of L-Pro, D-Pro, **P***, Btd and any L- or D-natural and non-natural amino acid;
**Xaa**_{**4**} is independently selected from a group consisting of **P***, **Btd** and any **L-**or D-natural amino acid and any non-natural amino acid, wherein
**P***, can be represented by a formula 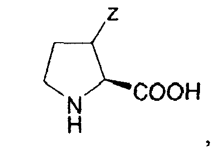 and
**Btd** can be represented by formula 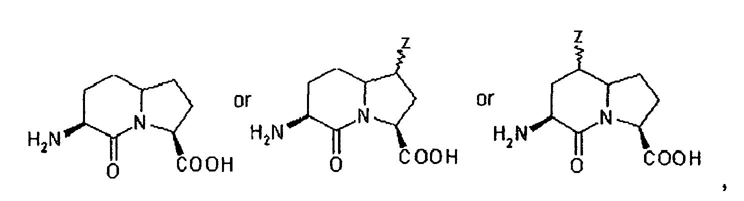 wherein z is independently selected from a group consisting of hydrogen, substituted, unsubstituted, hetero-, straight-chained, branched, cyclic, saturated or unsaturated aliphatic radicals; or substituted, unsubstituted, or heteroaromatic radicals, PEG and derivatives thereof, and combinations thereof;
**Xaa**₅ is independently selected from the group consisting of any L- or D-natural amino acid and any non-natural amino acid with functional side chain to allow cyclization with **Xaa**_{**6**}.
**Xaa**_{**6**} is independently selected from the group consisting of any L- or D-natural amino acid and any non-natural amino acid with functional side chain to allow cyclization with Xaa₅; and
the **linker** is optional comprises any natural or non-natural amino acid,
wherein **R**_{**L**} is independently selected from a group consisting of substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof.

42. The method of claim 41, wherein R_{N}, R_{C}, or R_{L} comprises a functional group that is detectable using diagnostic or assay procedures.

43. The method of claim 41, wherein R_{N}, R_{C}, or R_{L} comprises a functional group that affects a pharmacokinetics of a native IL-8 sequence or an IL-8 mimetic.

44. The method of claim 41, wherein R_{N}, R_{C}, or R_{L} comprises a functional group that affects a pharmacokinetics of a native IL-8 sequence or an IL-8 mimetic and is detectable using diagnostic or assay procedures.

45. The method of claim 41, wherein R_{N}, R_{C}, or R_{L} comprises a functional group that comprises a bioactive or biobeneficial agent.

46. The method of claim 41, wherein R_{N}, R_{C}, or R_{L} comprises a functional group comprising a codrug.

47. The method of claim 41, wherein R_{C} or R_{L} comprises a functional group that can reduce an ability of a C-terminus to act as a substrate for carboxypeptidases.

48. The method of claim 41, wherein R_{N} or R_{L} comprises functional groups that can reduce an ability of an N-terminus to act as a substrate for aminopeptidases.

49. The method of claim 41, wherein R_{N}, R_{C}, or R_{L} comprises a functional group that is independently selected from a group consisting of hydrogen, alkyl, aryl, aralkyl, oxygen-containing groups, nitrogen-containing groups, sulfur-containing groups, thio-derivatives of urethanes where at least one oxygen atom is replaced by a sulfur atom, silyl, phosphoryl, phosphonate, phosphinate, ethylenically unsaturated groups, biotinylated groups, natural and non-natural amino acids, bioactive agents, biobeneficial agents, diagnostic agents, chemokines, chemokine mimetics, chemokine receptor ligands, and combinations thereof.

50. The method of claim 41, wherein R_{N}, R_{C}, or R_{L} comprises a functional group that is independently selected from a group consisting of acyl, hydroxyalkyl, hydroxyalkylaryl, hydroxy acid, polyalkylene glycol, amine, amide, azide, hydrazide, alkyl amine, alkyl amide, aralkyl amine, aralkyl amide, carbamate, cholyl, natural and non-natural amino acid, biotin-containing, enzyme-containing, radiopaque, radioactive, paramagnetic, fluorescent, and luminescent groups, and derivatives and combinations thereof.

51. The method of claim 41, wherein R_{N}, R_{C}, or R_{L} comprises a functional group that is independently selected from a group consisting of formyl, acetyl, diethylenetriaminepentaacetyl, O-methoxyacetyl, (-)-methoxyacetyl, 2-norbornaneacetyl, γ-oxo-5-acenaphtbenebutyryl, 2-imino-1-imidazolidineacetyl, tetrahydro-3-furyl, (S)-(-)-indoline-2-carboxyl, PEGyl, hydroxyethyl, hydroxymethylbenzyl ether, ethyl amide, phenethyl amide, N-acetylneuraminyl, 3-(O-aminoethyl-iso-)-cholyl, trans-4-cotininecarboxyl, (-)-2-oxo-4-thiazolidinecarboxyl, (4-morpholine)carbonyl, 2-thiopheneacetyl, 2-thiophenesulfonyl, D-amino acid, β-alanine, 2-iminobiotinyl, horse radish peroxidase, alkaline phosphatase, β-galactosidase, acetylcholinesterase, streptavidin/biotin, avidin/biotin, umbelliferone, fluorescein, fluorescein isothiocyanate, rhodamine, dichlorotriazinylamine fluorescein, dansyl chloride, phycoerythrin, luminal, ¹⁴C, ¹²³I, ¹²⁴I, ¹²⁵I, ¹³¹I, ^{99m}Tc, ³²P, ³H, and radioactive iodotyrosyl groups, and derivatives and combinations thereof.

52. The method of claim 41, wherein the disease is selected from a group consisting of autoimmune disorders, inflammatory disorders, organ transplant rejections, disorders associated with the treatment of cancer, cardiovascular disorders, hematological disorders, neurological disorders, infectious disorders, and combinations thereof.

53. The method of claim 41, wherein the administering modulates CXCR1 or CXCR2 receptor activity.

54. The method of claim 41, wherein the administration mobilizes intracellular calcium in the subject.

55. The method of claim 41, wherein the administration increases cell expansion in the subject or *ex vivo.*

56. The method of claim 55, wherein the cells are selected from group consisting of , endothelial cells, leukocytes, red blood cells, megakaryocytes, stem cells, progenitor cells, and combinations thereof.

57. The method of claim 56, wherein the endothelial cells are vascular endothelial cells.

58. The method of claim 56, wherein the leukocytes are neutrophils or lymphocytes.

59. The method of claim 56, wherein the stem cells are selected from a group consisting of neuronal stem cells, neural crest stem cells, hematopoietic stem cells, lymphoid stem cells, myeloid stem cells, mesenchymal stem cells, and combinations thereof.

60. The method of claim 41, wherein the administering increases the hemocrit in the subject.

61. The method of claim 41, wherein the administration maintains cells in a quiescent state in a subject.

62. The method of claim 61, wherein the quiescent state protects cells undergoing treatment with a cytotoxic agent or radiation from apoptosis.

63. The method of claim 41, wherein the administration assists in gene therapy by arresting the cell cycle.

64. The method of claim 61, 62 or 63, wherein the cells are selected from a group consisting of endothelial cells, red blood cells, leukocytes, megakaryocytes, stem cells, progenitor cells and combinations thereof.

65. The method of claim 64, wherein the leukocytes are neutrophils, lymphocytes, or combinations thereof

66. The method of claim 64, wherein the stem cells are selected from a group consisting of neuronal stem cells, neural crest stem cells, hematopoietic stem cells, lymphoid stem cells, myeloid stem cells, mesenchymal stem cells, and combinations thereof.

67. The method of claim 41, wherein the administration mobilizes cells in a subject.

68. The method of claim 67, wherein said cells are red blood cells, leukocytes, stem cells or progenitor cells.

69. The method of claim 68, wherein the leukocytes are neutrophils or lymphocytes.

70. The method of claim 68, wherein the stem cells are selected from a group consisting of neuronal stem cells, neural crest stem cells, hematopoietic stem cells, lymphoid stem cells, myeloid stem cells, mesenchymal stem cells, and combinations thereof.

71. The method of claim 41, wherein the administration increases angiogenesis in a subject.

72. The method of claim 41, wherein the administration is performed in combination with a second administration comprising a second composition comprising a second agent.

73. The method of claim 72, wherein the second agent is selected from a group consisting of an IL-8 mimetic, a bioactive agent or a diagnostic agent.

74. A method comprising contacting a cell with an IL-8 mimetic, wherein the contacting comprises combining an IL-8 mimetic with a cell *in vitro,* wherein the IL-8 mimetic is selected from a group consisting of SEQ ID NO:9 to SEQ ID NO: 162, SEQ ID NO:1641 to SEQ ID NO:1675, variants a155-a342 and conservatively modified variants, prodrugs, codrugs, and combinations thereof, wherein
**R**_{**N**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, (polyethylene glycol) PEG and derivatives thereof, and combinations thereof;
**R**_{**C**} is independently selected from a group consisting of hydrogen, substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof;
**Xaa**_{**1**} is independently selected from a group consisting of any L- or D-natural amino acid and any non-natural amino acid;
**Xaa**_{**2**} is independently selected from a group consisting of any L- or D-natural amino acid and any non-natural amino acid;
**Xaa**_{**3**} is independently selected from a group consisting of L-Pro, D-Pro, **P*,** Btd and any L- or D-natural and non-natural amino acid;
**Xaa**_{**4**} is independently selected from a group consisting of **P***, **Btd** and any L-or D-natural amino acid and any non-natural amino acid, wherein
**P*** can be represented by a formula 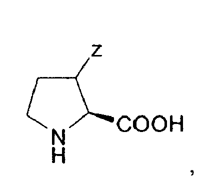 and
**Btd** can be represented by formula 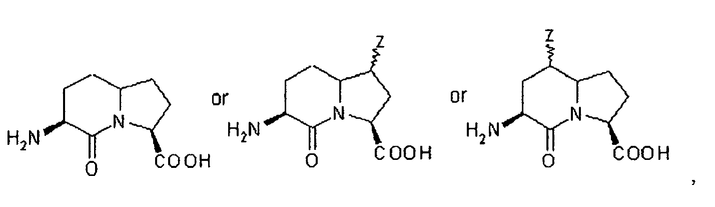 wherein z is independently selected from a group consisting of hydrogen, substituted, unsubstituted, hetero-, straight-chained, branched, cyclic, saturated or unsaturated aliphatic radicals; or substituted, unsubstituted, or heteroaromatic radicals, PEG and derivatives thereof, and combinations thereof;
**Xaa**_{**5**} is independently selected from the group consisting of any L- or D-natural amino acid and any non-natural amino acid with functional side chain to allow cyclization with Xaa₆.
**Xaa**_{**6**} is independently selected from the group consisting of any L- or D-natural amino acid and any non-natural amino acid with functional side chain to allow cyclization with Xaa₅; and
the **linker** is optional comprises any natural or non-natural amino acid,
wherein **R**_{**L**} is independently selected from a group consisting of substituted, unsubstituted, hetero-, straight-chained, branched, cyclic, saturated or unsaturated aliphatic radicals; or substituted, unsubstituted, or hetero- aromatic radicals, PEG and derivatives thereof, and combinations thereof.

75. The method of claim 74, wherein R_{N}, R_{C}, or R_{L} comprises a functional group that is detectable using diagnostic or assay procedures.

76. The method of claim 74, wherein R_{N}, R_{C}, or R_{L} comprises a functional group that affects a pharmacokinetics of a native IL-8 sequence or an IL-8 mimetic and is detectable using diagnostic or assay procedures.

77. The method of claim 74, wherein R_{N}, R_{C}, or R_{L} comprises a functional group that comprises a bioactive or biobeneficial agent.

78. The method of claim 74, wherein R_{N}, R_{C}, or R_{L} comprises a functional group comprising a codrug.

79. The method of claim 74, wherein R_{C} or R_{L} comprises a functional group that can reduce an ability of a C-terminus to act as a substrate for carboxypeptidases.

80. The method of claim 74, wherein R_{N} or R_{L} comprises functional groups that can reduce an ability of an N-terminus to act as a substrate for aminopeptidases.

81. The method of claim 74, wherein R_{N}, R_{C}, or R_{L} comprises a functional group that is independently selected from a group consisting of hydrogen, alkyl, aryl, aralkyl, oxygen-containing groups, nitrogen-containing groups, sulfur-containing groups, thio-derivatives of urethanes where at least one oxygen atom is replaced by a sulfur atom, silyl, phosphoryl, phosphonate, phosphinate, ethylenically unsaturated groups, biotinylated groups, natural and non-natural amino acids, bioactive agents, biobeneficial agents, diagnostic agents, chemokines, chemokine mimetics, chemokine receptor ligands, and combinations thereof.

82. The method of claim 74, wherein R_{N}, R_{C}, or R_{L} comprises a functional group that is independently selected from a group consisting of acyl, hydroxyalkyl, hydroxyalkylaryl, hydroxy acid, polyalkylene glycol, amine, amide, azide, hydrazide, alkyl amine, alkyl amide, aralkyl amine, aralkyl amide, carbamate, cholyl, natural and non-natural amino acid, biotin-containing, enzyme-containing, radiopaque, radioactive, paramagnetic, fluorescent, and luminescent groups, and derivatives and combinations thereof.

83. The method of claim 74, wherein R_{N}, R_{C}, or R_{L} comprises a functional group that is independently selected from a group consisting of formyl, acetyl, diethylenetriaminepentaacetyl, O-methoxyacetyl, (-)-methoxyacetyl, 2-norbornaneacetyl, γ-oxo-5-acenaphthenebutyryl, 2-imino-1-imidazolidineacetyl, tetrahydro-3-furyl, (S)-(-)-indoline-2-carboxyl, PEGyl, hydroxyethyl, hydroxymethylbenzyl ether, ethyl amide, phenethyl amide, N-acetylneuraminyl, 3-(O-aminoethyl-iso-)-cholyl, trans-4-cotininecarboxyl, (-)-2-oxo-4-thiazolidinecarboxyl, (4-morpholine)carbonyl, 2-thiopheneacetyl, 2-thiophenesulfonyl, D-amino acid, β-alanine, 2-iminobiotinyl, horse radish peroxidase, alkaline phosphatase, β-galactosidase, acetylcholinesterase, streptavidin/biotin, avidin/biotin, umbelliferone, fluorescein, fluorescein isothiocyanate, rhodamine, dichlorotriazinylamine fluorescein, dansyl chloride, phycoerythrin, luminal, ¹⁴C, ¹²³I, ¹²⁴I, ¹²⁵I, ¹³¹I, ^{99m}Tc, ³²P, ³H, and radioactive iodotyrosyl groups, and derivatives and combinations thereof.

84. The method of claim 74, wherein the cell is selected from a group consisting of red blood cells, leukocytes, megakaryocytes, endothelial cells, stem cells, and progenitor cells.

85. The method of claim 84, wherein the leukocytes are neutrophils, lymphocytes, or a combination thereof.

86. The method of claim 84, wherein the endothelial cells are vascular endothelial cells.

87. The method of claim 84, wherein the stem cells are selected from a group consisting of neuronal stem cells, neural crest stem cells, hematopoietic stem cells, lymphoid stem cells, myeloid stem cells, mesenchymal stem cells, and combinations thereof.

88. The method of claim 74, further comprising assessing the activity of the IL-8 mimetic using a diagnostic agent.

89. The method of claim 74, further comprising producing a vaccine with antigens obtained from the combining.

90. An article of manufacture comprising;
a first composition comprising a first IL-8 mimetic;
instructions for administering the first composition to a subject and monitoring the subject.

91. The article of manufacture of claim 90, wherein the first composition comprises a second agent.

92. The article of manufacture of claim 91, wherein the second agent is a glycosaminoglycan, a phospholipid, a poly(alkylene glycol), or any prodrugs, codrugs, metabolites, analogs, homologues, congeners, derivatives, salts and combinations thereof.

93. The article of manufacture of claim 91, wherein the second agent is heparin, phosphatidylcholine, PEG, or a combination thereof.

94. The article of manufacture of claim 91, wherein the second agent is in a codrug form with an IL-8 mimetic.

95. The article of manufacture of claim 90 further comprising a second composition comprising a second agent for administering in combination with the first IL-8 mimetic,
wherein the instructions further comprise instruction on administering the second composition and monitoring the subject.
